# EUROPEAN PATENT APPLICATION

(11) **EP 4 578 854 A1**
(43) Date of publication of application: **02.07.2025**
(21) Application number: 23856739.0
(22) Date of filing: 26.08.2023
(51) Int. Cl.: C07D 417/14, A01N 43/78, A01P 3/00

(54) **COMPOUND CONTAINING CHAIN-SHAPED CARBOXYLIC ACID AMIDE STRUCTURE, PREPARATION METHOD THEREFOR, USE THEREOF, AND BACTERICIDE**

(30) Priority: 26.08.2022 CN 202211035626
(71) Applicant: Jiangsu Flag Chemical Industry Co., Ltd., Nanjing, Jiangsu 210047 (CN)
(72) Inventor: YANG, Guangfu, Wuhan, Hubei 430079 (CN); ZHOU, Liming, Wuhan, Hubei 430079 (CN); CHENG, Chuanxiang, Wuhan, Hubei 430079 (CN); CHEN, Mengdi, Wuhan, Hubei 430079 (CN); LI, Honghao, Wuhan, Hubei 430079 (CN); CHAI, Song, Wuhan, Hubei 430079 (CN); ZHANG, Pu, Nanjing, Jiangsu 210047 (CN); XIONG, Zi, Nanjing, Jiangsu 210047 (CN); WU, Yaojun, Nanjing, Jiangsu 210047 (CN)
(74) Representative: reuteler & cie SA
(86) International application number: PCT/CN2023/115117
(87) International publication number: WO 2024/041654

(57) **Abstract**

The present invention relates to the field of pesticide bactericides. Disclosed are a compound containing a chain-shaped carboxylic acid amide structure, a preparation method therefor, use thereof, and a bactericide. The compound has a structure as represented by formula (I). The compound of the present invention has an excellent prevention and treatment effect on plant diseases caused by oomycetes such as cucumber downy mildew, phytophthora infestans, and phytophthora capsica, is equivalent to oxathiapiprolin which is a current commercial oomycete disease prevention and treatment agent, and has a very good market development prospect.

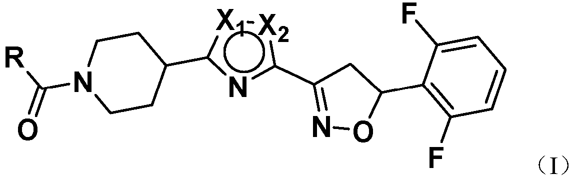

## Description

### Cross Reference to Related Applications

The present application claims the benefit of Chinese patent application 202211035626.7, filed 26/08/2022, the contents of which are incorporated herein by reference.

### Field of the Invention

The present invention relates to the field of pesticide bactericides, in particular to a compound containing a chain-shaped carboxylic acid amide structure, a preparation method therefor, use thereof, and a bactericide.

### Background of the Invention

Oomycetes are one of the important pathogens causing plant diseases, and have the characteristics of wide parasitic range, strong destructiveness, large harmfulness, rapid development and the like.

Downy mildew fungus is one of the most representative oomycetes, which has a short incubation period and can be infected for multiple times, and can cause a disease pandemic in a very short time, for example, cucumber downy mildew caused by the pathogen can cause the death of all cucumbers in just a few days.

Plant diseases caused by downy mildew can be generally divided into the following three categories: 1) leaf diseases, mainly downy mildew; 2) root and crown diseases of annual and perennial crops, such as soil wetness, seedling blight, root, neck and stem rot; 3) systemic disease, refers to disease caused by infection of the roots through soil or seeds, distribution of pathogens in the vascular system of plants, and manifestation of symptoms at the growing points or leaves.

In temperate and tropical climates, most annual or perennial agricultural, horticultural and ornamental crops (such as grapes, cucumbers, potatoes, tobacco, tomatoes, hops, citrus, sunflowers, vegetables and soybeans) are likely to be attacked by downy mildew, which makes downy mildew a very destructive plant pathogen that causes diseases that are difficult to control and thus cause significant losses to agricultural production. Effective control of downy mildew has long been a priority.

The control of oomycete diseases is increasingly difficult, at present, chemical control is still the most convenient and effective method in control measures, and a protective bactericide with multiple action sites and a systemic bactericide with single action site are mainly used in production. However, with the prolonged and long-term abuse of these fungicides, severe resistance has developed in many pathogenic bacteria.

Therefore, the development of new oomycete fungicides that are not cross-resistant with traditional fungicides is an urgent development direction in this field.

### Summary of the Invention

The object of the present invention is to overcome the aforementioned drawbacks of the prior art and to provide a new class of oomycete fungicides that are not cross-resistant to conventional fungicides.

In order to achieve the above object, a first aspect of the present invention provides a compound containing a chain-shaped carboxylic acid amide structure, which has a structure represented by formula (I), or an agrochemically acceptable salt, hydrate and solvate thereof, 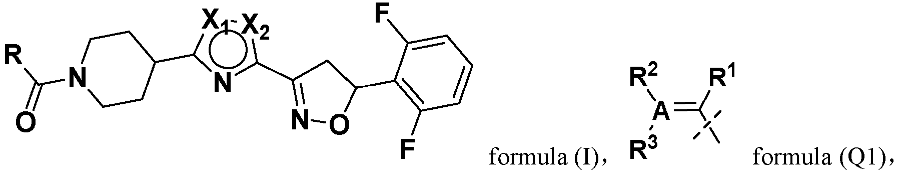 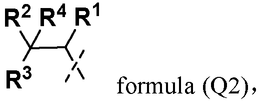 wherein, in formula (I), one of X₁ and X₂ is S, and the other is CH;
R is a structure represented by formula (Q1) or a structure represented by formula (Q2);
in formula (Q1), R¹, R², R³ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; or, R² and R³ are ring-combined together to form a 3-7 membered saturated heterocyclic group containing at least one O atom as a ring-forming atom, which is unsubstituted or substituted with at least one group from the combination A; A is N or CH; and when A is N, one of R² and R³ is absent;
in formula (Q2), R¹, R², R³, R⁴ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; and at least one of R¹, R², R³, R⁴ is alkoxy of C₁-C₁₂ or alkylthio of C₁-C₁₂; or, R² and R³ are ring-combined together to form a 3-7 membered saturated heterocyclic group containing at least one O atom as a ring-forming atom, which is unsubstituted or substituted with at least one group from the combination A;
the combination A consists of alkyl of C₁-C₁₂, halogen, alkyl of C₁-C₁₂ substituted with at least one halogen, alkoxy of C₁-C₁₂.

A second aspect of the present invention provides a method for producing the compound containing a chain-shaped carboxylic acid amide structure or an agrochemically acceptable salt, hydrate and solvate thereof described in the first aspect, the method comprises: under condensation reaction conditions, a compound shown in formula (II) and a compound shown in formula (III) are subjected to contact reaction, 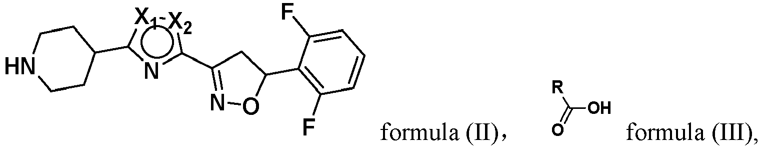 and the definition of substituents in the compound represented by formula (II) and the compound represented by formula (III) is the same as that in the first aspect.

A third aspect of the present invention provides the use of the compound containing a chain-shaped carboxylic acid amide structure, or an agrochemically acceptable salt, hydrate, and solvate thereof described in the first aspect in controlling plant oomycete diseases.

A fourth aspect of the present invention provides the use of the compound containing a chain-shaped carboxylic acid amide structure described in the first aspect or an agrochemically acceptable salt, hydrate and solvate thereof as an agricultural fungicide.

A fifth aspect of the present invention provides a fungicide, the fungicide consists of active ingredients and excipients, the active ingredients comprise the compound containing a chain-shaped carboxylic acid amide structure or an agrochemically acceptable salt, hydrate and solvate thereof described in the first aspect.

The compound of the present invention has an excellent control efficacy against plant diseases caused by oomycetes such as cucumber downy mildew, phytophthora infestans, phytophthora capsici, etc., and is equivalent to the current commercial oomycete disease control agent oxathiapiprolin, and has a good prospect for market development .

### Detailed Description of the Embodiments

The endpoints of the ranges and any values disclosed herein are not limited to the precise range or value, and these ranges or values shall be understood to encompass values close to these ranges or values. For numerical ranges, each range between its endpoints and individual point values, and each individual point value can be combined with each other to give one or more new numerical ranges, and such numerical ranges should be construed as specifically disclosed herein.

The following provides exemplary explanations for some groups of the present invention, unless otherwise specified, unlisted parts shall be interpreted by reference to the following exemplary explanations .

"halogen" means fluorine, chlorine, bromine, iodine.

"alkyl of C₁-C₁₂" refers to an alkyl group with a total number of carbon atoms of 1-12, including a straight-chain alkyl group and branched-chain alkyl group, such as it can be a straight-chain alkyl group or branched alkyl group with a total number of carbon atoms of 1, 2, 3, 4, 5, 6, 7, 8, 9, 10, 11, 12, such as it can be n-butyl, isobutyl, tert-butyl, n-pentyl, isopentyl, n-hexyl, etc.; the definition of " alkyl of C₁-C₁₀ " is similar to the definition of " alkyl of C₁-C₁₂ ", except that the total number of carbon atoms is different.

The definition of "alkoxy of C₁-C₁₂" is similar to "alkyl of C₁-C₁₂", except that "alkoxy of C₁-C₁₂" is directly connected to the parent nucleus through an O atom, represents alkoxy groups with a total number of 1-12 carbon atoms, including a straight-chain alkoxy group and branched-chain alkoxy group, for example, it can be a straight-chain alkoxy group or branched-chain alkoxy group with a total number of carbon atoms of 1, 2, 3, 4, 5, 6, 7, 8, 9, 10, 11, 12, illustratively, it can be methoxy, ethoxy, n-propyloxy, isopropyloxy, n-butyloxy, isobutyloxy, tert-butyloxy, n-pentyloxy, isopentyloxy, n-hexyloxy. The definition of "alkoxy of C₁-C₁₀" is similar to the definition of "alkoxy of C₁-C₁₂", except that the total number of carbon atoms is different.

The definition of "alkylthio of C₁-C₁₂" is similar to "alkyl of C₁-C₁₂", except that "alkylthio of C₁-C₁₂" is directly connected to the parent nucleus through an S atom, represents alkylthio groups with a total number of 1-12 total carbon atoms, including a straight-chain alkylthio group and branched-chain alkylthio group, for example, it can be a straight-chain alkylthio group or branched-chain alkylthio group with a total number of carbon atoms of 1, 2, 3, 4, 5, 6, 7, 8, 9, 10, 11, 12, illustratively, it can be methylthio, ethylthio, n-propylthio, isopropylthio, n-butylthio, isobutylthio, tert-butylthio, n-pentylthio, isopentylthio, n-hexylthio. The definition of "alkylthio of C₁-C₁₀" is similar to the definition of "alkylthio of C₁-C₁₂", except that the total number of carbon atoms is different.

"cycloalkyl of C₃-C₁₂" means a cycloalkyl group with a total number of carbon atoms of 3-12, and the ring-forming atoms are all C atoms, and for example, it can be a total number of ring forming carbon atoms of 3, 4, 5, 6, 7, 8, 9, 10, 11, 12, and any position on the "cycloalkyl of C₃-C₁₂" that can be replaced is directly connected with the parent nucleus. The definition of "cycloalkyl of C₃-C₁₀" is similar to the definition of "cycloalkyl of C₃-C₁₂", except that the total number of carbon atoms is different.

"alkoxy of C₁-C₁₂ substituted with at least one halogen" means that at least one H atom in "alkoxy of C₁-C₁₂" is substituted by a halogen, and the substituted halogen can be one or more. "alkoxy of C₁-C₁₀ substituted with at least one halogen" has a similar definition, merely differing in the total number of carbon atoms.

"alkyl of C₁-C₁₂ substituted with at least one halogen" means that at least one H atom in "alkyl of C₁-C₁₂" is substituted by a halogen, and the substituted halogen can be one or more. "alkyl of C₁-C₁₀ substituted with at least one halogen" has a similar definition, merely differing in the total number of carbon atoms.

"alkoxy of C₁-C₁₂ substituted with phenyl" means that at least one H atom in "alkoxy of C₁-C₁₂" is substituted by phenyl. "alkoxy of C₁-C₁₀ substituted with phenyl" has a similar definition, merely differing in the total number of carbon atoms.

"R² and R³ are ring-combined together to form a 3-7 membered saturated heterocyclic group containing at least one O atom as a ring-forming atom, which is unsubstituted or substituted with at least one group from the combination A" means that the total number of ring-forming carbon atoms is 3, 4, 5, 6, 7 and which contains at least one O atom.

Unless otherwise specified, a group "substituted with at least one group from the combination A" means that at least one group in the combination A can be substituted at any substitutable positions. The dotted line in the structural formula shown by the group represents the position of the connection between the group and the parent nucleus.

As described above, a first aspect of the present invention provides a compound containing a chain-shaped carboxylic acid amide structure, which has a structure represented by formula (I), or an agrochemically acceptable salt, hydrate and solvate thereof.

According to a preferred concrete embodiment, in formula (I),
one of X₁ and X₂ is S, and the other is CH;
R is a structure represented by formula (Q1); in formula (Q1), R¹, R², R³ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; or, R² and R³ are ring-combined together to form a 3-7 membered saturated heterocyclic group containing at least one O atom as a ring-forming atom, which is unsubstituted or substituted with at least one group from the combination A;
A is N or CH; and when A is N, one of R² and R³ is absent; the combination A consists of alkyl of C₁-C₁₂, halogen, alkyl of C₁-C₁₂ substituted with at least one halogen, alkoxy of C₁-C₁₂.

According to another preferred concrete embodiment, in formula (I), one of X₁ and X₂ is S, and the other is CH;
R is a structure represented by formula (Q1); in formula (Q1), R¹, R², R³ are each independently selected from the group consisting of H, alkyl of C₁-C₁₀, alkoxy of C₁-C₁₀, cycloalkyl of C₃-C₁₀, alkylthio of C₁-C₁₀, alkoxy of C₁-C₁₀ substituted with at least one halogen, alkoxy of C₁-C₁₀ substituted with phenyl, halogen, phenyl, phenyl substituted with at least one group of combination A, cyano, nitro, pyridyl, pyrazolyl, pyrazolyl substituted with at least one group of combination A; or, R² and R³ are ring-combined together to form a 3-7 membered saturated heterocyclic group containing at least one O atom as a ring-forming atom, which is unsubstituted or substituted with at least one group from the combination A; A is N or CH; and when A is N, one of R² and R³ is absent;
the combination A consists of alkyl of C₁-C₁₀, halogen, alkyl of C₁-C₁₀ substituted with at least one halogen, alkoxy of C₁-C₁₀.

According to a particularly preferred concrete embodiment, the compound of the structure shown in formula (I) is selected from any one of the following:

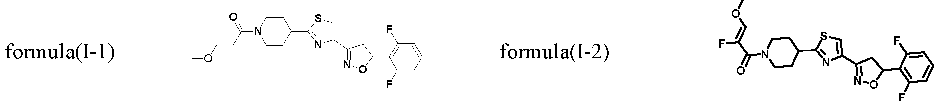

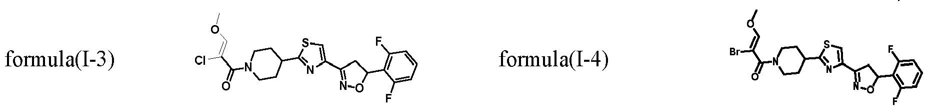

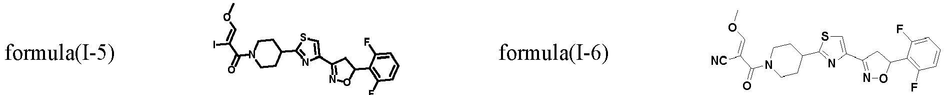

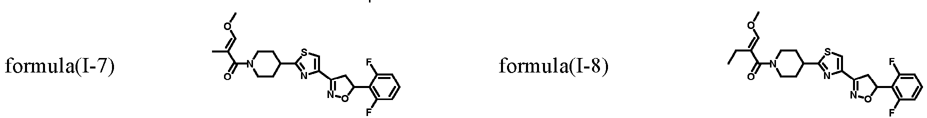

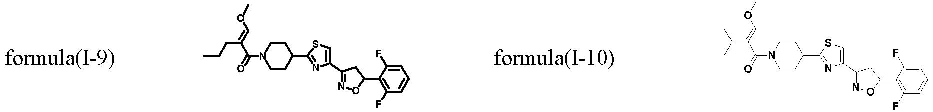

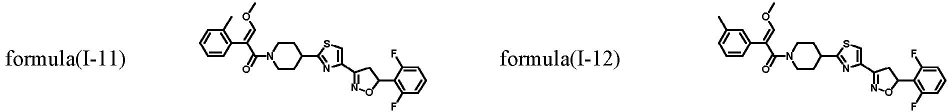

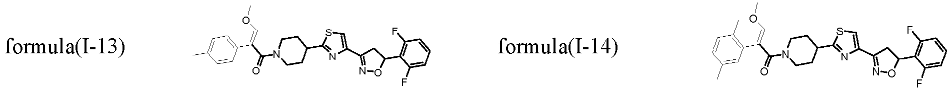

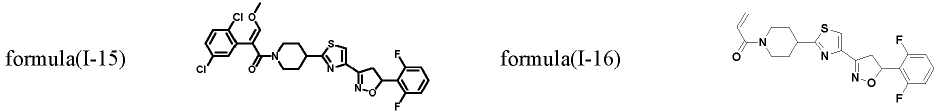

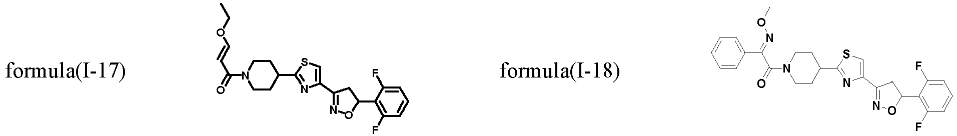

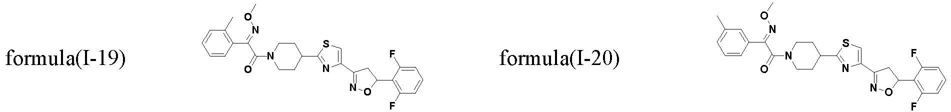

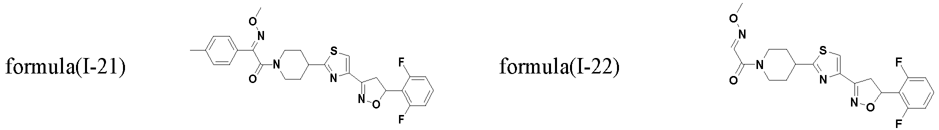

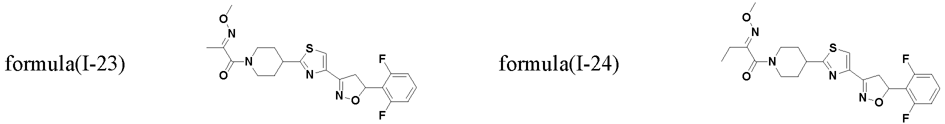

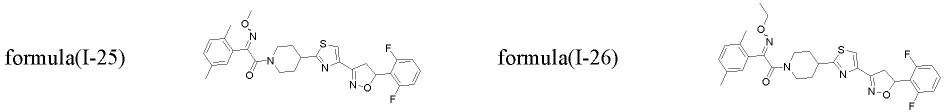

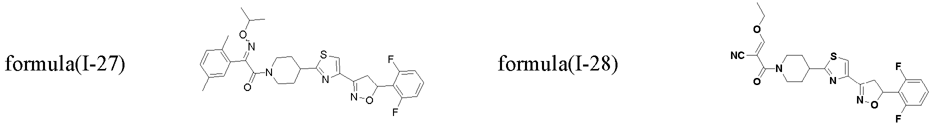

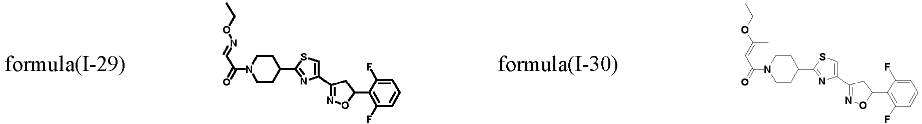

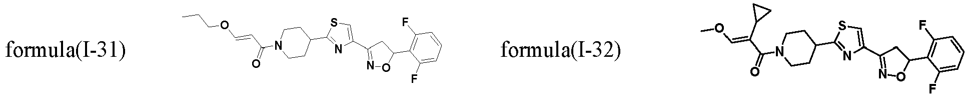

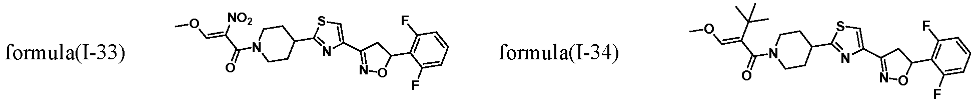

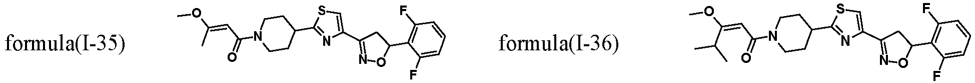

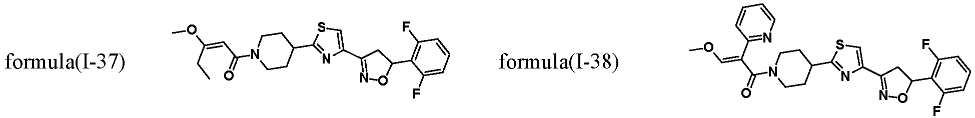

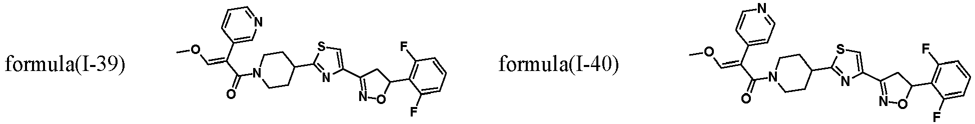

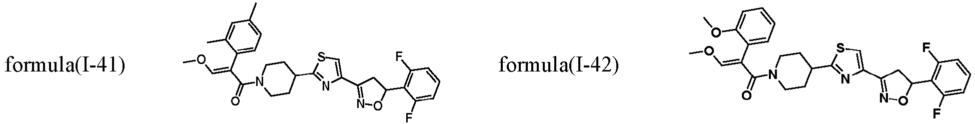

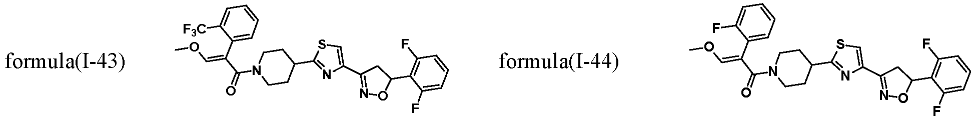

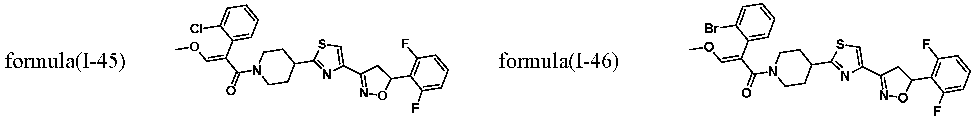

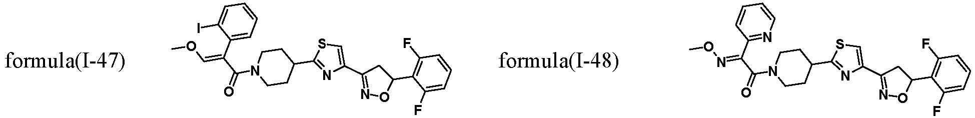

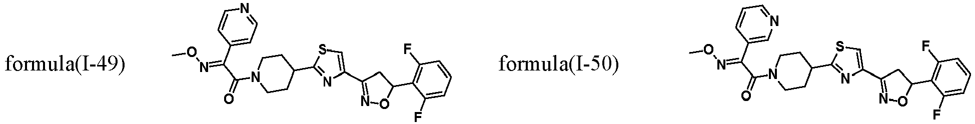

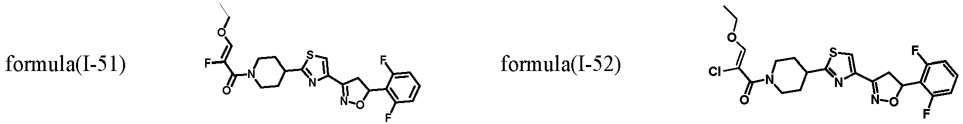

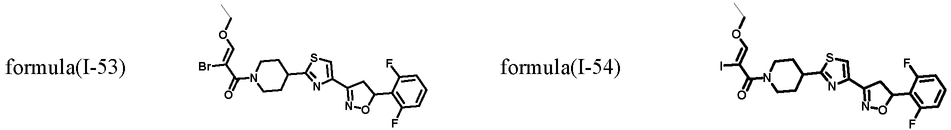

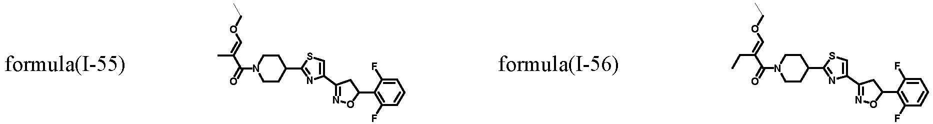

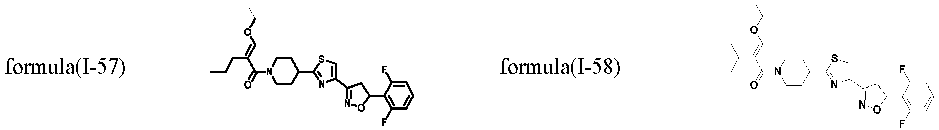

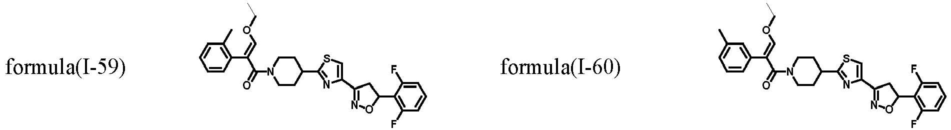

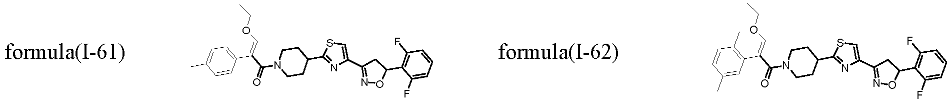

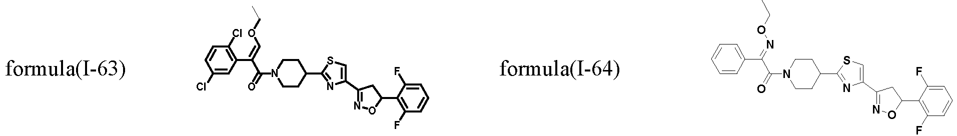

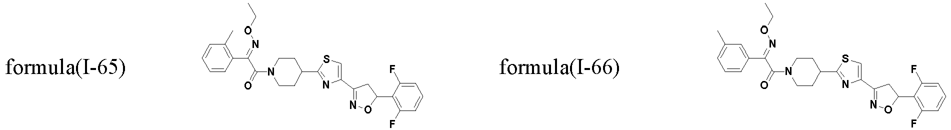

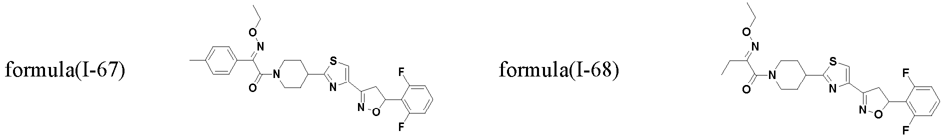

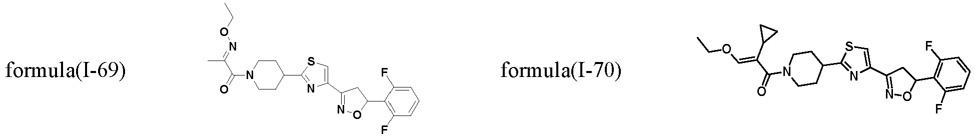

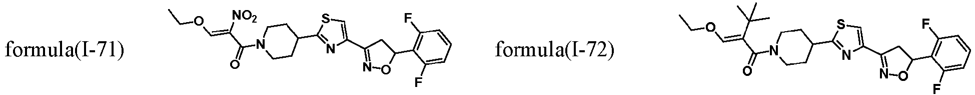

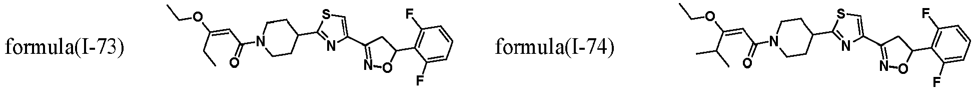

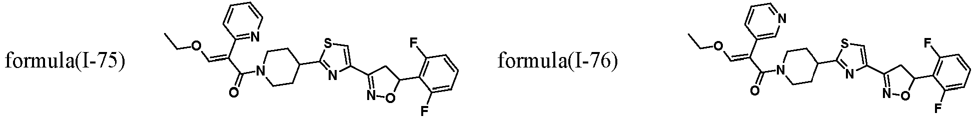

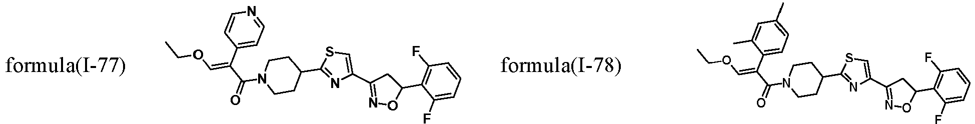

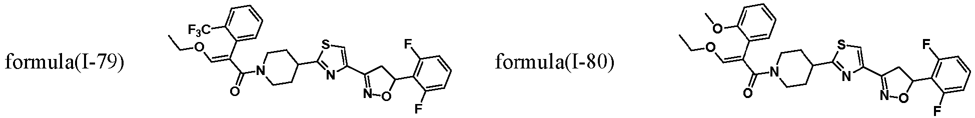

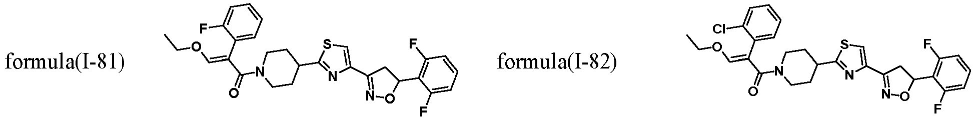

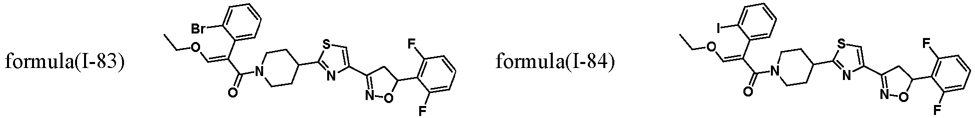

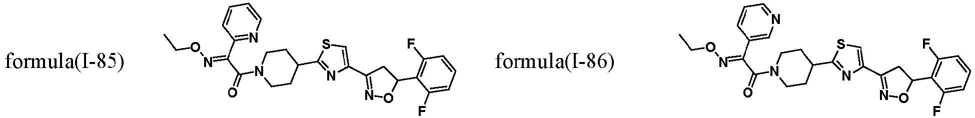

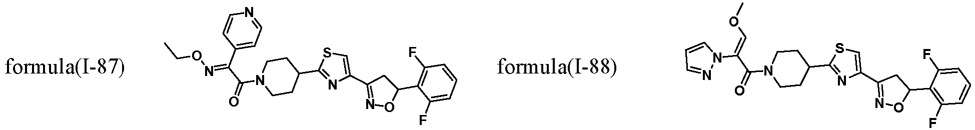

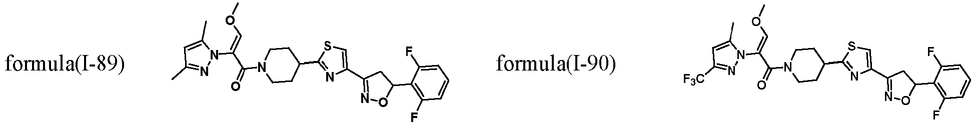

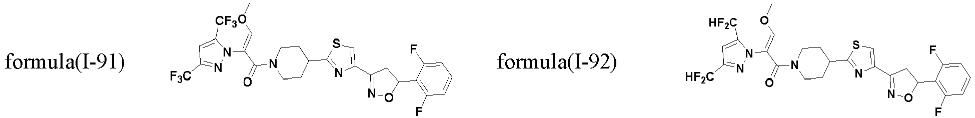

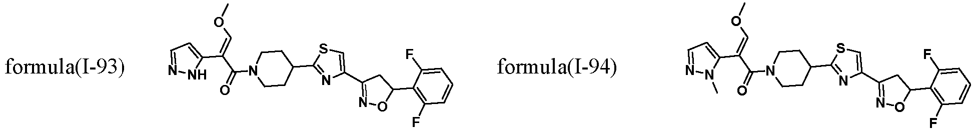

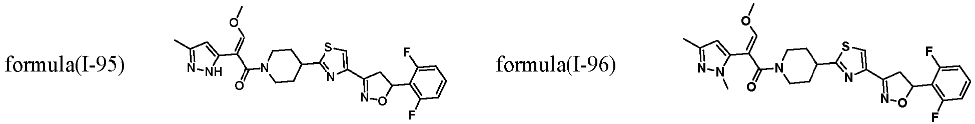

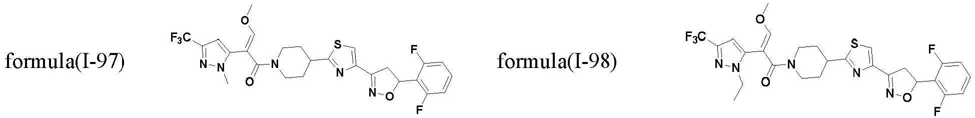

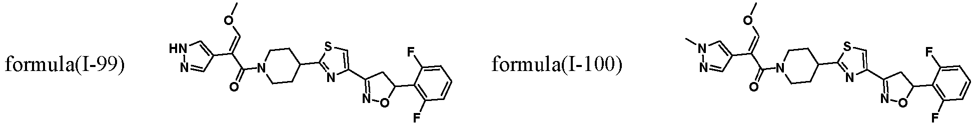

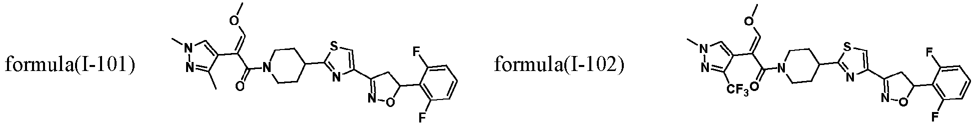

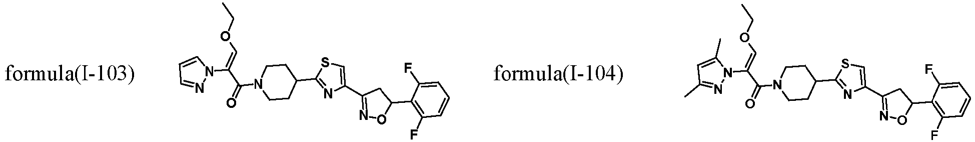

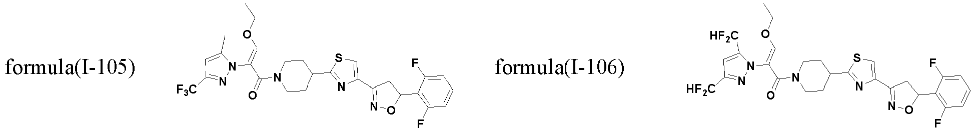

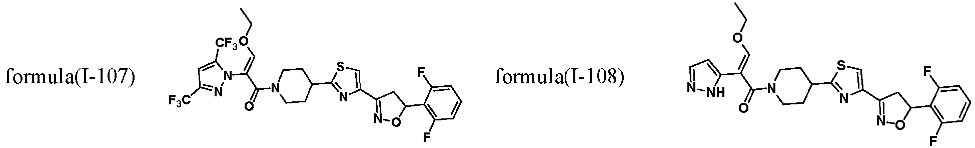

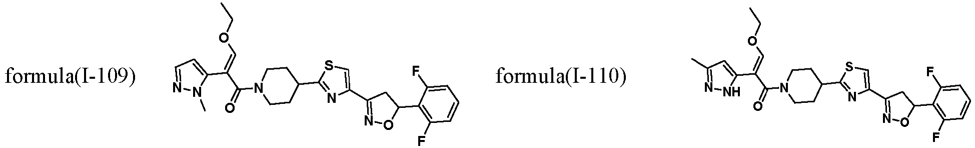

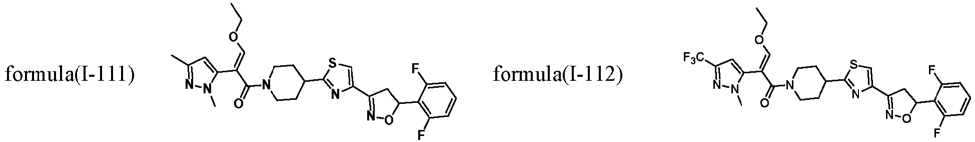

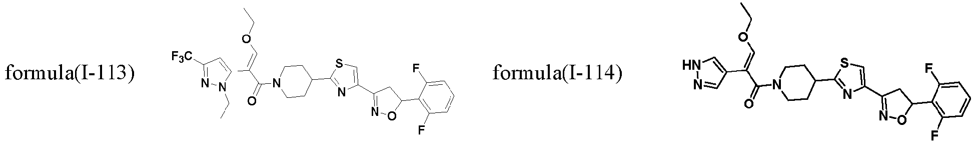

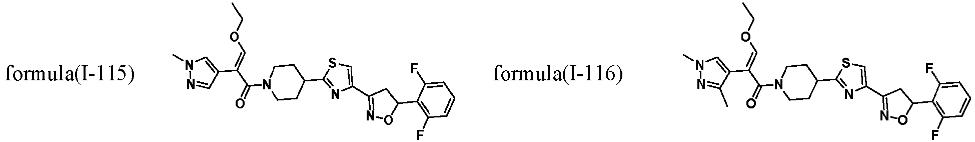

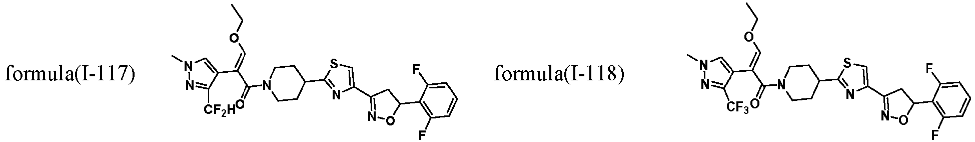

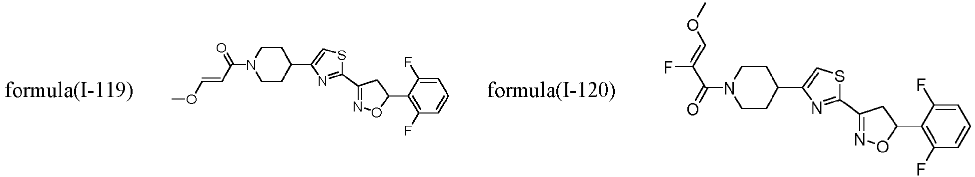

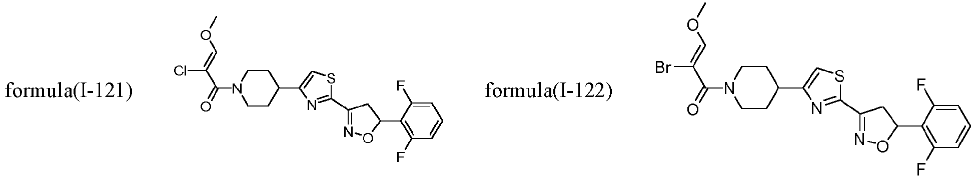

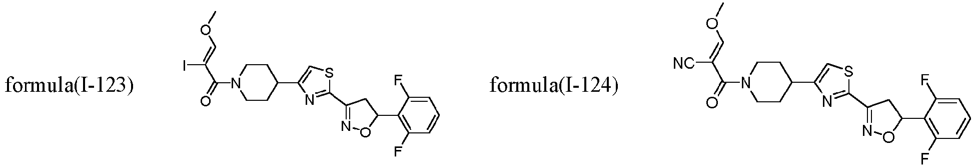

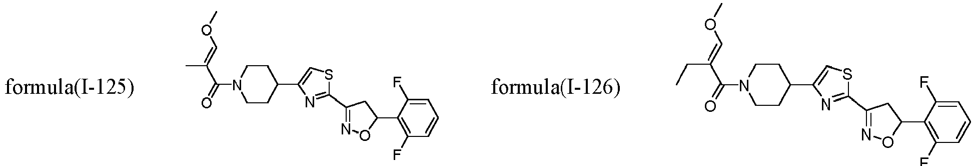

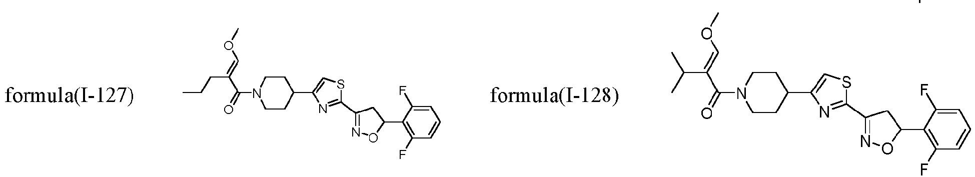

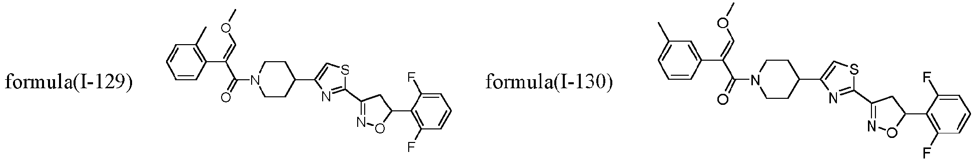

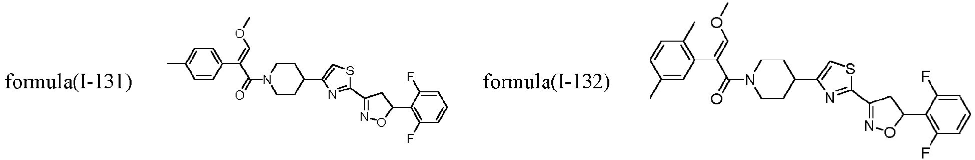

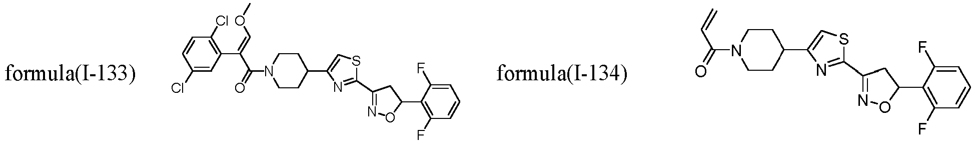

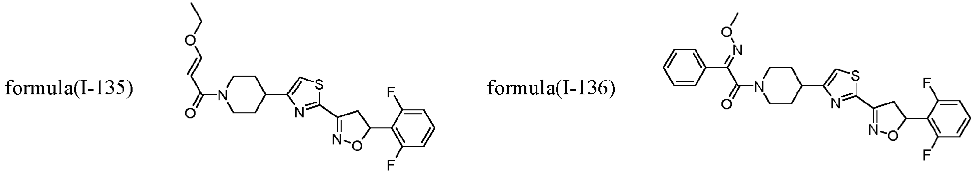

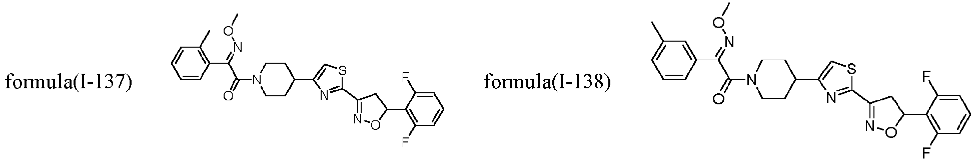

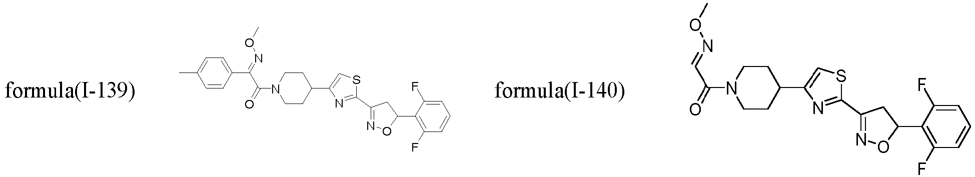

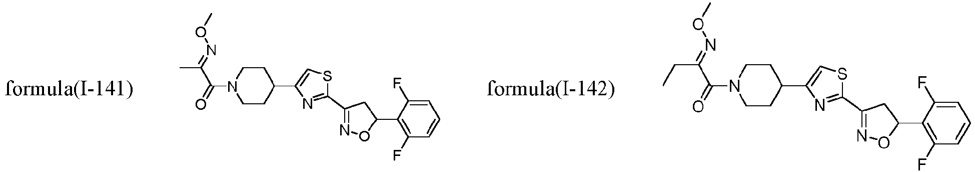

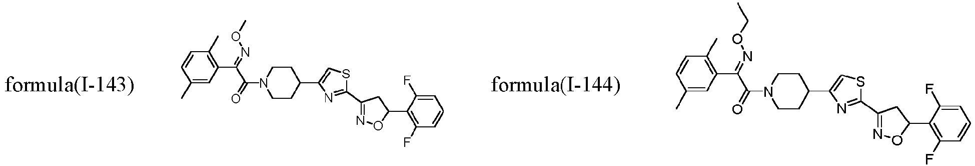

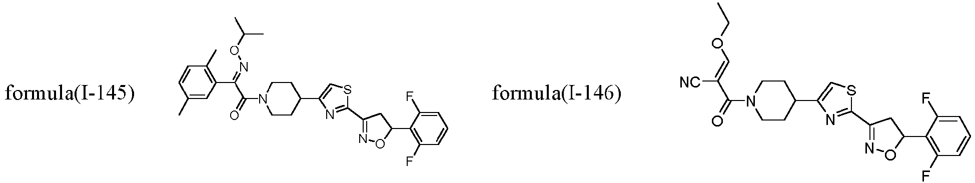

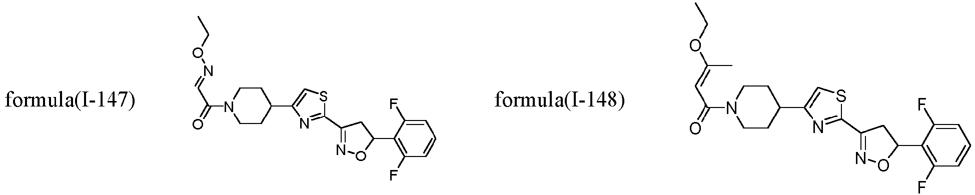

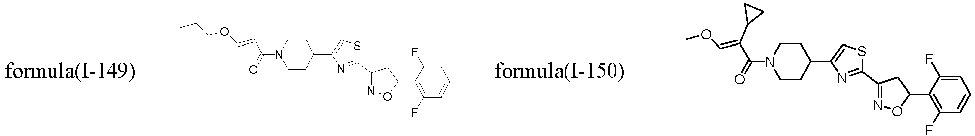

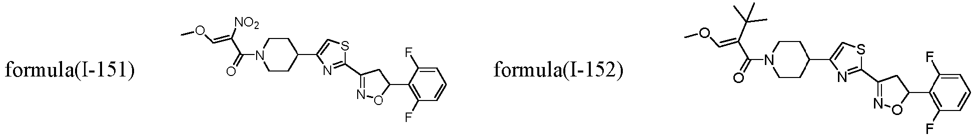

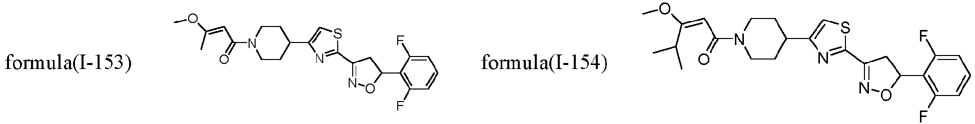

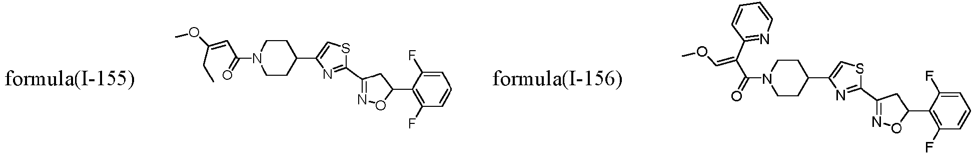

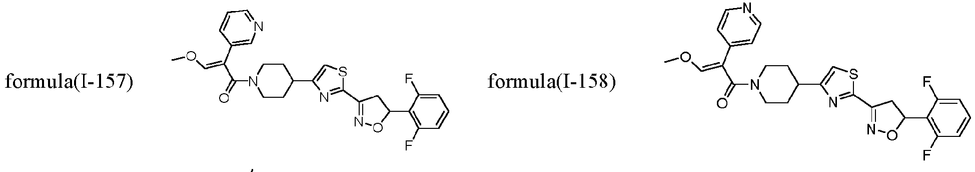

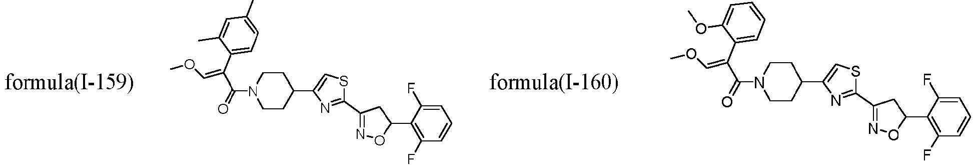

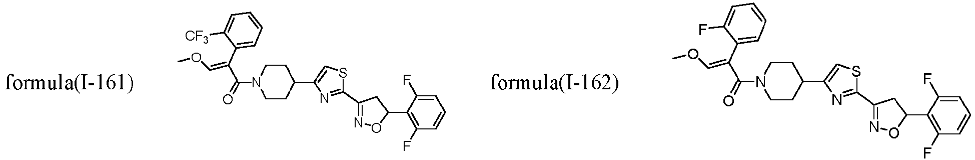

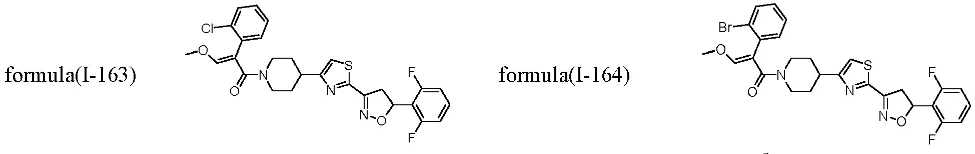

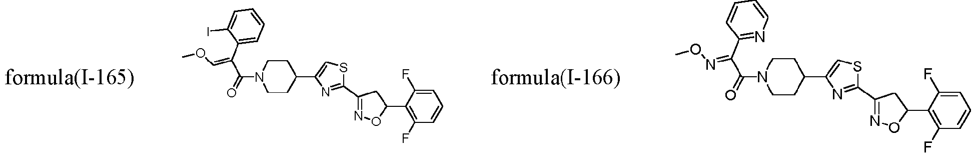

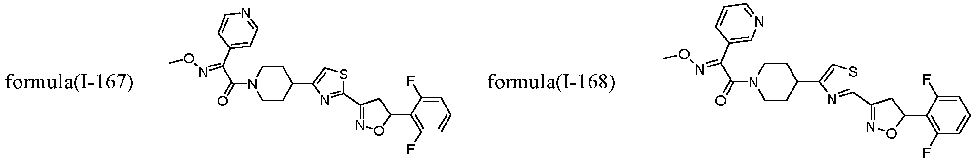

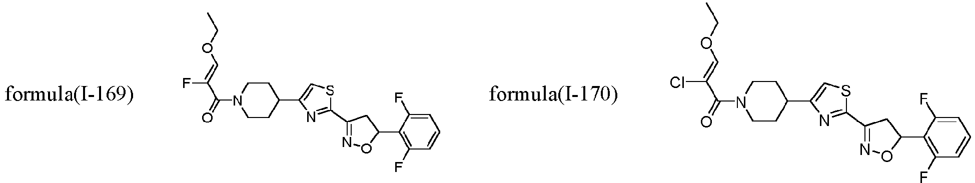

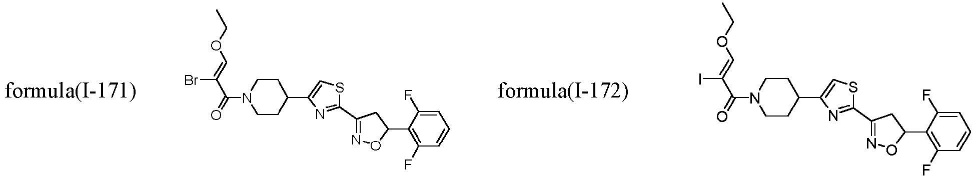

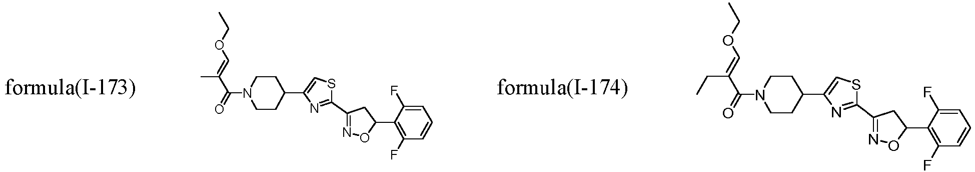

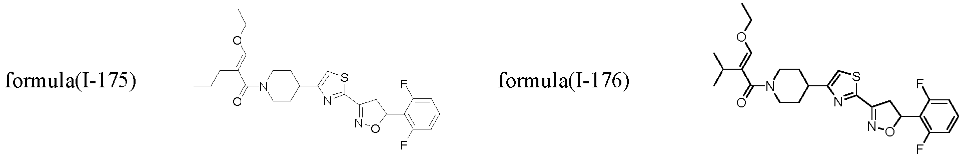

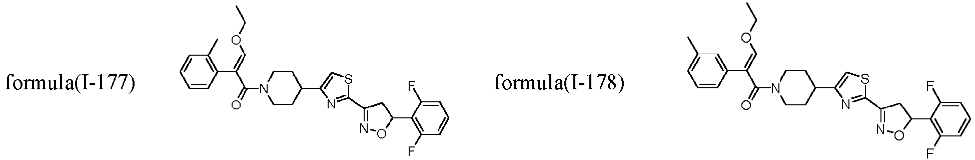

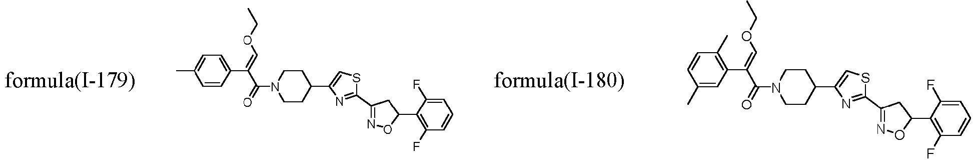

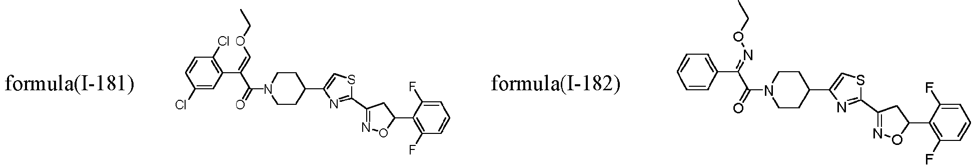

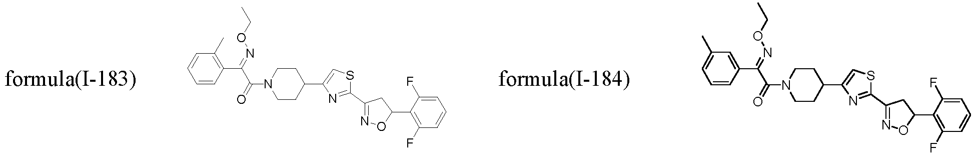

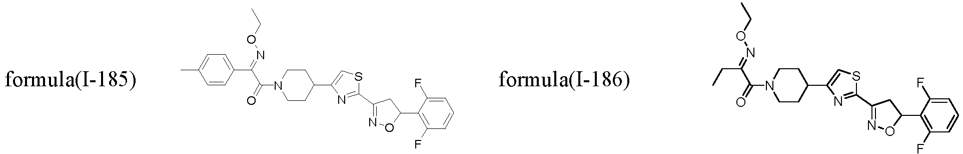

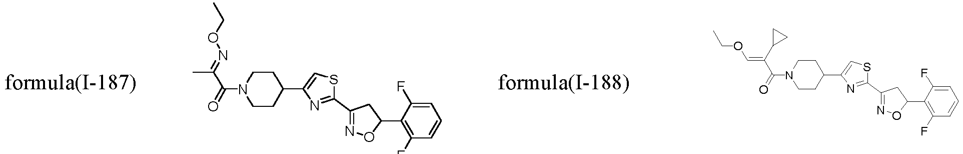

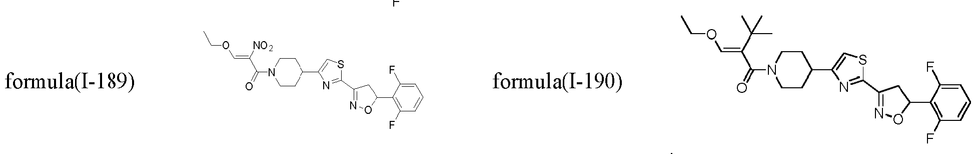

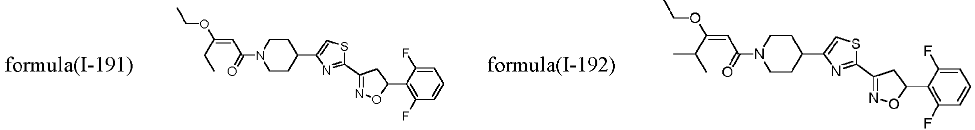

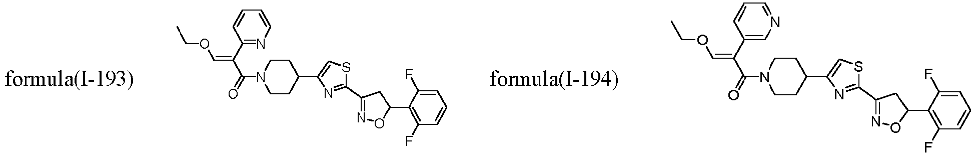

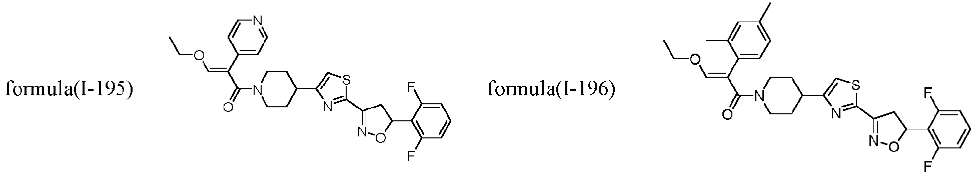

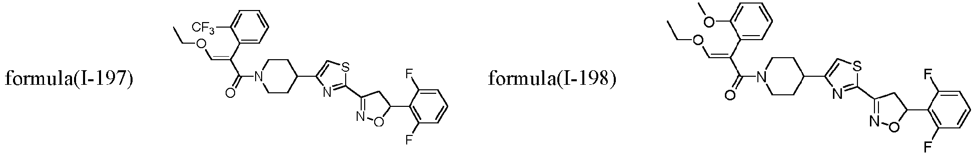

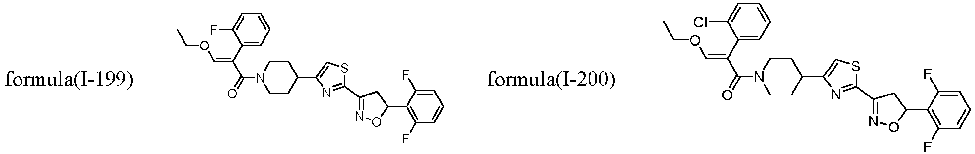

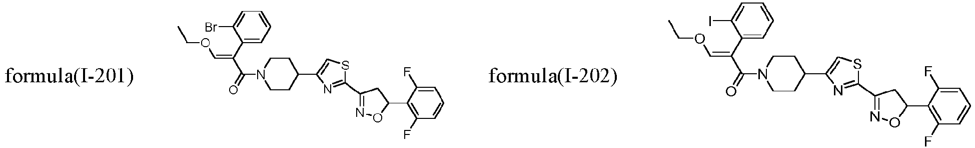

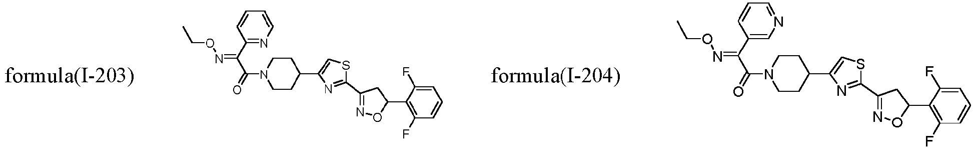

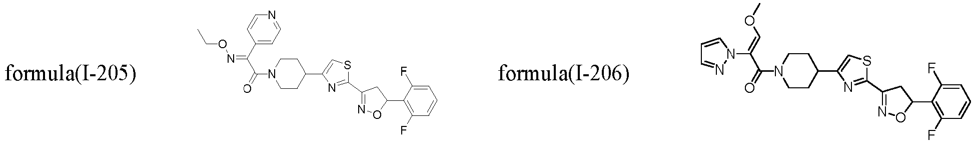

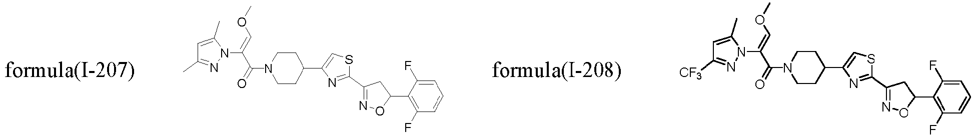

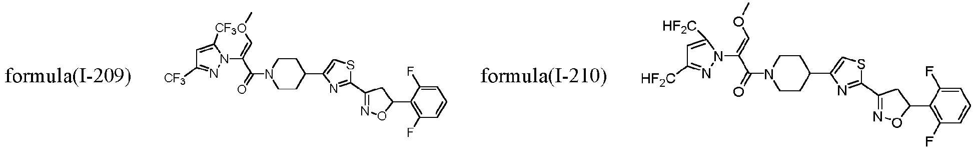

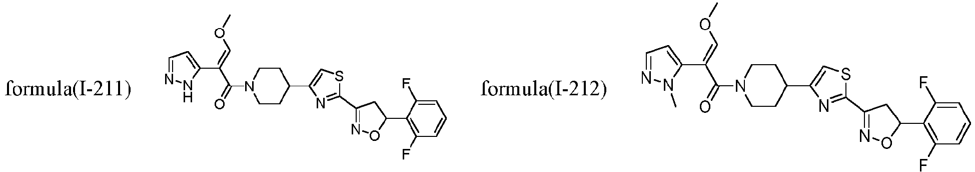

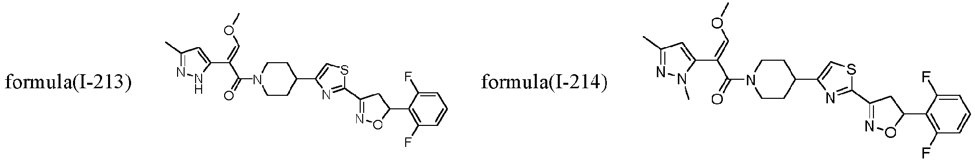

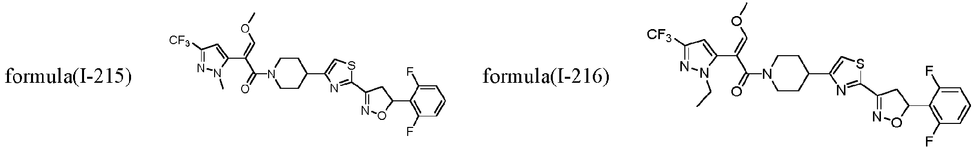

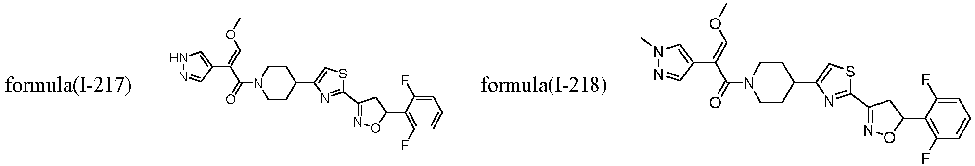

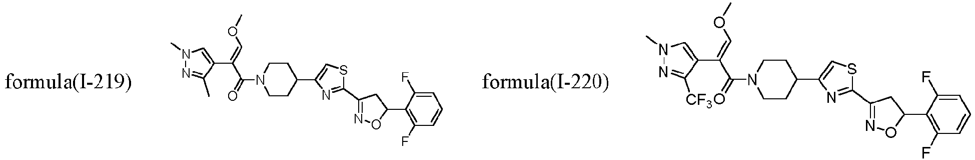

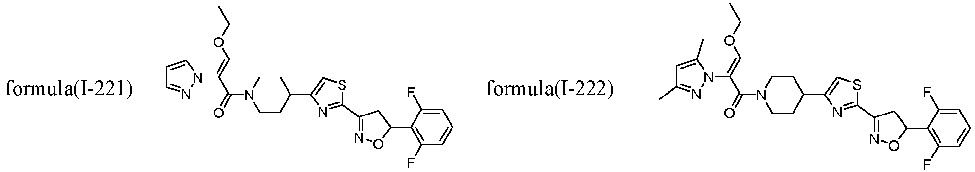

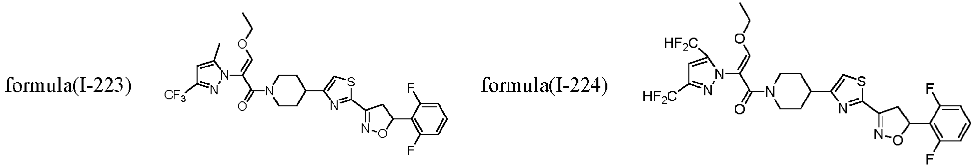

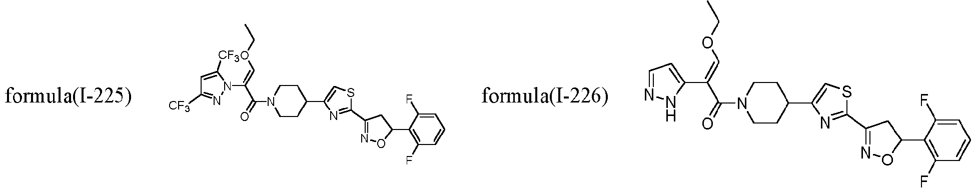

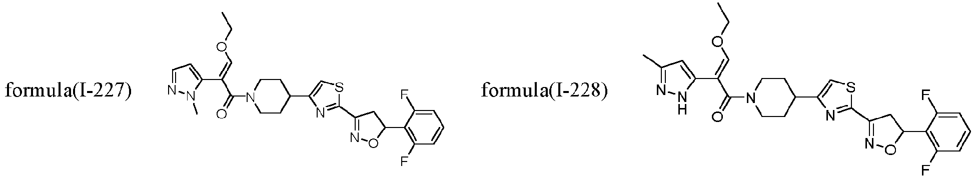

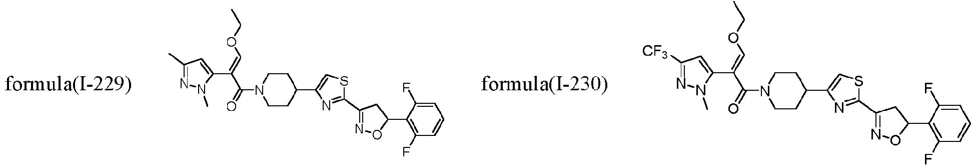

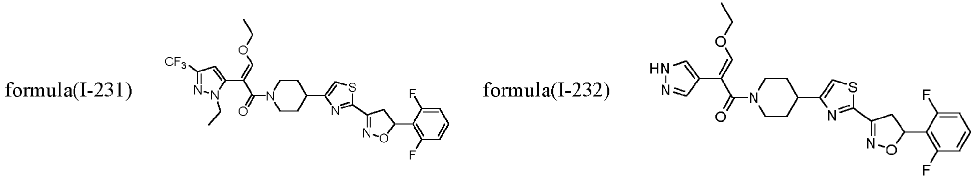

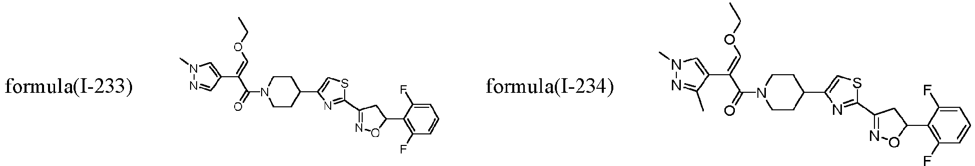

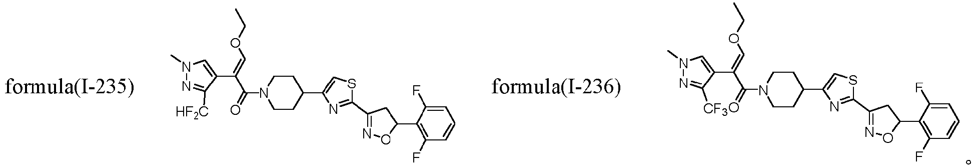

According to a preferred embodiment, in formula (I),
one of X₁ and X₂ is S, and the other is CH;
R is a structure represented by formula (Q2); R¹, R², R³, R⁴ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; and at least one of R¹, R², R³, R⁴ is alkoxy of C₁-C₁₂ or alkylthio of C₁-C₁₂; or, R² and R³ are ring-combined together to form a 3-7 membered saturated heterocyclic group containing at least one O atom as a ring-forming atom, which is unsubstituted or substituted with at least one group from the combination A;
the combination A consists of alkyl of C₁-C₁₂, halogen, alkyl of C₁-C₁₂ substituted with at least one halogen, alkoxy of C₁-C₁₂.

According to another preferred embodiment, in formula (I), one of X₁ and X₂ is S, and the other is CH;
R is a structure represented by formula (Q2); R¹, R², R³, R⁴ are each independently selected from the group consisting of H, alkyl of C₁-C₁₀, alkoxy of C₁-C₁₀, cycloalkyl of C₃-C₁₀, alkylthio of C₁-C₁₀, alkoxy of C₁-C₁₀ substituted with at least one halogen, alkoxy of C₁-C₁₀ substituted with phenyl, halogen, phenyl, phenyl substituted with at least one group of combination A, cyano, nitro, pyridyl, pyrazolyl, pyrazolyl substituted with at least one group of combination A; and at least one of R¹, R², R³, R⁴ is alkoxy of C₁-C₁₀ or alkylthio of C₁-C₁₀; or, R² and R³ are ring-combined together to form a 3-7 membered saturated heterocyclic group containing at least one O atom as a ring-forming atom, which is unsubstituted or substituted with at least one group from the combination A; the combination A consists of alkyl of C₁-C₁₀, halogen, alkyl of C₁-C₁₀ substituted with at least one halogen, alkoxy of C₁-C₁₀.

According to another particularly preferred concrete embodiment, the compound of the structure shown in formula (I) is selected from any one of the following:

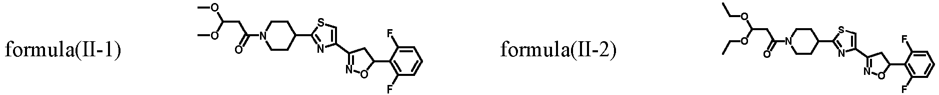

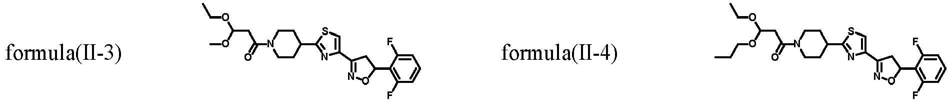

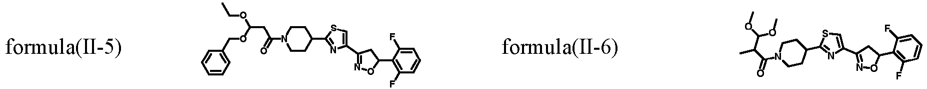

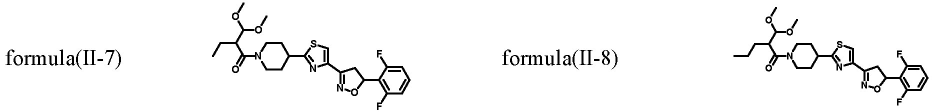

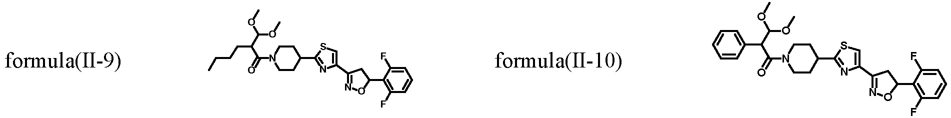

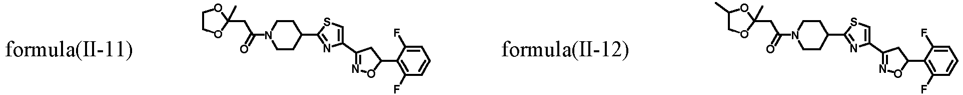

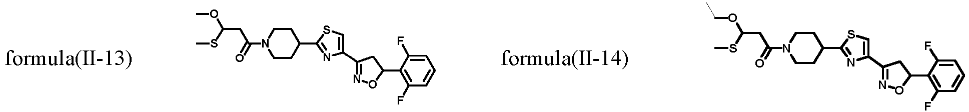

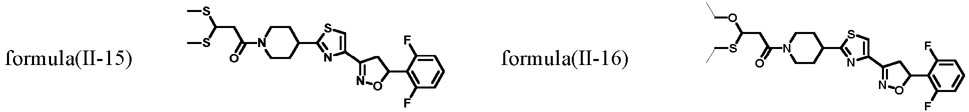

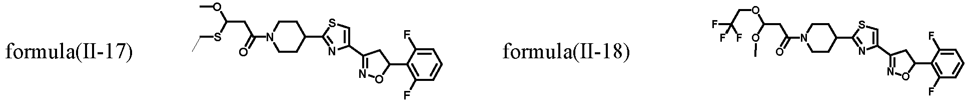

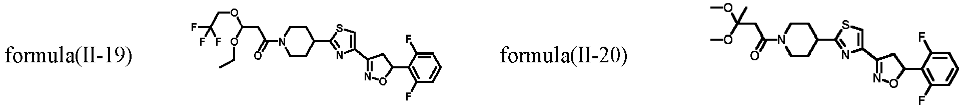

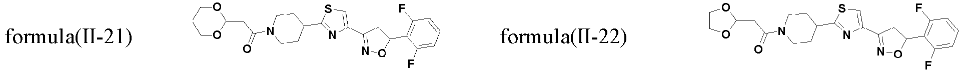

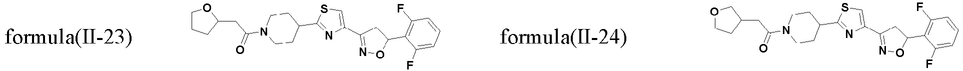

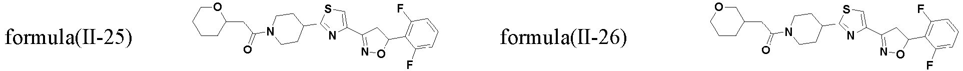

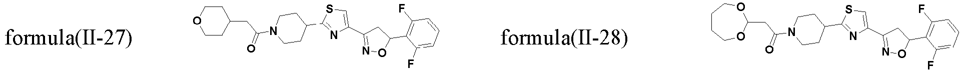

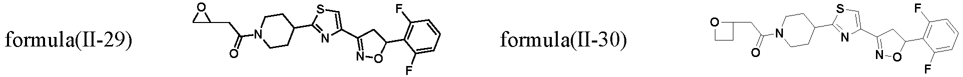

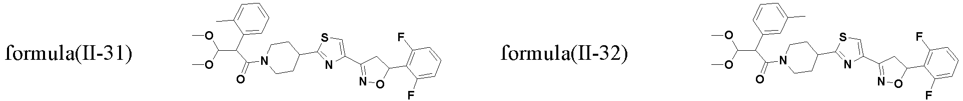

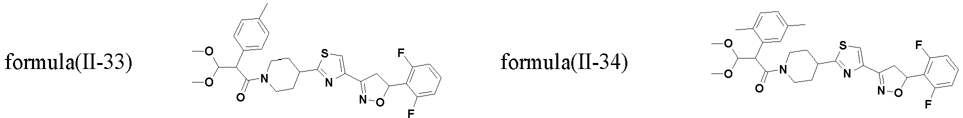

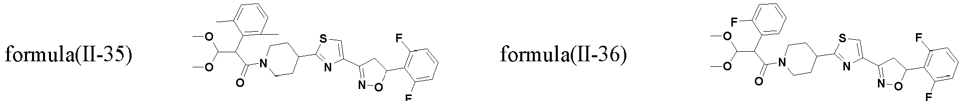

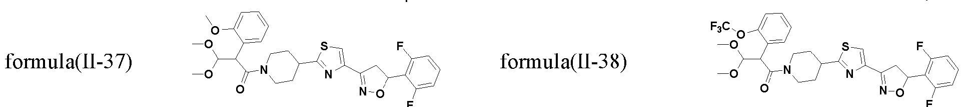

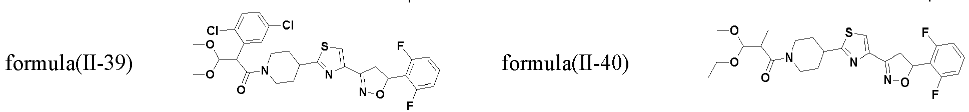

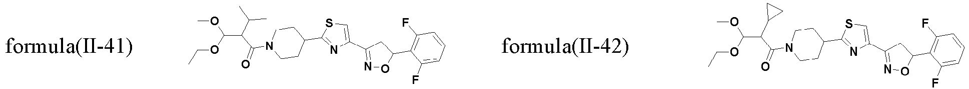

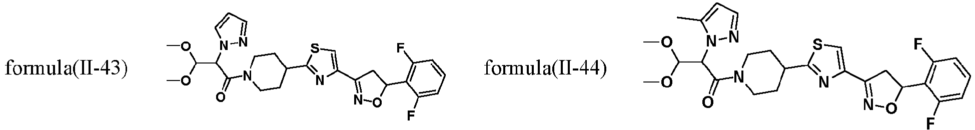

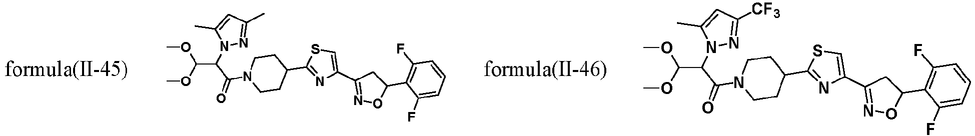

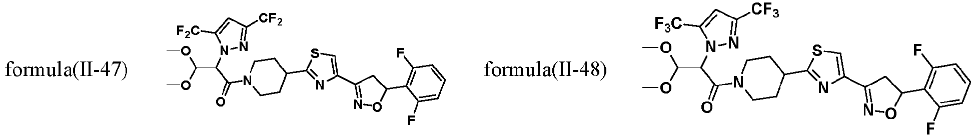

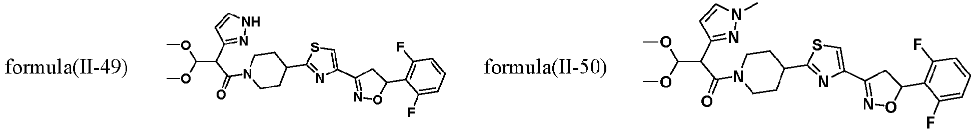

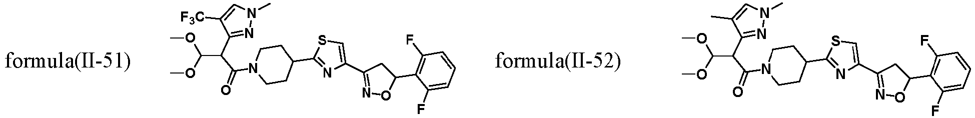

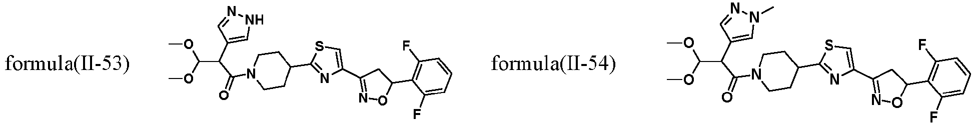

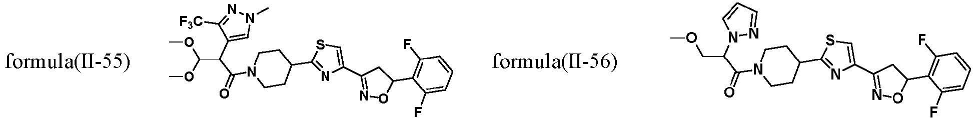

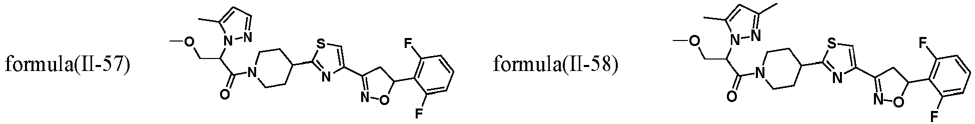

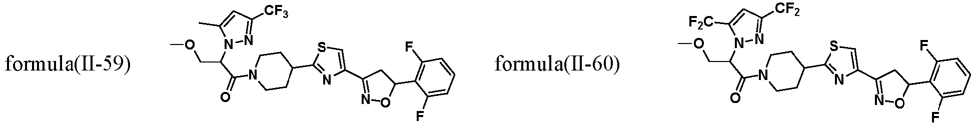

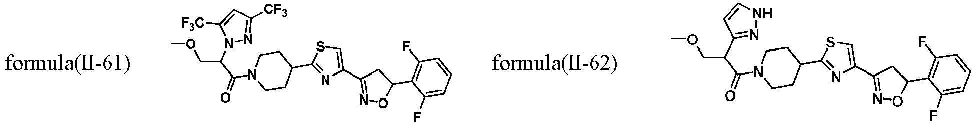

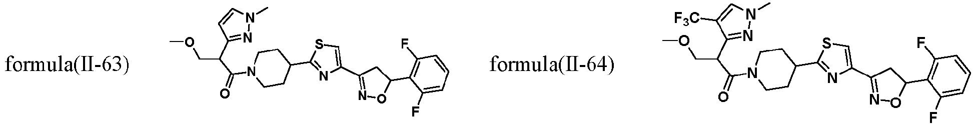

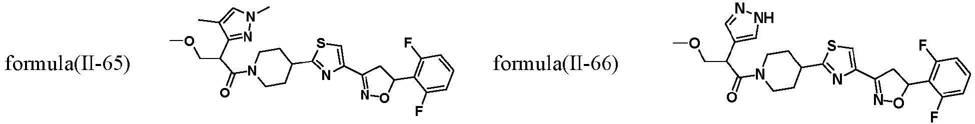

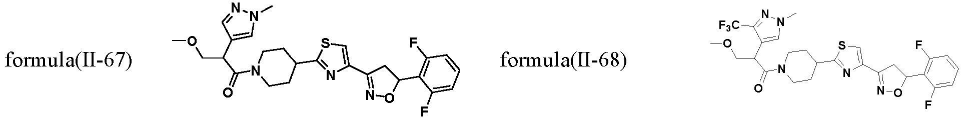

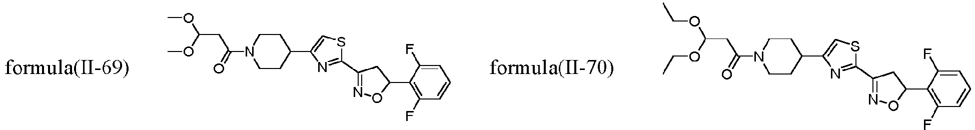

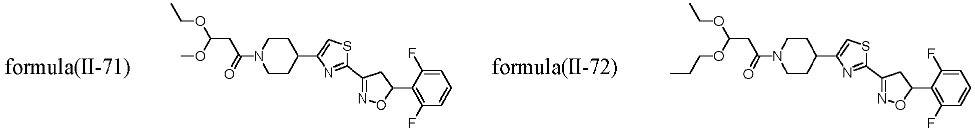

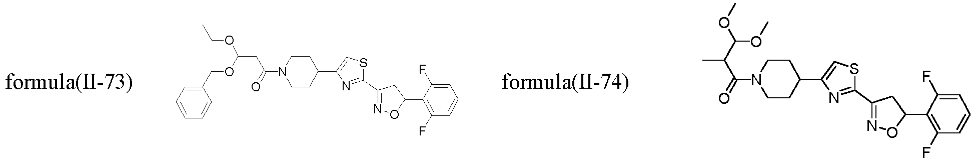

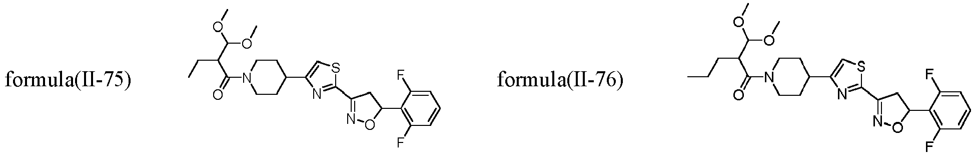

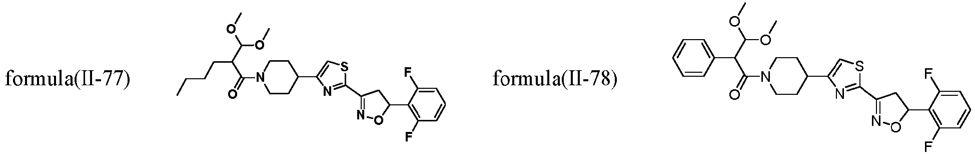

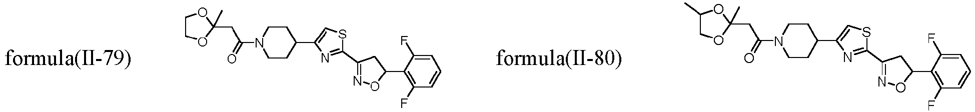

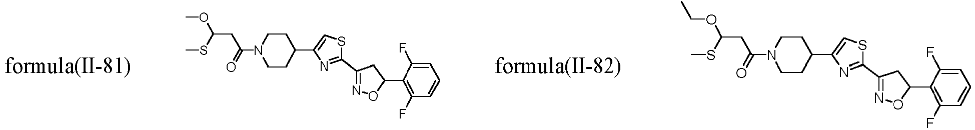

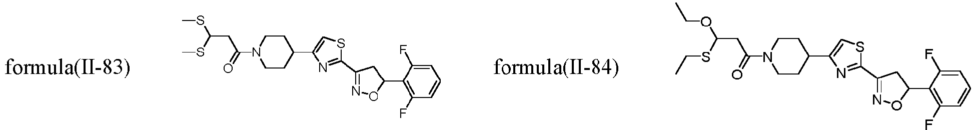

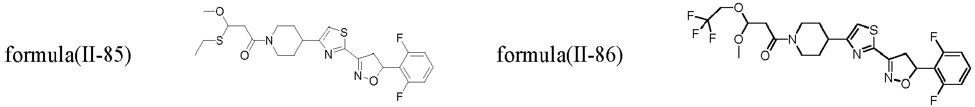

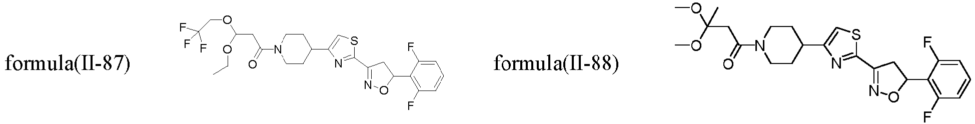

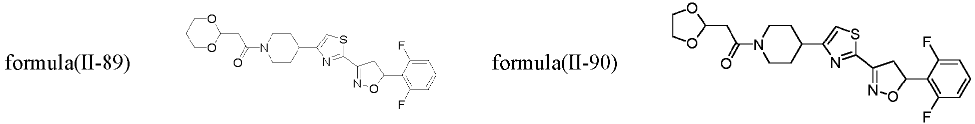

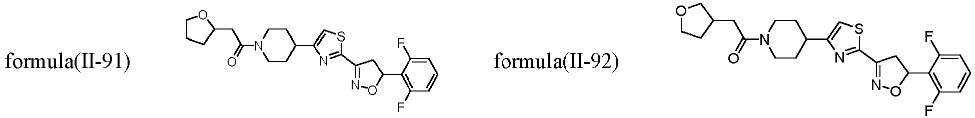

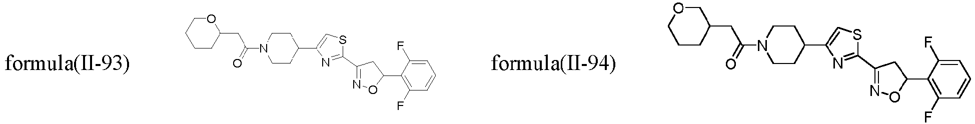

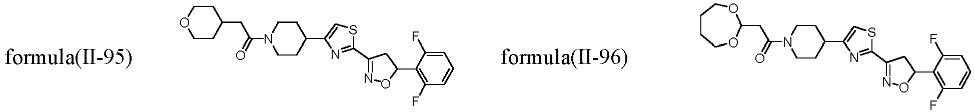

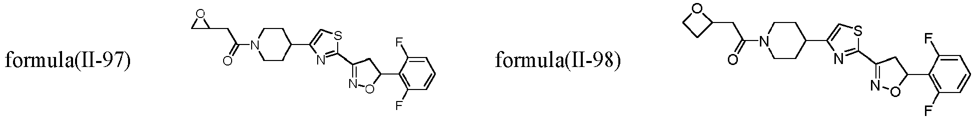

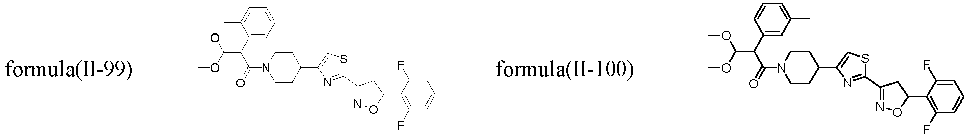

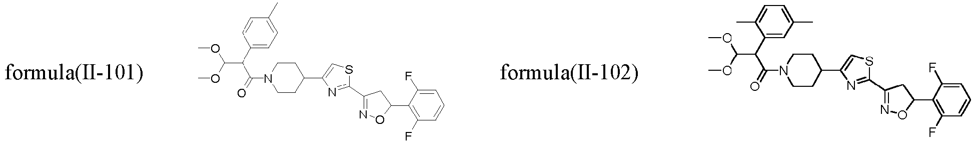

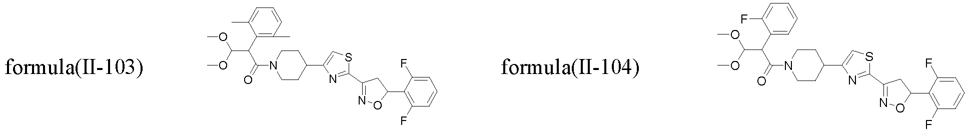

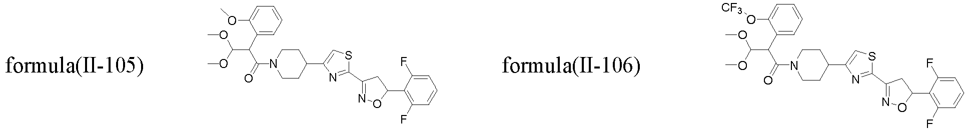

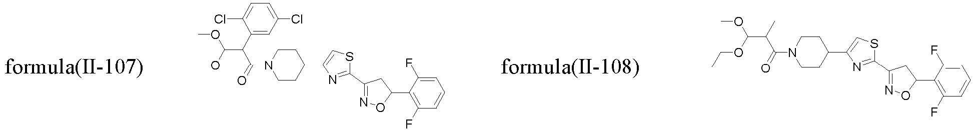

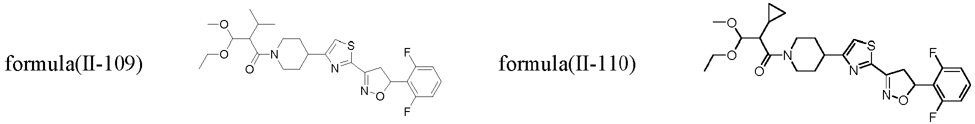

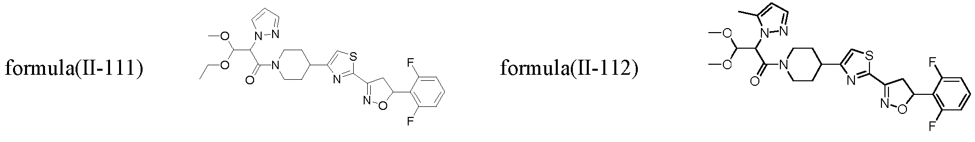

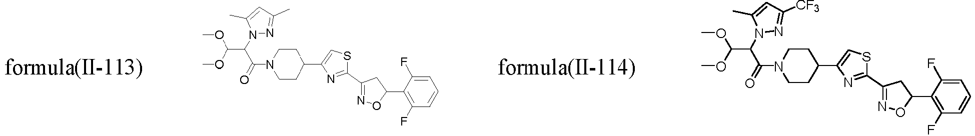

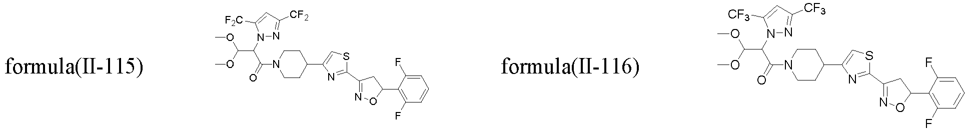

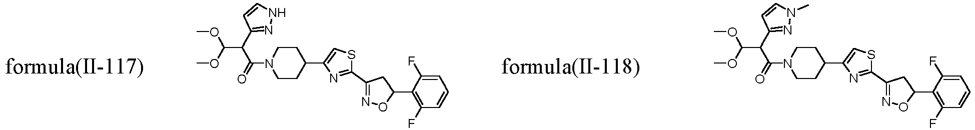

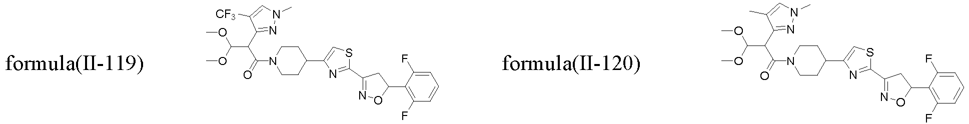

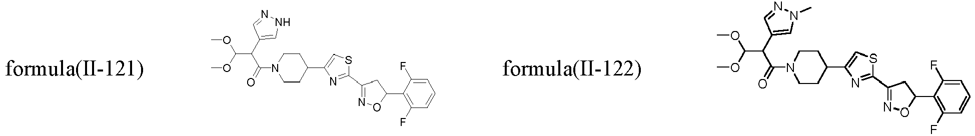

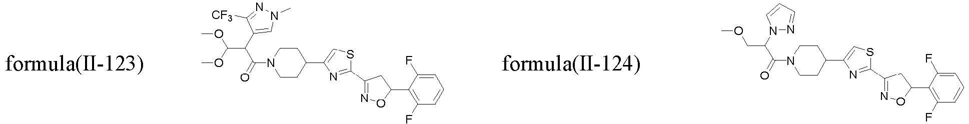

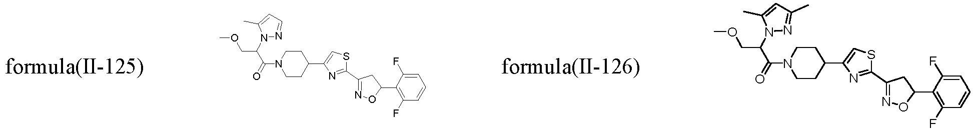

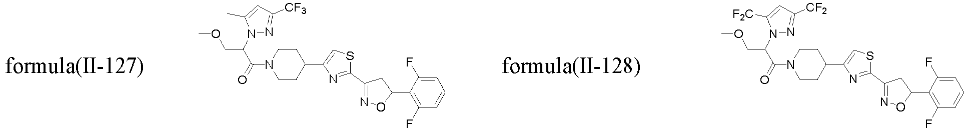

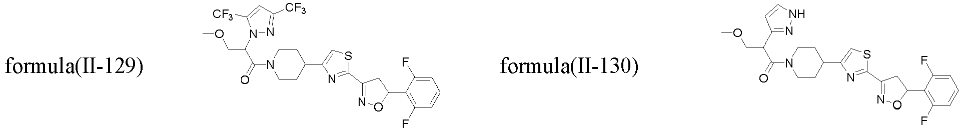

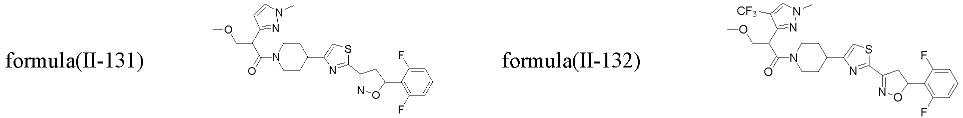

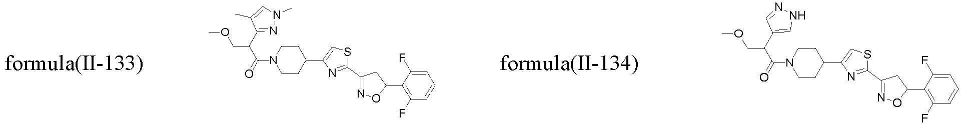

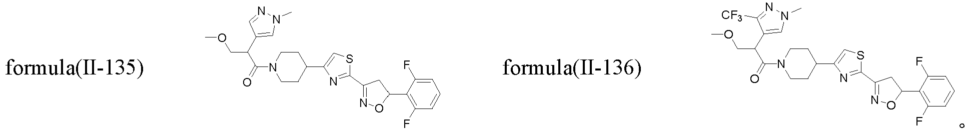

There is no particular limitation on the stereostructure of the compound shown in formula (I) in the present invention. The compound shown in formula (I) can exist in different stereoisomers or optical isomers or tautomers, and the present invention includes all stereoisomers or optical isomers or tautomers and mixtures of various ratios thereof.

Any asymmetric atom (e.g., carbon, etc.) of a compound disclosed in the present invention can exist in racemic or enantiomerically enriched forms, such as (R) -, (S) -or (R, S) -configuration.

The present invention does not particularly limit the method for producing the compound containing a chain-shaped carboxylic acid amide structure. A person skilled in the art can produce the compound containing a chain-shaped carboxylic acid amide structure by combining the features of the structural formulae with a known method in the field of organic synthesis, but, in order to obtain the compound containing a chain-shaped carboxylic acid amide structure of the present invention or an agrochemically acceptable salt, hydrate and solvate thereof in a higher yield, the present invention provides the method for producing the compound containing a chain-shaped carboxylic acid amide structure or an agrochemically acceptable salt, hydrate and solvate thereof as described in the second aspect below; the method comprises:
under condensation reaction conditions, a compound shown in formula (II) and a compound shown in formula (III) are subjected to contact reaction,
and the definition of substituents in the compound represented by formula (II) and the compound represented by formula (III) is the same as that in the first aspect.

Preferably, the condensation reaction is carried out in the presence of an alkaline agent and in an anhydrous environment.

Preferably, the alkaline reagent is at least one of triethylamine, N, N -diisopropylethylamine and 4-dimethylaminopyridine.

The condensation reaction of the present invention is carried out under alkaline conditions.

Preferably, the contact reaction is performed in the presence of a solvent, the solvent is preferably selected from at least one dichloromethane, tetrahydrofuran, N, N -dimethylformamide, acetonitrile and acetone.

Preferably, the conditions of the contact reaction include: reaction temperature is -5 °C to 60 °C, and reaction time is 1h to 48 h.

In the present invention, the compound represented by formula (II) and the compound represented by formula (III) can be obtained from commercial sources, or can be synthesized according to the structural formula by a method in the prior art. The present invention exemplifies in embodiments methods for preparing compounds shown in formula (II), and those skilled in the field should not be construed as a limitation of the invention.

Preferably, in the second aspect of the present invention, the compound represented by formula (II) and the compound represented by formula (III) are used in a molar ratio of 1: (1-3); more preferably 1: (1.1-2.2).

In the second aspect of the present invention, the product obtained after the contact reaction can also be subjected to a post-treatment operation conventionally used in the field to obtain a product of higher purity, for example, the post-treatment operation method includes: extraction, washing, rotary evaporation, column chromatography, recrystallization, etc., and the present invention is not particularly limited thereto as long as the aforementioned compound containing a chain-shaped carboxylic acid amide structure of the present invention can be obtained.

As described above, a third aspect of the present invention provides the use of the compound containing a chain-shaped carboxylic acid amide structure described in the first aspect or an agrochemically acceptable salt, hydrate and solvate thereof described in the first aspect in controlling plant oomycete diseases.

Preferably, the plant oomycete disease is selected from at least one of cucumber downy mildew, potato late blight, and pepper phytophthora blight.

As described above, a fourth aspect of the present invention provides the use of the compound containing a chain-shaped carboxylic acid amide structure according to the foregoing first aspect or an agrochemically acceptable salt, hydrate and solvate thereof described in the first aspect as an agricultural fungicide.

As described above, a fifth aspect of the present invention provides a fungicide which consists of active ingredients and excipients, the active ingredients including at least one of the compound containing a chain-shaped carboxylic acid amide structure according to the first aspect described above, or an agrochemically acceptable salt, hydrate, and solvate thereof described in the first aspect.

Preferably, the content of the active ingredient is 1 wt% to 99.9 wt%; more preferably, the content of the active ingredient is 5 wt% to 95 wt%.

Preferably, the dosage form of the fungicide is selected from at least one of emulsifiable concentrate, suspension concentrate, wettable powder, powder, granule, aqueous solution, poison bait, mother liquor and mother powder.

In the present invention, the excipients can be various excipients conventionally used in the field, such as surfactants, solvents, etc.

Examples of the present invention are described in detail below.

In the following examples, unless otherwise specified, the various raw materials used are all commercially available in chemical purity.

The following room temperature is 25±1°C.

### Example 1

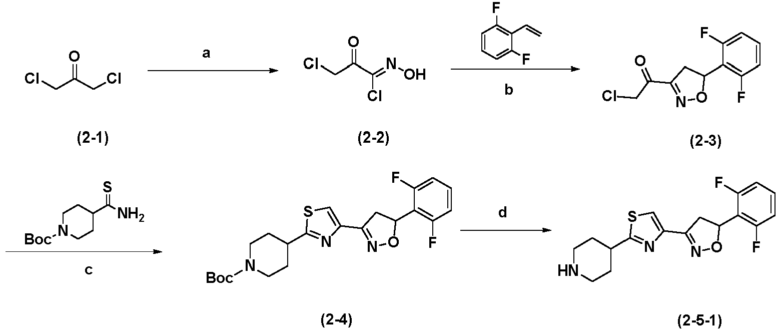

Synthesis of formula (2-2): a compound represented by the formula (2-1) (790.12 mmol) was placed in a 1L two-necked flask at 25 °C and 400 mL of a 2M ether hydrochloride solution was added thereto, and tert-butyl nitrite (534.62 mmol) was slowly added dropwise to the flask to conduct a reaction for 3 hours. After the reaction, the reaction mixture was concentrated under reduced pressure to a semisolid, washed with 1-chlorobutane, and a white crystalline solid was obtained by suction filtration, the filtrate was concentrated, the above process was repeated 2 times, and the filtered solids were combined to a total of 81.10 g. It was directly used for the next reaction.

Synthesis of formula (2-3): to a 500 mL pear-shaped flask, a magnetic stir bar, the compound represented by the formula (2-2) (193.48 mmol), 250 mL acetonitrile, and sodium hydrogencarbonate (1.14 mol) were added in this order, stirring was started, and 2, 6-difluorostyrene (193.51 mmol) was slowly added dropwise from a dropping funnel with a constant pressure. The reaction lasted for 30 min, and TLC was used to monitor the reaction to completion. And carrying out suction filtration on the system, washing a filter cake for three times by using acetonitrile, combining filtrates, and concentrating under reduced pressure to obtain a light yellow viscous liquid. Solids precipitated after standing overnight, suction filtration was performed, and the filter cake was washed with a solution of V_{petroleum ether} :V_{ethyl acetate} = 10:1, removing the solvent under reduced pressure to obtain a pale yellow solid, concentrating filtrates again, and solids precipitated, repeating the above steps twice, and combining the obtained solids to obtain 54.39 g of the compound represented by formula (2-3), wherein the mass spectrum result shows a molecular ion peak at 259.30. The data of proton nuclear magnetic resonance are as follows: ¹H NMR (400 MHz, CDCl₃) δ 7.47 - 7.25 (m, 1H), 6.88 (t, *J* = 8.0 Hz, 2H), 6.20 - 6.08 (m, 1H), 4.67 (s, 2H), 3.60 - 3.52 (m, 1H), 3.33 (dd, *J =* 17.2, 8.0 Hz, 1H).

Synthesis of formula (2-4): to a 250 mL pear-shaped flask, magnetons, a compound represented by formula (2-3) (38.51 mmol), and 100 mL anhydrous methanol were sequentially added, and tert-butyl 4-(aminocarbothioyl)tetrahydropyridine-1(2H)-carboxylate (38.02 mmol) was added with stirring, and the mixture was heated to reflux for about 4 hours to complete the reaction. Concentrating under reduced pressure to remove most of methanol, adding 100 mL of water, extracting with ethyl acetate, separating, drying the organic phase with anhydrous sodium sulfate, filtering, concentrating the filtrate, and purifying by silica gel column chromatography to obtain the compound shown in formula (2-4) with a yield of 89%. The data of proton nuclear magnetic resonance are as follows: ¹H NMR (400 MHz, CDCl₃) δ 7.71 (s, 1H), 7.29 - 7.25 (m, 1H), 6.96 (t, *J =* 8.0 Hz, 2H), 6.03 (dd, *J=* 12.0, 9.2 Hz, 1H), 4.21 (s, 2H), 3.77 (dd, *J =* 17.6, 12.0 Hz, 1H), 3.60 (dd, *J =* 17.6, 9.2 Hz, 1H), 3.19 (tt, *J =* 12.0, 4.0 Hz, 1H), 2.86 (t, *J =* 10.4 Hz, 2H), 2.09 (d, *J=* 12.8 Hz, 2H), 1.70 (qd, *J =* 12.8, 4.8 Hz, 2H), 1.39 (s, 9H).

Synthesis of formula (2-5-1): adding 80 mL of methanol into a compound (40.0 mmol) shown in the formula (2-4) to completely dissolve the compound, then adding 45 mL of hydrochloric acid/methanol (4M) solution, heating to 50 °C, reacting for 4h, after the reaction is finished, concentrating under reduced pressure to remove the solvent, wherein the product exists in a hydrochloride form, adding 2M sodium hydroxide solution to neutralize the product to make the system alkaline, extracting with ethyl acetate for multiple times, drying the organic phase, concentrating to obtain a yellowish-brown oily crude product, and performing silica gel column chromatography to obtain a light brown solid with the yield of 95%. The data of proton nuclear magnetic resonance are as follows: ¹H NMR (400 MHz, CDCl₃) δ 8.39 (s, 1H), 8.01 (s, 1H), 7.48 - 7.39 (m, 1H), 7.20 (t, *J =* 8.4 Hz, 2H), 5.98 (dd, *J =* 12.0, 9.2 Hz, 1H), 3.87 (dd, *J =* 17.6, 12.0 Hz, 1H), 3.61 - 3.49 (m, 1H), 3.41 - 3.37 (m, 3H), 3.02 (t, *J =* 12.0 Hz, 2H), 2.19 (d, *J =* 14.0 Hz, 2H), 1.88 (q, *J =* 12.4 Hz, 2H).

### Example 2

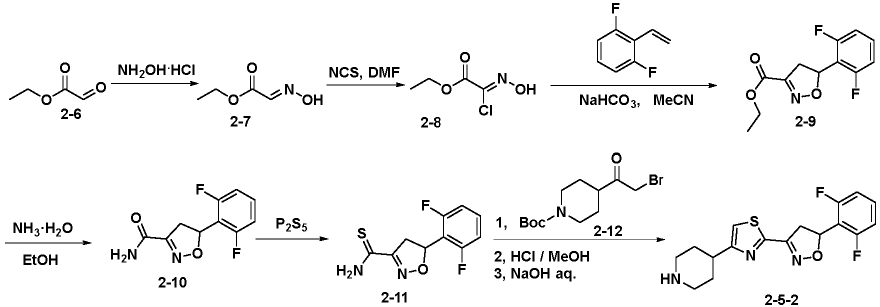

Synthesis of formula (2-7): ethyl glyoxylate (50% toluene solution, 369 mmol) and hydroxylamine hydrochloride (369 mmol) were dissolved in CH₃CN:H₂O(9:1, 300 mL) , stirred for 5 minutes at room temperature and Et₃N (51.7 mL, 369 mmol) was added dropwise. The reaction was carried out at room temperature for 1 hour, then concentrated under reduced pressure. The residue was dissolved in H₂O (50 mL) and Et₂O (300 mL). The organic layer was washed with saturated NH₄Cl (50 mL), dried over Na₂SO₄, filtered, and concentrated. The yield of a colourless crystalline material was 97%.

Synthesis of formula (2-8): ethyl 2-(hydroxyimino) acetate (123.2 mmol) was added to the DMF solution at 5°C. The reaction mixture was stirred at room temperature for 12 hours, water (200 mL) was added, and extraction was performed with ethyl acetate (2×150 mL). The organic layer was separated, dried over anhydrous sodium sulfate, and concentrated under reduced pressure to give the crude product in 74% yield.

Synthesis of formula (2-9): to a 500 mL pear-shaped flask, magnetons, the compound represented by the formula (2-8) (1.52 g), 250 mL acetonitrile, and sodium hydrogencarbonate (5.04 g) were added in this order, stirring was started, and 2, 6-difluorostyrene (1.4 g) was slowly added dropwise from a dropping funnel having a constant pressure. The reaction was lasted for 30 min and the reaction was monitored by TLC until completion. The system was suction - filtered, and the filter cake was washed three times with acetonitrile. The filtrates were combined and concentrated under reduced pressure to obtain a light yellow viscous liquid. Solids precipitated after standing overnight, suction filtration was performed, and the filter cake was washed with a solution of V_{petroleum ether} :V_{ethyl acetate} = 10:1, removing the solvent under reduced pressure to obtain light yellow solid, concentrating the filtrate again, and solids precipitated, repeating the above steps twice, and combining the obtained solids to obtain the compound shown in formula (2-9).

Synthesis of formula (2-10): the compound represented by the formula (2-9) was placed in a sealed tube, dissolved in ethanol, and ammonia monohydrate (5 eq.) was added. The reaction was carried out at room temperature for 24 hours, after the reaction is finished, solids precipitated. Suction filtration was performed, and the filter cake was washed with a solution of V_{petroleum ether} :V_{ethyl acetate} = 10:1. The filter cake was collected and dried to obtain a white solid.

Synthesis of formula (2-11): the compound represented by the formula (2-10) (1.8 g) was dissolved in anhydrous tetrahydrofuran, and phosphorus pentasulfide (3.55g) was added thereto and reacted at 50 °C for 6 hours. Ethyl acetate and 1N hydrochloric acid were then added and the resulting suspension was filtered. The residue was washed with water and ethyl acetate to give a compound represented by the formula (2-11).

Synthesis of formula (2-5-2): a compound represented by the formula 2-11 (1.69 g) was dissolved in methanol, and a compound represented by the formula (2-12) (2.14 g) was added thereto and refluxed for 6 hours. After the reaction, the reaction was cooled to room temperature, and 3 mL of concentrated hydrochloric acid was added, followed by TLC until the reaction was complete. 2M sodium hydroxide solution was added to the system to adjust the pH to 10. The mixture was extracted with dichloromethane, dried over anhydrous sodium sulfate, and concentrated to obtain a yellowish-brown oily substance (2-5-2).

### Example 3: preparation of the Compound of formula (I)

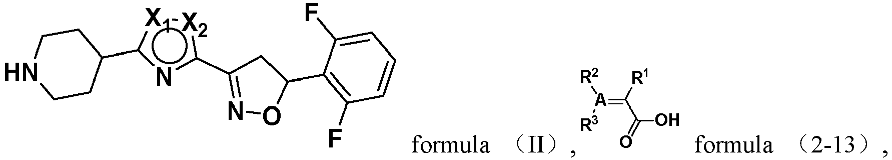

This example is used to illustrate the synthesis method of target compounds of formulae (I-1) to (I-236).

Specifically, the preparation of a compound represented by the formula (I-1) is exemplified:
trans-3-methoxyacrylic acid (1.4 mmol, 1.4 eq.) was added to a 100 mL eggplant-shaped flask at room temperature, dissolved in 20 mL dichloromethane, and then EDCI (2.1 mmol, 2.1 eq.), HOBt (2.10 mmol, 2.1 eq.), TEA (0.42 mL, 3.0 mmol, 3.0 eq.) were sequentially added, and the mixture was activated by stirring for 60 min. Subsequently, the compound represented by formula (2-5-1) (1.0 mmol, 1.0 eq.) was added to the system. Continue the reaction for 6 h. After the reaction was completed, water was added, and the mixture was extracted twice with dichloromethane. The organic phase was dried, concentrated, and mixed with silica gel for sample loading. Purification by column chromatography (V_{petroleum ether} : V_{ethyl acetate} = 2: 1) gave the target compound shown in the formula (I-1), as a white solid with a yield of 78%.

### Example 4

Synthetic route and method of acid intermediates **I-1a** to **I-5a**

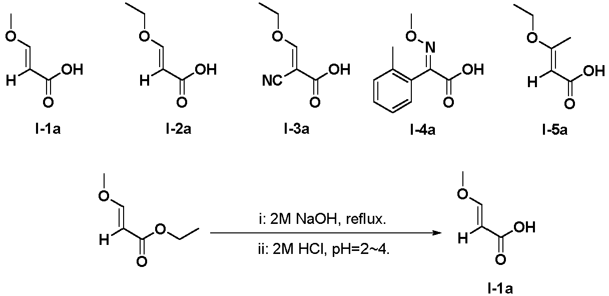

For the fragments of esters corresponding to some acids, the relevant acid intermediates can be obtained directly by ester hydrolysis.

A method for synthesizing a compound represented by the formula (I-1 a) is exemplified: a 50 -mL of eggplant-shaped bottle was taken, methyl trans-3-methoxyacrylate (10.0 mmol, 1 eq.) is added, 10 mL of sodium hydroxide solution (20.0 mmol, 2.0 eq.) was added, the reaction temperature was raised to 80 °C, the reaction was carried out overnight, TLC was used for monitoring the completion of hydrolysis, and heating was stopped. After the temperature was reduced to room temperature, the pH was adjusted to acidic with 2 mol/L hydrochloric acid. White solids were precipitated. Suction filtration was performed, and the filter cake was washed twice with a small amount of water. The filter cake was collected and dried, and the pure acid product was obtained as a white solid with a yield of 56.8%.

### Example 5

Synthetic route and method of acid intermediates **I-6a** to **I-10a**

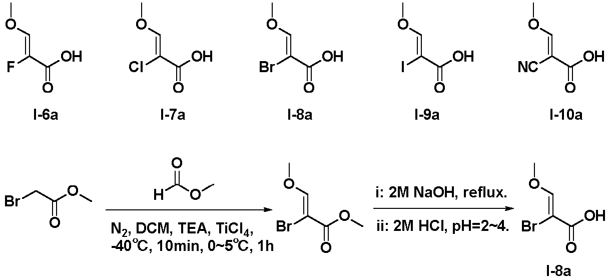

For the acid fragment modified with electron-absorbing substituent at position 2 of acrylic acid, the corresponding ester is firstly obtained by adopting the above synthetic route, and then the acid fragment is obtained by hydrolysis.

Preparation of compound **I-8a** as an example: taking a 100 mL three-neck flask, adding 20 mL of ultra-dry dichloromethane under the atmosphere of N₂, weighing methyl bromoacetate (10.0 mmol, 1.0 eq.), dissolving methyl formate (30.0 mmol, 3.0 eq.), adding triethylamine (24.0 mmol, 2.4 eq.) into the system after cooling to -40 °C in a low-temperature reactor, slowly dropwise adding TiCl₄ (15 mL, 1mol/L in DCM, 15.0 mmol, 1.5 eq.) into the reaction system after the temperature was reduced to -40 °C again, continuing to react for 10 min after dropwise adding, heating to 5 °C for continuing to react for 1h, after the reaction was completed, pouring the reaction mixture into ice water, extracting the reaction mixture with dichloromethane twice, drying organic phase, concentrating it under reduced pressure. Mixing the concentrated residue with silica gel for sample loading, purifying by column chromatography (V_{petroleum ether} :V_{ethyl acetate} = 20: 1) to obtain a yellow oily compound with a yield of 76.7%. Hydrolyzing and acidifying to obtain the corresponding acid **I-8a.**

### Example 6

Synthetic route and method of acid intermediates **I-11a** to **I-14a**

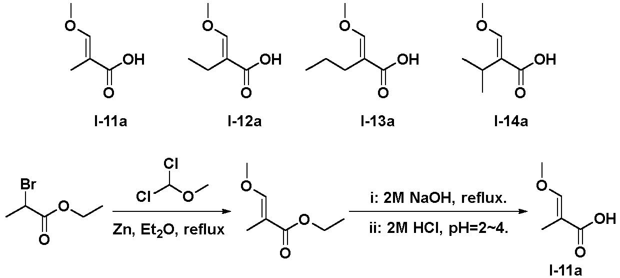

For the acid fragment modified with methyl isoelectron-donating substituents at the position 2 of acrylic acid, the corresponding ester is firstly obtained by adopting the above synthetic route, and then the acid fragment is obtained by hydrolysis.

Preparation of compound **I-11a** as an example: a 50 mL eggplant-shaped bottle was prepared, zinc powder (90.0 mmol, 3.0 eq.) and 15 mL diethyl ether were added, and a suspension was prepared by stirring. Dichloromethyl methyl ether (30 mmol, 1.0 eq.) and ethyl 2-bromopropionate (60.0 mmol, 2.0 eq.) were added into a constant pressure dropping funnel, and 15 mL diethyl ether were added for dilution. Controlling the temperature to reflux the diethyl ether, slowly dripping the mixture into the eggplant-shaped bottle, and continuously refluxing the reaction liquid for 20 min after the dripping is finished to stop the reaction. The zinc powder was removed by suction filtration on silica gel, washed thoroughly with dichloromethane, the filtrate was washed once with 5v% acetic acid-water solution, twice with water, washed with saturated brine and dried, the organic phase was mixed, silica gel column chromatography (V_{petroleum ether} :V_{ethyl acetate} = 10:1) was used to obtain a transparent oily compound with a yield of 57%. Hydrolysis and acidification were carried out to obtain the corresponding acid **I-11a**.

### Example 7

The synthesis route and the method of the acid intermediate **I-15a** to **I-19a,** taking the synthesis route and the method of the acid fragment compound **I-15a** as an example:

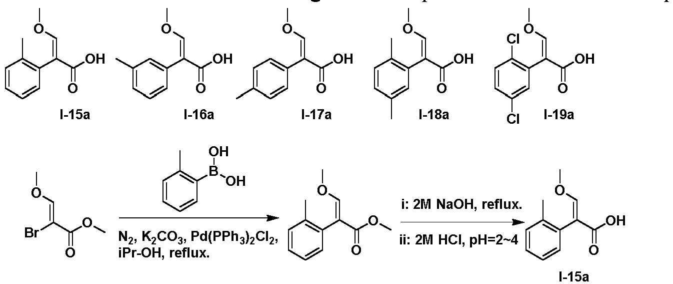

For the acid fragment modified with benzene ring at the position 2 of the acrylic acid, a bromo-compound is firstly obtained by adopting the above synthesis route , then the bromo-compound is coupled with phenylboronic acid by Suzuki to obtain corresponding ester, and the acid fragment is obtained by hydrolysis.

Preparation of compound **I-15a** as an example: a 100 mL three-neck flask was taken, under the nitrogen atmosphere, methyl- (Z) -2-bromo-3-methoxyacrylate (10.0 mmol, 1.0 eq.) and 2-methylphenylboronic acid (30.0 mmol, 3.0 eq.) , anhydrous potassium carbonate (50.0 mmol, 5.0 eq.), bis(triphenylphosphine)palladium(II) dichloride (1.0 mmol, 0.1 eq.), and 40 mL of isopropanol were added. The mixture was heated to reflux and the reaction was allowed to proceed overnight. The reaction was monitored by TLC. After the reaction was completed, water was added and the mixture was extracted twice with ethyl acetate. The organic phase was dried, the residue was concentrated, and it was mixed with silica gel for sample loading. The product was purified by column chromatography (V_{petroleum ether} :V_{ethyl acetate} = 20: 1) to obtain a light yellow oily compound with a yield of 92.1%. The compound was further hydrolyzed and acidified to obtain the corresponding acid **I-15a.**

### Example 8

The synthesis route and the method of the acid intermediate **I-20a** to **I-25a,** taking the synthesis route and the method of the acid fragment compound **I-20a** as an example:

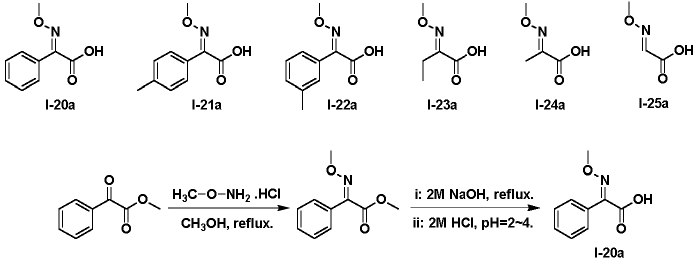

The introduction of a methoxy oxime structure into a carboxylic acid intermediate, adopting the above synthesis route, starting from a substituted methyl acetylformate, reacting with methoxyamine hydrochloride to introduce the methoxy oxime structure, and the acid fragment is obtained by hydrolysis.

Preparation of compound **I-20a** as an example: taking a 50 mL eggplant-shaped bottle, adding methyl phenyl acetyl acetate (11.0 mmol, 1.0 eq.), methoxyamine hydrochloride (22.0 mmol, 2.0 eq.) and 20 mL methanol, heating the system to reflux, continuing to react for 8 h, adding water after the reaction was finished, extracting with ethyl acetate, drying the organic phase, concentrating and stirring, and purifying by column chromatography (V_{petroleum ether} :V_{ethyl acetate} = 20:1) to obtain a white solid compound with a yield of 70.6%. Then hydrolyzing and acidifying to obtain the corresponding acid **I-20a.**

### Example 9

This example is used to illustrate the synthesis method of target compounds of formulae (**II-1**) to (**II-136**).

Specifically, the preparation of the compound represented by the formula (II-1) is exemplified:
Carboxylic acid 3, 3-dimethoxypropionic acid (1.0 mmol, 1.0 eq.) was added to a 100 mL eggplant-shaped bottle at room temperature and dissolved in 20 mL of dichloromethane, EDCI (1.5 mmol, 1.5 eq.), HOBt (1.5 mmol, 1.5 eq.) and TEA (2.1 mmol, 2.14 eq.) were added in this order, activated with stirring for 60 min, and an intermediate (0.71 mmol, 0.71 eq.) represented by formula(2-5-1) obtained in the previous stage was added to the system. And continuing the reaction for 6h, adding water after the reaction is finished, extracting by dichloromethane, drying the organic phase, concentrating and stirring, and purifying by column chromatography (V_{petroleum ether} : V_{ethyl acetate} = 2:1) to obtain the target compound II-1 as a white solid with a yield of 68%.

### Example 10

The synthetic route and the method of the acid intermediate **II-1a** to **II-4a,** taking the synthetic route and the method of the compound **II-1a** as an example:

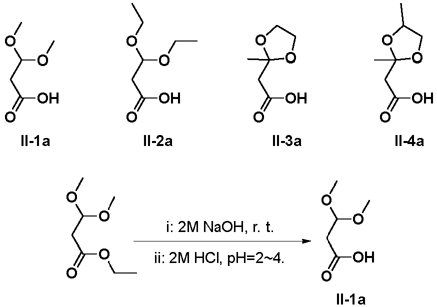

The synthesis route and the method of the acid intermediates **II-1a** to **II-4a,** taking the preparation of compound **II-1a** as an example:
Taking a 50 mL eggplant-shaped bottle, adding ethyl-3, 3-dimethoxypropionate (10.0 mmol, 1 eq.), then adding 10 mL of 2 mol/L sodium hydroxide solution (20.0 mmol, 2.0 eq.), adding 30 mL of methanol, the system was in a homogeneous phase, the reaction was carried out overnight, TLC was used for monitoring the completion of hydrolysis, and heating was stopped. After cooling to room temperature, acidifying with 2 mol/L hydrochloric acid, extracting with ethyl acetate, drying organic phase, and concentrating the residue to obtain pure acid. The product was obtained as a pale - yellow oil with a yield of 85%.

### Example 11

The synthesis route and the method of the acid intermediate **II-5a** to **II-10a,** taking the synthesis route and the method of the acid fragment compound **II-5a** as an example:

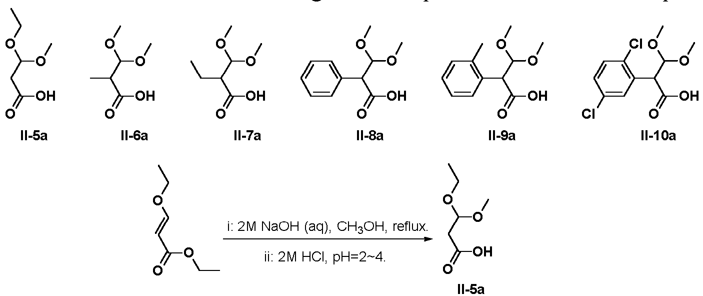

The synthesis route and method of acid intermediates II-5a to II-10a, taking the preparation of compound II-5a as an example:
ethyl- (E) -3-ethoxyacrylate (10.0 mmol, 1 eq.) was added into a 50 mL eggplant-shaped bottle, then 7.5 mL of a 2 mol/L sodium hydroxide solution (15.0 mmol, 1.5 eq.) was added, and 20 mL of methanol was added, the temperature was raised to 80 °C for reaction overnight, TLC monitored that hydrolysis was complete, and heating was stopped. After cooling to room temperature, 2 mol/L hydrochloric acid was used for adjusting the pH value to 3, extracting with ethyl acetate, and drying organic phase, and concentrating the residue to obtain a crude acid. The product was obtained as a clear oil with a yield of 84%.

### Characterization data for the target compound:

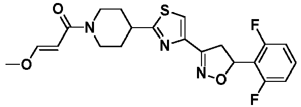

Formula (I-1). white solid, m.p.= 134-136°C. ¹H NMR (600 MHz, DMSO-*d₆*) δ 8.02 (d, *J =* 3.0 Hz, 1H), 7.49 (t, *J* = 7.8 Hz, 1H), 7.44 (d, *J* = 12.0 Hz, 1H), 7.15 (t, *J* = 9.0 Hz, 2H), 6.00 (t, *J* = 10.2 Hz, 1H), 5.90 (d, *J* = 12.0 Hz, 1H), 4.46 (s, 1H), 4.16 (s, 1H), 3.89 (dd, *J* = 16.8, 12.6 Hz, 1H), 3.67 (s, 3H), 3.53 (dd, *J =* 17.4, 8.6 Hz, 1H), 3.37 (s, 1H), 3.15 (d, *J =* 12.6 Hz, 1H), 2.74 (d, *J =* 12.6 Hz, 1H), 2.08 (d, *J* = 13.2 Hz, 2H), 1.57 (s, 2H). ¹³C NMR (100 MHz, CDCl₃) δ 174.79, 166.03, 162.69, 162.59, 160.13 (d, *J* = 7.6 Hz), 152.28, 145.14, 130.55 (t, *J* = 10.5 Hz), 117.70, 115.77 (t, *J =* 16.1 Hz), 111.98 - 111.73 (m), 94.83, 72.87 (t, *J =* 3.3 Hz), 57.85, 41.55, 40.71, 32.55. HRMS (MALDI) calculated value C₂₁H₂₂F₂N₃O₃S [M+H]⁺ 434.1344, found 434.1342. 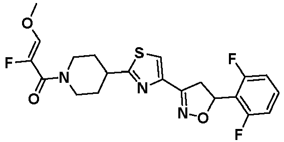 Formula (I-2). white solid; m.p.= 141-143°C. ¹H NMR (400 MHz, DMSO-*d₆*) δ 8.02 (s, 1H), 7.55 - 7.44 (m, 1H), 7.16 (t, *J =* 8.4 Hz, 2H), 6.83 (d, *J =* 21.6 Hz, 1H), 6.00 (dd, *J =* 12.0, 8.8 Hz, 1H), 4.16 (d, *J =* 13.6 Hz, 2H), 3.90 (dd, *J =* 17.2, 12.0 Hz, 1H), 3.80 (s, 3H), 3.53 (dd, *J =* 17.2, 8.6 Hz, 1H), 3.44 - 3.35 (m, 1H), 3.07 (t, *J =* 12.6 Hz, 2H), 2.12 (dd, *J =* 13.6, 3.6 Hz, 2H), 1.71 - 1.57 (m, 2H). ¹³C NMR (100 MHz, DMSO-*d*₆) δ 175.27, 162.39 (d, *J =* 7.9 Hz), 161.36, 161.15, 159.91 (d, *J =* 7.8 Hz), 152.58, 144.78, 141.29, 141.22, 140.18, 137.65, 131.83 (t, *J =* 10.8 Hz), 120.34, 116.09 (t, *J =* 16.6 Hz), 112.78 - 112.53 (m), 72.50 (d, *J =* 3.0 Hz), 61.85, 44.33, 41.61, 32.70. HRMS (MALDI) calculated value C₂₁H₂₁F₃N₃O₃S [M+H]⁺ 452.1250, found 452.1250. 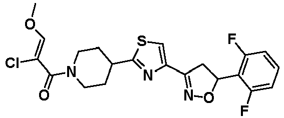 Formula (I-3). white solid; m.p.= 126-128°C. ¹H NMR (400 MHz, CDCl₃) δ 7.69 (s, 1H), 7.38 - 7.31 (m, 1H), 7.01 (s, 1H), 6.99 - 6.91 (m, 2H), 6.11 (dd, *J* = 12.0, 9.2 Hz, 1H), 4.36 (d, *J* = 13.6 Hz, 2H), 3.91 (s, 3H), 3.84 (dd, *J =* 17.2, 12.0 Hz, 1H), 3.67 (dd, *J =* 17.2, 9.2 Hz, 1H), 3.39 - 3.30 (m, 1H), 3.15 - 3.04 (m, 2H), 2.24 (dd, *J* = 13.6, 3.7 Hz, 2H), 1.95 - 1.82 (m, 2H). ¹³C NMR (100 MHz, DMSO-*d*₆) δ 175.30, 164.12, 162.40 (d, *J* = 7.7 Hz), 159.92 (d, *J* = 7.8 Hz), 152.58, 151.83, 144.79, 131.86 (t, *J =* 11.0 Hz), 120.40, 116.11 (t, *J* = 16.5 Hz), 112.80 - 112.56 (m), 102.27, 72.51, 61.61, 44.85, 41.63, 32.50. HRMS (MALDI) calculated value C₂₁H₂₁ClF₂N₃O₃S [M+H]⁺ 468.0955, found 468.0953. 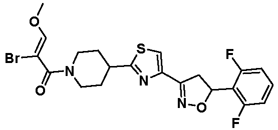 Formula(I-4). white solid; m.p.= 76-78°C. ¹H NMR (400 MHz, CDCl₃) δ 7.69 (s, 1H), 7.39 - 7.32 (m, 1H), 7.04 (s, 1H), 6.99 - 6.92 (m, 2H), 6.11 (dd, *J =* 12.0, 9.2 Hz, 1H), 4.37 (d, *J =* 13.6 Hz, 2H), 3.91 (s, 3H), 3.84 (dd, *J =* 17.2, 12.0 Hz, 1H), 3.67 (dd, *J =* 17.2, 9.2 Hz, 1H), 3.39 - 3.30 (m, 1H), 3.14 - 3.02 (m, 2H), 2.24 (dd, *J* = 13.6, 3.6 Hz, 2H), 1.94 - 1.83 (m, 2H). ¹³C NMR (150 MHz, CDCl₃) δ 174.47, 164.93, 162.18, 160.52, 152.25, 145.16, 130.55, 117.74, 115.73 (t, *J =* 15.6 Hz), 111.92, 111.77, 91.57, 72.85, 61.39, 45.06, 41.53, 40.46, 32.27. HRMS (MALDI) calculated value C₂₁H₂₁BrF₂N₃O₃S [M+H]⁺ 514.0431, found 514.0434. 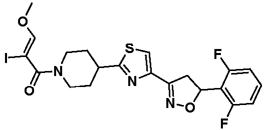 **Formula(I-5).** light yellow solid; m.p.= 76-78°C. ¹H NMR (400 MHz, CDCl₃) δ 7.69 (s, 1H), 7.38 - 7.30 (m, 1H), 6.95 (t, *J =* 8.0 Hz, 2H), 6.76 (s, 1H), 6.11 (dd, *J =* 12.0, 9.2 Hz, 1H), 4.36 (d, *J =* 13.6 Hz, 2H), 3.90 (s, 3H), 3.84 (dd, *J =* 17.2, 12.0 Hz, 1H), 3.66 (dd, *J =* 17.2, 9.2 Hz, 1H), 3.40 - 3.30 (m, 1H), 3.07 (t, *J* = 12.0 Hz, 2H), 2.27 - 2.18 (m, 2H), 1.94 - 1.81 (m, 2H). ¹³C NMR (100 MHz, CDCl₃) δ 174.52, 165.90, 162.61 (d, *J =* 7.5 Hz), 160.11 (d, *J =* 7.7 Hz), 155.16, 152.23, 145.17, 130.59 (t, *J =* 10.6 Hz), 117.82, 115.74 (t, *J =* 16.2 Hz), 112.00-111.75 (m), 72.87 (t, *J =* 2.8 Hz), 63.68, 60.96, 45.22, 41.55 (d, *J* = 2.3 Hz), 40.43, 32.13. HRMS (MALDI) calculated value C₂₁H₂₂F₂IN₃O₃S [M+H]⁺ 560.0311, found 560.0308. 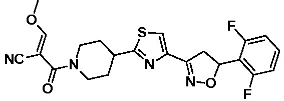 **Formula(I-6).** white solid; m.p.= 87-89°C. ¹H NMR (400 MHz, CDCl₃) δ 7.75 (s, 1H), 7.69 (s, 1H), 7.35 - 7.28 (m, 1H), 6.92 (t, *J =* 8.0 Hz, 2H), 6.08 (dd, *J =* 12.0, 9.2 Hz, 1H), 4.84 (d, *J =* 13.6 Hz, 1H), 3.85 - 3.76 (m, 4H), 3.73 (d, *J =* 12.8 Hz, 1H), 3.63 (dd, *J =* 17.2, 9.2 Hz, 1H), 3.54 (t, *J =* 12.0 Hz, 1H), 3.48 - 3.35 (m, 2H), 2.31 (d, *J =* 13.6 Hz, 2H), 2.09 - 1.94 (m, 2H). ¹³C NMR (100 MHz, CDCl₃) δ 172.92, 167.29, 162.62 (d, *J* = 7.6 Hz), 160.12 (d, *J* = 7.6 Hz), 155.65, 152.13, 145.38, 130.62 (t, *J* = 10.7 Hz), 117.95, 117.92, 115.73 (t, *J* = 16.2 Hz), 112.02 - 111.77 (m), 72.94 (t, *J* = 2.9 Hz), 69.71, 56.09, 52.16, 45.68, 41.55, 39.18, 32.73, 31.59. HRMS (MALDI) calculated value C₂₂H₂₁F₂N₄O₃S [M+H]⁺ 459.1297, found 459.1302. 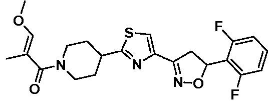 **Formula(I-7).** yellow oil. ¹H NMR (400 MHz, CDCl₃) δ 7.68 (s, 1H), 7.39 - 7.30 (m, 1H), 6.95 (t, *J* = 8.0 Hz, 2H), 6.46 - 6.41 (m, 1H), 6.11 (dd, *J =* 12.0, 9.2 Hz, 1H), 4.34 (d, *J =* 13.6 Hz, 2H), 3.84 (dd, *J =* 17.2, 12.0 Hz, 1H), 3.74 (s, 3H), 3.66 (dd, *J =* 17.2, 9.2 Hz, 1H), 3.38 - 3.29 (m, 1H), 3.10 - 2.98 (m, 2H), 2.22 (dd, *J =* 13.6, 3.6 Hz, 2H), 1.88 - 1.74 (m, 5H). ¹³C NMR (100 MHz, CDCl₃) δ 174.75, 171.37, 162.63 (d, *J =* 7.0 Hz), 160.13 (d, *J =* 7.0 Hz), 152.24, 150.31, 145.18, 130.56 (t, *J =* 10.0 Hz), 117.71, 115.77 (t, *J =* 16.0 Hz), 111.98 - 111.74 (m), 109.47, 72.86 (t, *J =* 3.0 Hz), 60.33, 44.67, 41.55, 40.77, 32.59, 11.83. HRMS (MALDI) calculated value C₂₂H₂₄F₂N₃O₃S [M+H]⁺ 448.1501, found 448.1502. 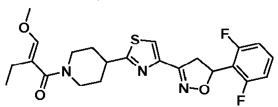 **Formula(I-8).** yellow oil. ¹H NMR (400 MHz, CDCl₃) δ 7.68 (d, *J* = 0.8 Hz, 1H), 7.39 - 7.31 (m, 1H), 6.95 (t, *J =* 8.0 Hz, 2H), 6.28 (s, 1H), 6.11 (dd, *J =* 12.0, 9.2 Hz, 1H), 4.42 (d, *J =* 13.6 Hz, 2H), 3.84 (dd, *J* = 17.2, 12.0 Hz, 1H), 3.72 (d, *J* = 0.8 Hz, 3H), 3.66 (dd, *J* = 17.2, 9.2 Hz, 1H), 3.40 - 3.28 (m, 1H), 3.05 (t, *J =* 12.0 Hz, 2H), 2.35 (q, *J =* 7.6 Hz, 2H), 2.27 - 2.19 (m, 2H), 1.87 - 1.72 (m, 2H), 1.08 - 1.01 (m, 3H). ¹³C NMR (100 MHz, CDCl₃) δ 174.77, 170.77, 162.64 (d, *J* = 7.0 Hz), 160.14 (d, *J* = 8.0 Hz), 152.21, 148.54, 145.17, 130.56 (t, *J* = 10.0 Hz), 117.71, 117.69, 116.00, 115.78 (t, *J =* 16.0 Hz), 111.99 - 111.74 (m), 72.89 (t, *J =* 3.0 Hz), 60.27, 41.56, 40.76, 32.64, 19.64, 12.87. HRMS (MALDI) calculated value C₂₃H₂₆F₂N₃O₃S [M+H]⁺ 462.1657, found 462.1660. 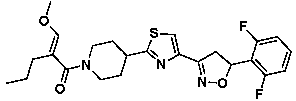 **Formula(I-9).** yellow oil. ¹H NMR (400 MHz, CDCl₃) δ 7.65 (s, 1H), 7.35 - 7.25 (m, 1H), 6.92 (t, *J* = 8.0 Hz, 2H), 6.28 (s, 1H), 6.08 (dd, *J =* 12.0, 9.2 Hz, 1H), 4.38 (d, *J =* 13.6 Hz, 2H), 3.81 (dd, *J =* 17.2, 12.0 Hz, 1H), 3.68 (s, 3H), 3.63 (dd, *J =* 17.2, 9.2 Hz, 1H), 3.35 - 3.25 (m, 1H), 3.01 (s, 2H), 2.27 (dd, *J* = 8.8, 6.4 Hz, 2H), 2.23 - 2.15 (m, 2H), 1.82 - 1.69 (m, 2H), 1.47 - 1.37 (m, 2H), 0.92 (t, *J =* 7.2 Hz, 3H). ¹³C NMR (100 MHz, CDCl₃) δ 174.78, 170.93, 162.63 (d, *J =* 8.0 Hz), 160.13 (d, *J* = 8.0 Hz), 152.23, 149.03, 145.18, 130.56 (t, *J* = 11.0 Hz), 117.73, 115.77 (t, *J* = 16.0 Hz), 114.70, 111 - 111.74 (m), 72.87 (t, *J* = 3.0 Hz), 60.25, 44.70, 41.54, 40.78, 32.62, 28.38, 21.64, 14.10. HRMS (MALDI) calculated value C₂₄H₂₈F₂N₃O₃S [M+H]⁺ 476.1814, found 476.1816. 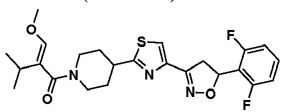 **Formula(I-10).** white solid; m.p.= 58-60°C. ¹H NMR (400 MHz, CDCl₃) δ 7.65 (s, 1H), 7.31 (tt, *J=* 8.4, 6.4 Hz, 1H), 6.96 - 6.88 (m, 2H), 6.11 - 6.03 (m, 2H), 4.44 (d, *J =* 13.2 Hz, 2H), 3.86 - 3.76 (m, 1H), 3.66 (s, 3H), 3.65 - 3.59 (m, 1H), 3.30 (tt, *J=* 11.2, 3.6 Hz, 1H), 3.01 (t, *J =* 12.8 Hz, 2H), 2.86 (p, *J=* 7.2 Hz, 1H), 2.23 - 2.15 (m, 2H), 1.81 - 1.67 (m, 2H), 1.15 (s, 3H), 1.14 (s, 3H). ¹³C NMR (100 MHz, CDCl₃) δ 173.73, 168.89, 161.60 (d, *J=* 7.3 Hz), 159.10 (d, *J=* 7.5 Hz), 151.20, 145.62, 144.14, 129.53 (t, *J =* 10.6 Hz), 118.39, 116.72, 114.73 (t, *J =* 16.1 Hz), 110.95 - 110.70 (m), 71.85, 59.23, 43.50, 40.53, 39.75, 31.61, 25.77, 20.20. HRMS (MALDI) calculated value C₂₄H₂₇F₂N₃NaO₃S [M+Na]⁺: 498.1633, found 498.1632. 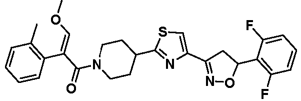 **Formula(I-11).** white solid; m.p.= 69-71°C. ¹H NMR (400 MHz, CDCl₃) δ 7.62 (s, 1H), 7.29 (d, *J =* 7.6 Hz, 1H), 7.25 (d, *J =* 4.8 Hz, 1H), 7.26 - 7.20 (m, 3H), 7.00 (s, 1H), 6.91 (t, *J =* 8.0 Hz, 2H), 6.06 (dd, *J =* 12.0, 9.2 Hz, 1H), 4.12 (s, 2H), 3.79 (s, 4H), 3.60 (dd, *J =* 17.2, 9.2 Hz, 1H), 3.26 - 3.14 (m, 1H), 2.86 - 2.74 (m, 2H), 2.27 (s, 3H), 1.95 (d, *J =* 13.2 Hz, 2H), 1.45 (s, 2H). ¹³C NMR (100 MHz, CDCl₃) δ 174.89, 162.63 (d, *J* = 8.0 Hz), 160.13 (d, *J* = 8.0 Hz), 154.80, 152.26, 145.07, 136.52, 133.80, 130.56, 130.55 (t, *J* = 10.0 Hz), 130.15, 127.55, 125.74, 117.63, 115.78 (t, *J* = 16.0 Hz), 114.85, 111.99 - 111.74 (m), 72.84 (t, *J* = 3.0 Hz), 61.19, 41.55, 40.52, 32.11, 20.13. HRMS (MALDI) calculated value C₂₈H₂₈F₂N₃O₃S [M+H]⁺ 524.1814, found 524.1819. 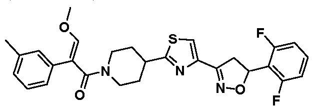 **Formula(I-12).** white solid; m.p.= 71-73°C. ¹H NMR (400 MHz, CDCl₃) δ 7.63 (s, 1H), 7.36 - 7.24 (m, 3H), 7.22 (dd, *J =* 8.8, 7.3 Hz, 1H), 7.03 (d, *J =* 7.2 Hz, 1H), 6.91 (t, *J =* 8.0 Hz, 2H), 6.64 (s, 1H), 6.06 (dd, *J =* 12.0, 9.2 Hz, 1H), 4.26 (s, 2H), 3.82 (s, 3H), 3.80 - 3.74 (m, 1H), 3.61 (dd, *J =* 17.2, 9.2 Hz, 1H), 3.28 - 3.15 (m, 1H), 2.96 - 2.84 (m, 2H), 2.34 (s, 3H), 2.05 (s, 2H), 1.61 (s, 2H). ¹³C NMR (100 MHz, CDCl₃) δ 174.83, 162.64 (d, *J* = 8.0 Hz), 160.14 (d, *J* = 8.0 Hz), 152.25, 150.58, 145.14, 137.86, 133.69, 130.56 (t, *J* = 10.0 Hz), 128.49, 128.30, 127.71, 125.09, 117.66, 115.79 (t, *J* = 16.0 Hz), 115.22, 111.99 - 111.75 (m), 72.85, 61.39, 41.58, 40.58, 32.34, 21.60. HRMS (MALDI) calculated value C₂₈H₂₈F₂N₃O₃S [M+H]⁺ 524.1814, found 524.1819. 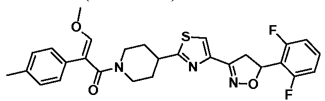 **Formula(I-13).** white solid; m.p.= 81-83°C. ¹H NMR (600 MHz, DMSO-*d₆*) δ 8.01 (s, 1H), 7.50 (p, *J =* 7.2 Hz, 1H), 7.31 (d, *J* = 7.8 Hz, 2H), 7.21 - 7.01 (m, 4H), 6.61 (s, 1H), 6.00 (dd, *J =* 12.0, 8.4 Hz, 1H), 4.11 (s, 1H), 3.89 (dd, *J =* 17.4, 12.0 Hz, 1H), 3.78 (s, 3H), 3.52 (dd, *J =* 17.4, 8.5 Hz, 1H), 3.38 (s, 2H), 2.94 (t, *J =* 12.6 Hz, 2H), 2.27 (s, 3H), 2.07 - 1.95 (m, 2H), 1.51 (s, 2H). ¹³C NMR (150 MHz, DMSO-*d₆*) δ 174.82, 162.43 (d, *J =* 8.0 Hz), 160.15 (d, *J =* 7.8 Hz), 152.05, 150.38, 144.88, 137.69, 133.45, 130.22 (t, *J =* 10.0 Hz), 128.29, 128.08, 127.59, 124.42, 117.37, 115.69 (t, *J =* 16.0 Hz), 115.22, 111.98 - 111.74 (m), 72.87 (t, *J =* 3.0 Hz), 61.28, 41.58, 40.56, 32.31, 21.22. HRMS (MALDI) calculated value C₂₈H₂₈F₂N₃O₃S M+H⁺ 524.1814, found 524.1816. 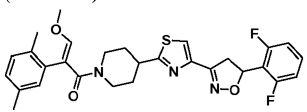 **Formula(I-14).** white solid; m.p.= 79-81°C. ¹H NMR (400 MHz, CDCl₃) δ 7.61 (s, 1H), 7.34 - 7.27 (m, 1H), 7.09 (d, *J =* 7.6 Hz, 1H), 7.05 (s, 1H), 6.98 (dd, *J =* 7.6, 1.6 Hz, 1H), 6.96 (s, 1H), 6.91 (t, *J* = 8.0 Hz, 2H), 6.05 (dd, *J =* 12.0, 9.2 Hz, 1H), 4.14 (s, 2H), 3.82 - 3.71 (m, 4H), 3.60 (dd, *J =* 17.2, 9.2 Hz, 1H), 3.19 - 3.09 (m, 1H), 2.84 - 2.75 (m, 2H), 2.30 (s, 3H), 2.22 (s, 3H), 2.00 - 1.87 (m, 2H), 1.45 (s, 2H). ¹³C NMR (100 MHz, CDCl₃) δ 174.98, 169.86, 162.64 (d, *J* = 8.0 Hz), 160.14 (d, *J* = 6.0 Hz), 154.61, 152.27, 145.07, 135.05, 133.53, 133.33, 130.47, 128.44, 117.61, 115.79 (t, *J =* 16.3 Hz), 114.80, 111.99, 111.75, 72.85, 61.15, 41.55, 40.57, 32.15, 20.98, 19.67. HRMS (MALDI) calculated value C₂₉H₃₀F₂N₃O₃S [M+H]⁺ 538.1970, found 538.1976. 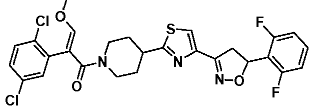 **Formula(I-15).** white solid; m.p.= 94-96°C. ¹H NMR (400 MHz, CDCl₃) δ 7.57 (s, 1H), 7.36 (d, *J =* 2.4 Hz, 1H), 7.25 - 7.18 (m, 2H), 7.12 (dd, *J =* 8.8, 2.5 Hz, 1H), 6.89 (s, 1H), 6.84 (t, *J =* 8.4 Hz, 2H), 6.00 (dd, *J* = 12.0, 9.2 Hz, 1H), 4.15 (s, 2H), 3.76 (s, 3H), 3.75 - 3.68 (m, 1H), 3.55 (dd, *J* = 17.2, 9.2 Hz, 1H), 3.22 - 3.14 (m, 1H), 2.90 - 2.79 (m, 2H), 1.99 (d, *J =* 11.2 Hz, 2H), 1.53 (d, *J =* 12.8 Hz, 2H). ¹³C NMR (100 MHz, CDCl₃) δ 174.77, 168.22, 162.63 (d, *J =* 8.0 Hz), 160.13 (d, *J =* 8.0 Hz), 155.83, 152.25, 145.11, 134.77, 132.41, 131.81, 131.75, 130.85, 130.56 (t, *J* = 10.0 Hz), 128.70, 117.71, 115.78 (t, *J* = 16.0 Hz), 111.99, 111.94 - 111.69 (m), 72.86 (t, *J* = 2.0 Hz), 61.62, 41.57, 40.54, 32.16. HRMS (MALDI) calculated value C₂₇H₂₄Cl₂F₂N₃O₃S [M+H]⁺ 578.0878, found 578.0881. 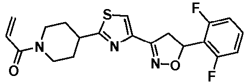 **Formula(I-16).** white solid; m.p.= 119-121°C. ¹H NMR (400 MHz, CDCl₃) δ 7.64 (s, 1H), 7.34 - 7.25 (m, 1H), 6.95 - 6.87 (m, 2H), 6.59 (dd, *J =* 16.8, 10.4 Hz, 1H), 6.28 (dd, *J =* 16.8, 2.0 Hz, 1H), 6.06 (dd, *J =* 12.0, 9.2 Hz, 1H), 5.70 (dd, *J =* 10.4, 2.0 Hz, 1H), 4.70 (s, 1H), 4.18 - 3.98 (m, 1H), 3.86 - 3.73 (m, 1H), 3.62 (dd, *J* = 17.2, 9.2 Hz, 1H), 3.38 - 3.29 (m, 1H), 3.23 (s, 1H), 2.87 (s, 1H), 2.20 (d, *J =* 12.0 Hz, 2H), 1.85 - 1.73 (m, 2H). ¹³C NMR (100 MHz, DMSO-*d₆*) δ 175.41, 164.74, 162.38 (d, *J* = 7.9 Hz), 159.90 (d, *J* = 7.8 Hz), 152.58, 144.75, 131.85 (t, *J* = 10.8 Hz), 128.93, 127.64, 120.36, 116.09 (t, *J* = 16.5 Hz), 112.79 - 112.55 (m), 72.52, 72.49, 72.46, 45.21, 41.61, 40.02, 33.20, 32.33. HRMS (MALDI) calculated value C₂₀H₂₀F₂N₃O₂S [M+H]⁺ 404.1239, found 404.1241. 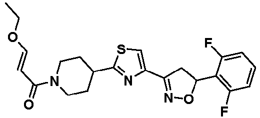 **Formula(I-17).** white solid; m.p.= 78-80°C. ¹H NMR (400 MHz, CDCl₃) δ 7.65 (d, *J* = 2.8 Hz, 1H), 7.59 (d, *J =* 11.6 Hz, 1H), 7.36 - 7.26 (m, 1H), 6.91 (t, *J =* 8.4 Hz, 2H), 6.07 (dd, *J =* 12.0, 9.2 Hz, 1H), 5.68 (d, *J =* 12.0 Hz, 1H), 4.36 (s, 2H), 3.95 (q, *J =* 7.2 Hz, 2H), 3.80 (dd, *J =* 17.2, 12.0 Hz, 1H), 3.63 (dd, *J =* 17.2, 9.2 Hz, 1H), 3.40 - 3.25 (m, 1H), 3.17 - 2.76 (m, 2H), 2.18 (dd, *J =* 13.6, 3.6 Hz, 2H), 1.85 - 1.70 (m, 2H), 1.34 (t, *J =* 7.2 Hz, 2H), 1.26 (t, *J =* 7.2 Hz, 1H). ¹³C NMR (100 MHz, CDCl₃) δ 174.82, 166.24, 162.63 (d, *J* = 7.5 Hz), 162.01, 160.13 (d, *J* = 7.5 Hz), 152.25, 145.10, 130.54 (t, *J =* 10.6 Hz), 117.71, 117.67, 115.77 (t, *J =* 16.2 Hz), 112.02 - 111.68 (m), 95.38, 72.87 (t, *J= 2.9* Hz), 67.51, 41.55, 40.71, 14.75. HRMS (MALDI) calculated value C₂₂H₂₄F₂N₃O₃S [M+H]⁺: 448.1501, found 448.1504. 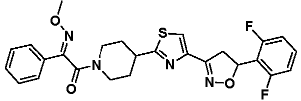 **Formula(I-18).** white solid; m.p.= 77-79°C. ¹H NMR (400 MHz, CDCl₃) δ 7.69 - 7.60 (m, 3H), 7.41 - 7.38 (m, 3H), 7.35 - 7.25 (m, 1H), 6.98 - 6.87 (m, 2H), 6.13 - 6.01 (m, 1H), 4.77 (dd, *J =* 35.2, 13.6 Hz, 1H), 4.02 (d, *J =* 1.6 Hz, 3H), 3.86 - 3.77 (m, 1H), 3.76 - 3.66 (m, 1H), 3.61 (dd, *J =* 17.6, 8.8 Hz, 1H), 3.38 - 3.28 (m, 1H), 3.32 - 2.93 (m, 2H), 2.35 - 2.07 (m, 2H), 1.99 - 1.54 (m, 2H). ¹³C NMR (100 MHz, CDCl₃) δ174.48, 163.25, 162.63 (d, *J =* 7.4 Hz), 160.14 (d, *J =* 7.6 Hz), 152.59, 152.25, 145.24, 130.74, 130.58 (t, *J* = 10.0 Hz), 130.34, 128.88, 128.85, 126.21, 126.18, 117.77, 115.76 (t, *J* = 16.2 Hz), 111.87 (d, *J* = 24.7 Hz), 111.87 (d, *J =* 14.0 Hz), 72.89, 62.87, 46.07, 41.53, 40.54, 40.26, 32.95, 31.93, 29.69. HRMS (MALDI) calculated value C₂₆H₂₅F₂N₄O₃S [M+H]⁺ 511.1610, found 511.1610. 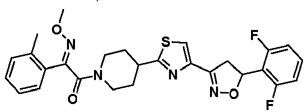 **Formula(I-19).** white solid; m.p.= 74-76°C. ¹H NMR (400 MHz, CDCl₃) δ 7.66 (s, 1H), 7.36 - 7.17 (m, 5H), 6.92 (t, *J* = 8.0 Hz, 2H), 6.08 (dd, *J =* 12.0, 9.2 Hz, 1H), 4.67 (d, *J =* 13.6 Hz, 1H), 4.51 - 4.43 (m, 1H), 3.94 (s, 3H), 3.80 (dd, *J =* 17.2, 12.0 Hz, 1H), 3.63 (dd, *J =* 17.2, 9.2 Hz, 1H), 3.31 - 3.41 (m, 2H), 3.03 - 2.91 (m, 1H), 2.33 (s, 3H), 2.29 - 2.18 (m, 2H), 1.93 - 1.80 (m, 2H). ¹³C NMR (100 MHz, CDCl₃) δ 174.50, 163.76, 162.65 (d, *J =* 8.0 Hz), 160.15 (d, *J =* 8.0 Hz), 152.26, 151.50, 145.22, 136.49, 131.04, 130.59 (t, *J =* 10.0 Hz), 130.27, 129.38, 127.29, 125.55, 117.80, 115.78 (t, *J* = 16.0 Hz), 112.01 - 111.76 (m), 72.91 (t, *J* = 3.0 Hz), 62.74, 46.77, 41.91, 41.55, 40.54, 32.82, 31.85, 20.21. HRMS (MALDI) calculated value C₂₇H₂₇F₂N₄O₃S [M+H]⁺ 525.1766, found 525.1773. 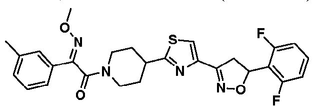 **Formula(I-20).** white solid; m.p.= 81-83°C. ¹H NMR (400 MHz, CDCl₃) δ 7.69 (d, *J =* 11.6 Hz, 1H), 7.55 - 7.40 (m, 2H), 7.38 - 7.28 (m, 2H), 7.26 (s, 1H), 6.99 - 6.91 (m, 2H), 6.15 - 6.06 (m, 1H), 4.81 (dd, *J* = 39.6, 13.6 Hz, 1H), 4.05 (d, *J* = 1.6 Hz, 3H), 3.84 (dd, *J* = 17.2, 10.8 Hz, 1H), 3.79 - 3.71 (m, 1H), 3.65 (dt, *J =* 17.6, 9.6 Hz, 1H), 3.37 (dt, *J =* 12.0, 5.6 Hz, 1H), 3.33 - 2.97 (m, 2H), 2.40 (s, 3H), 2.32 (t, *J =* 15.6 Hz, 1H), 2.17 (dd, *J =* 27.6, 13.2 Hz, 1H), 2.00 - 1.58 (m, 2H). ¹³C NMR (100 MHz, CDCl₃) δ 174.54, 163.34, 162.63 (d, *J* = 7.3 Hz), 160.13 (d, *J* = 7.4 Hz), 152.80, 152.24, 145.23, 138.63, 131.22, 130.59(t, *J* = 10.0 Hz), 128.78, 128.75, 126.62, 123.45, 117.77, 115.75 (t, *J* = 16.1 Hz), 112.00 - 111.75 (m), 72.89, 62.65, 46.11, 41.52, 40.58, 40.26, 32.97, 31.97, 21.43, 21.41. HRMS (MALDI) calculated value C₂₇H₂₇F₂N₄O₃S [M+H]⁺ 525.1766, found 525.1771. 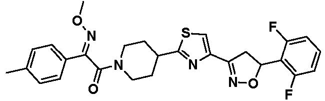 **Formula(I-21).** white solid; m.p.= 85-87°C. ¹H NMR (400 MHz, CDCl₃) δ 7.66 (d, *J =* 11.2 Hz, 1H), 7.52 (dd, *J* = 13.2, 8.0 Hz, 2H), 7.31 (q, *J* = 7.2 Hz, 1H), 7.19 (d, *J* = 8.0 Hz, 2H), 6.96 - 6.87 (m, 2H), 6.12 - 6.03 (m, 1H), 4.76 (dd, *J* = 32.0, 13.6 Hz, 1H), 4.00 (d, *J* = 2.0 Hz, 3H), 3.86 - 3.76 (m, 1H), 3.76 - 3.67 (m, 1H), 3.67 - 3.56 (m, 1H), 3.39 - 3.29 (m, 1H), 3.28 - 2.94 (m, 2H), 2.36 (d, *J* = 3.6 Hz, 3H), 2.28 (t, *J* = 16.8 Hz, 1H), 2.13 (dd, *J* = 23.2, 13.6 Hz, 1H), 1.99 - 1.52 (m, 2H). ¹³C NMR (150 MHz, DMSO-*d₆*) δ 175.14, 162.31, 161.92 (d, *J =* 7.5 Hz), 160.27 (d, *J =* 7.7 Hz), 152.83, 152.53, 144.78, 144.69, 140.76, 131.80 (t, *J* = 10.6 Hz), 130.08, 130.04, 128.12, 126.26, 120.38, 116.03 (t, *J =* 16.6 Hz), 112.69, 112.54, 72.46, 62.74, 45.70, 41.55, 40.19, 39.78, 33.03, 32.11, 21.34. HRMS (MALDI) calculated value C₂₇H₂₇F₂N₄O₃S [M+H]⁺ 525.1766, found 525.1775. 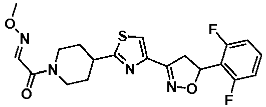 **Formula(I-22).** white solid; m.p.= 123-125°C. ¹H NMR (400 MHz, CDCl₃) 6 7.71 (s, 1H), 7.67 (s, 1H), 7.34 - 7.28 (m, 1H), 6.96 - 6.88 (m, 2H), 6.08 (dd, *J* = 12.0, 9.2 Hz, 1H), 4.67 (d, *J* = 14.4 Hz, 1H), 4.35 (d, *J =* 14.0 Hz, 1H), 3.98 (s, 2H), 3.94 (s, 1H), 3.81 (dd, *J=* 17.2, 12.0 Hz, 1H), 3.63 (dd, *J =* 17.2, 9.2 Hz, 1H), 3.42 - 3.33 (m, 1H), 3.33 - 3.19 (m, 1H), 2.98 - 2.89 (m, 1H), 2.23 (d, *J* = 13.6 Hz, 2H), 1.92 - 1.77 (m, 2H). ¹³C NMR (100 MHz, CDCl₃) δ 174.35, 162.62 (d, *J =* 7.6 Hz), 160.94, 160.12 (d, *J=* 7.5 Hz), 152.23, 145.19, 142.36, 130.60 (t, *J=* 10.6 Hz), 117.81, 115.75 (t, *J=* 16.1 Hz), 112.00 - 111.76 (m), 72.89, 62.76, 46.13, 41.98, 41.55, 40.39, 32.83, 31.90. HRMS (MALDI) calculated value C₂₀H₂₁F₂N₄O₃S [M+H]⁺: 435.1297, found 435.1301. 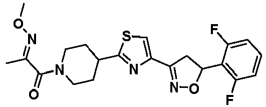 **Formula(I-23).** white solid; m.p.= 142-144°C. ¹H NMR (400 MHz, CDCl₃) δ 7.70 (s, 1H), 7.38 - 7.31 (m, 1H), 6.96 (t, *J* = 8.0 Hz, 2H), 6.12 (dd, *J* = 12.0, 9.2 Hz, 1H), 4.69 (d, *J* = 13.2 Hz, 1H), 4.24 (d, *J =* 13.6 Hz, 1H), 3.97 (s, 3H), 3.85 (dd, *J =* 17.2, 12.0 Hz, 1H), 3.67 (dd, *J =* 17.2, 9.2 Hz, 1H), 3.40 (tt, *J* = 11.2, 4.0 Hz, 1H), 3.32 - 3.22 (m, 1H), 3.01 - 2.91 (m, 1H), 2.25 (d, *J =* 13.2 Hz, 2H), 2.08 (s, 3H), 1.95 - 1.82 (m, 2H). ¹³C NMR (100 MHz, CDCl₃) δ 174.48, 164.89, 162.62 (d, *J =* 8.0 Hz), 160.12 (d, *J =* 7.0 Hz), 152.24, 151.21, 145.18, 130.57 (t, *J =* 10.0 Hz), 117.75, 115.75 (t, *J=* 16.0 Hz), 111.99 - 111.74 (m), 72.87 (t, *J =* 3.0 Hz), 62.23, 46.67, 41.76, 41.54 (t, *J* = 2.0 Hz), 40.54, 32.84, 31.88, 12.96. HRMS (MALDI) calculated value C₂₁H₂₃F₂N₄O₃S [M+H]⁺ 449.1453, found 449.1454. 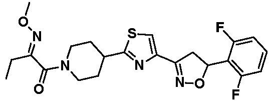 **Formula(I-24).** white solid; m.p.= 58-60°C. ¹H NMR (400 MHz, CDCl₃) δ 7.69 (s, 1H), 7.40 - 7.30 (m, 1H), 6.96 (t, *J* = 8.0 Hz, 2H), 6.11 (dd, *J* = 12.0, 9.2 Hz, 1H), 4.71 (d, *J* = 13.6 Hz, 1H), 4.20 (d, *J* = 13.6 Hz, 1H), 3.94 (s, 3H), 3.84 (dd, *J =* 17.2, 12.0 Hz, 1H), 3.67 (dd, *J =* 17.2, 9.2 Hz, 1H), 3.43 - 3.34 (m, 1H), 3.32 - 3.22 (m, 1H), 3.02 - 2.92 (m, 1H), 2.61 (q, *J =* 7.6 Hz, 2H), 2.25 (d, *J =* 13.6 Hz, 2H), 1.95 - 1.79 (m, 2H), 1.13 (t, *J =* 7.6 Hz, 3H). ¹³C NMR (100 MHz, DMSO-*d₆*) δ 175.16, 163.62, 162.38 (d, *J =* 8.0 Hz), 159.90 (d, *J* = 8.0 Hz), 156.47, 152.55, 144.79, 131.85 (t, *J* = 11.0 Hz), 120.44, 116.08 (t, *J =* 16.0 Hz), 112.79 - 112.54 (m), 72.50 (t, *J =* 3.0 Hz), 62.18, 46.31, 41.61, 41.13, 39.86, 32.98, 32.18, 20.25, 10.06. HRMS (MALDI) calculated value C₂₂H₂₅F₂N₄O₃S [M+H]⁺ 463.1610, found 463.1611. 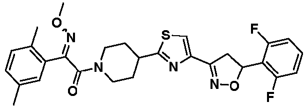 **Formula(I-25).** white solid; m.p.= 72-74°C. ¹H NMR (400 MHz, CDCl₃) δ 7.66 (s, 1H), 7.36 - 7.26 (m, 1H), 7.20 (s, 1H), 7.13 (d, *J =* 8.0 Hz, 1H), 7.08 (dd, *J =* 8.0, 1.6 Hz, 1H), 6.91 (t, *J =* 8.4 Hz, 2H), 6.07 (dd, *J =* 12.0, 9.2 Hz, 1H), 4.73 (s, 1H), 4.01 (s, 3H), 3.78 (dt, *J =* 16.8, 10.4 Hz, 2H), 3.62 (dd, *J =* 17.2, 9.2 Hz, 1H), 3.32 (t, *J =* 11.2 Hz, 1H), 3.19 (s, 1H), 2.98 (s, 1H), 2.50 (s, 3H), 2.30 (s, 3H), 2.29 - 2.11 (m, 2H), 1.84 (s, 2H). ¹³C NMR (100 MHz, CDCl₃) δ162.64 (d, *J =* 7.6 Hz), 160.14 (d, *J* = 7.6 Hz), 153.59, 152.18, 145.18, 135.50, 134.30, 131.82, 130.59 (t, *J* = 10.6 Hz), 130.29, 129.48, 129.35, 117.74, 115.76 (t, *J* = 16.2 Hz), 112.00 - 111.75 (m), 72.91 (t, *J* = 3.0 Hz)., 62.71, 41.56, 41.54, 40.52, 31.93, 21.75, 20.94. HRMS (MALDI) calculated value C₂₈H₂₉F₂N₄O₃S [M+H]⁺ 539.1923, found 539.1925. 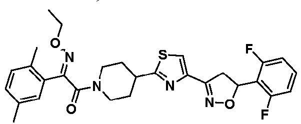 **Formula(I-26).** white solid; m.p.= 70-72°C. ¹H NMR (400 MHz, CDCl₃) δ 7.66 (s, 1H), 7.34 - 7.28 (m, 1H), 7.19 (s, 1H), 7.13 (d, *J =* 8.0 Hz, 1H), 7.07 (dd, *J =* 8.0, 1.6 Hz, 1H), 6.92 (t, *J =* 8.0 Hz, 2H), 6.07 (dd, *J =* 12.0, 9.2 Hz, 1H), 4.74 (d, *J =* 44.0 Hz, 1H), 4.26 (q, *J =* 7.2 Hz, 2H), 3.79 (dd, *J =* 17.2, 12.0 Hz, 2H), 3.62 (dd, *J =* 17.0, 9.2 Hz, 1H), 3.38 - 3.28 (m, 1H), 3.23 - 2.86 (m, 2H), 2.50 (s, 3H), 2.30 (s, 3H), 2.29 - 2.22 (m, 1H), 2.14 (s, 1H), 1.88 - 1.76 (m, 2H), 1.34 (t, *J =* 7.2 Hz, 3H). ¹³C NMR (100 MHz, CDCl₃) δ 162.64 (d, *J* = 7.5 Hz), 160.14 (d, *J* = 7.5 Hz), 153.17, 152.25, 145.23, 135.47, 134.26, 131.81, 130.58 (t, *J =* 10.7 Hz), 130.17, 129.75, 129.29, 117.70, 115.77 (t, *J =* 16.2 Hz), 112.01 - 111.76 (m), 72.90 (t, *J* = 3.0 Hz), 70.45, 41.55, 40.66, 21.87, 20.96, 14.85. HRMS (MALDI) calculated value C₂₉H₃₁F₂N₄O₃S [M+H]⁺ 553.2079, found 553.2083. 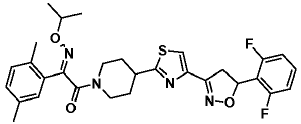 **Formula(I-27).** white solid; m.p.= 69-71°C. ¹H NMR (400 MHz, CDCl₃) δ 7.67 (s, 1H), 7.34 - 7.28 (m, 1H), 7.17 (s, 1H), 7.13 (d, *J =* 8.0 Hz, 1H), 7.10 - 7.04 (m, 1H), 6.91 (t, *J =* 8.4 Hz, 2H), 6.11 - 6.02 (m, 1H), 4.82 (s, 1H), 4.53 - 4.42 (m, 1H), 3.79 (dd, *J =* 17.2, 11.2 Hz, 2H), 3.62 (dd, *J =* 17.2, 9.2 Hz, 1H), 3.32 (d, *J* = 12.0 Hz, 1H), 3.20 - 2.84 (m, 2H), 2.51 (s, 3H), 2.30 (s, 4H), 2.16 (d, *J* = 12.8 Hz, 1H), 1.91 - 1.77 (m, 2H), 1.33 (s, 3H), 1.31 (s, 3H). ¹³C NMR (100 MHz, CDCl₃) δ162.64 (d, *J =* 7.5 Hz), 160.14 (d, *J* = 7.5 Hz), 152.27, 145.22, 135.43, 134.24, 131.80, 130.59 (t, *J* = 10.6 Hz), 130.03, 130.00, 129.25, 117.68, 115.77 (t, *J* = 16.3 Hz), 112.00 - 111.75 (m), 76.52, 72.89, 41.57, 40.81, 22.05, 21.72, 20.97. HRMS (MALDI) calculated value C₃₀H₃₃F₂N₄O₃S [M+H]⁺ 567.2236, found 567.2240. 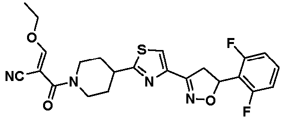 **Formula(I-28).** white solid; m.p.= 106-108°C. ¹H NMR (400 MHz, CDCl₃) δ 7.74 (s, 1H), 7.69 (s, 1H), 7.35 - 7.28 (m, 1H), 6.92 (t, *J* = 8.4 Hz, 2H), 6.08 (dd, *J =* 12.0, 9.2 Hz, 1H), 4.84 (d, *J* = 13.6 Hz, 1H), 4.24 (q, *J* = 7.2 Hz, 2H), 3.85 - 3.69 (m, 2H), 3.63 (dd, *J* = 17.2, 9.2 Hz, 1H), 3.54 (t, *J* = 12.0 Hz, 1H), 3.43 (d, *J =* 25.6 Hz, 2H), 2.30 (d, *J =* 13.6 Hz, 2H), 2.07 - 1.95 (m, 2H), 1.31 (t, *J =* 7.2 Hz, 3H). ¹³C NMR (100 MHz, CDCl₃) δ 172.95, 166.82, 162.61 (d, *J =* 8.0 Hz), 160.11 (d, *J* = 8.0 Hz), 155.60, 152.18, 145.40, 130.62 (t, *J* = 10.0 Hz), 117.95, 117.92, 115.72 (t, *J* = 16.0 Hz), 112.01 - 111.76 (m), 72.92 (t, *J =* 3.0 Hz), 70.05, 60.99, 56.05, 45.65, 41.54, 39.21, 32.73, 31.61, 14.45. HRMS (MALDI) calculated value C₂₃H₂₂F₂N₄NaO₃S [M+Na]⁺: 495.1273, found 495.1270. 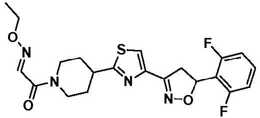 **Formula(I-29).** yellow oil. ¹H NMR (400 MHz, CDCl₃) δ 7.70 (s, 1H), 7.66 (s, 1H), 7.31 (dt, *J* = 12.8, 6.0 Hz, 1H), 6.92 (t, *J* = 8.0 Hz, 2H), 6.08 (t, *J* = 10.4 Hz, 1H), 4.68 (d, *J* = 14.0 Hz, 1H), 4.37 (d, *J =* 14.0 Hz, 1H), 4.31 - 4.20 (m, 2H), 3.81 (dd, *J* = 17.2, 12.0 Hz, 1H), 3.63 (dd, *J* = 17.2, 9.2 Hz, 1H), 3.32 (dt, *J =* 33.6, 11.6 Hz, 2H), 2.94 (t, *J =* 12.4 Hz, 1H), 2.22 (d, *J =* 13.2 Hz, 2H), 1.94 - 1.73 (m, 2H), 1.29 (t, *J* = 6.8 Hz, 3H). ¹³C NMR (100 MHz, CDCl₃) δ 174.32, 162.61 (d, *J* = 7.6 Hz), 161.18, 160.11 (d, *J =* 7.6 Hz), 152.24, 145.21, 142.10, 130.57 (t, *J =* 10.6 Hz), 117.75, 115.76 (t, *J* = 16.1 Hz), 111.99 - 111.74 (m), 72.86 (t, *J =* 3.0 Hz), 70.69, 46.14, 41.98, 41.54, 40.42, 32.84, 31.90, 14.55. HRMS (MALDI) calculated value C₂₁H₂₃F₂N₄O₃S [M+H]⁺ 449.1453, found 449.1455. 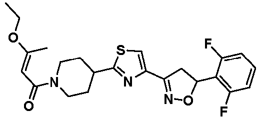 **Formula(I-30).** white solid; m.p.= 124-126°C. ¹H NMR (400 MHz, CDCl₃) δ 7.65 (d, *J =* 3.6 Hz, 1H), 7.36 - 7.26 (m, 1H), 6.92 (t, *J* = 8.4 Hz, 2H), 6.07 (dd, *J* = 12.0, 9.2 Hz, 1H), 5.17 (s, 1H), 4.39 (s, 2H), 3.89 - 3.76 (m, 3H), 3.67 - 3.55 (m, 1H), 3.36 - 3.27 (m, 1H), 3.00 (s, 2H), 2.29 - 2.16 (m, 5H), 1.88 - 1.71 (m, 2H), 1.34 (t, *J* = 6.8 Hz, 3H). ¹³C NMR (100 MHz, CDCl₃) δ 174.94, 167.50, 167.42, 162.64 (d, *J* = 7.4 Hz), 160.14 (d, *J* = 7.3 Hz), 152.26, 145.13, 130.54 (t, *J* = 10.6 Hz), 117.65, 115.79 (t, *J=* 16.1 Hz), 111.99 - 111.74 (m), 91.45, 72.87, 63.19, 41.57, 40.80, 32.64, 19.05, 14.37. HRMS (MALDI) calculated value C₂₃H₂₆F₂N₃O₃S [M+H]⁺ 462.1657, found 462.1658. 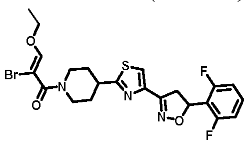 **Formula** (**I-53**) . white solid; m.p. = 86-88°C. ¹H NMR (400 MHz, CDCl₃) δ 7.66 (s, 1H), 7.36 - 7.24 (m, 2H), 6.91 (t, *J* = 8.0 Hz, 2H), 6.07 (dd, *J* = 12.0, 8.0 Hz, 1H), 4.99 (dd, *J* = 12.0, 8.0 Hz, 1H), 4.62 (d, *J =* 12.0 Hz, 1H), 4.51 (dd, *J =* 20.0, 8.0 Hz, 1H), 3.99 (d, *J =* 12.0 Hz, 1H), 3.83 - 3.73 (m, 2H), 3.38 - 3.20 (m, 2H), 2.87 (ddd, *J* = 13.2, 12.0, 4.0 Hz, 1H), 2.19 (q, *J* = 12.0, 8.4 Hz, 2H), 2.02 - 1.68 (m, 2H), 1.17 (td, *J* = 7.2, 1.6 Hz, 3H). ¹³C NMR(100 MHz, CDCl₃) δ 174.29 (d, *J* = 11.3 Hz), 165.84 (d, *J* = 20.7 Hz), 162.63 (d, *J* = 7.6 Hz), 160.13 (d, *J* = 7.6 Hz), 152.28, 145.21 (d, *J* = 2.4 Hz), 130.56 (t, *J* = 10.6 Hz), 117.68 (d, *J* = 8.7 Hz), 115.78 (t, *J* = 16.3 Hz), 111.97 (d, *J* = 5.9 Hz), 111.77 (d, *J* = 5.9 Hz), 103.34 (d, *J* = 38.4 Hz), 72.87, 65.81 (d, *J* = 12.4 Hz), 64.21 (d, *J* = 41.2 Hz), 46.12 (d, *J =* 32.9 Hz), 42.59, 42.19 (d, *J =* 38.0 Hz), 41.58, 32.36, 31.65, 15.21. HRMS(MALDI) calculated value C₂₂H₂₃BrF₂N₃O₃S [M+H]⁺ : 526.0606, found 526.0604. 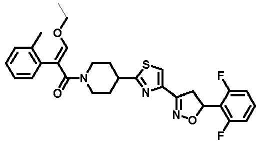 **Formula** (**I-59)** . white solid; m.p. = 121-123°C. ¹H NMR (400 MHz, CDCl₃) δ 7.55 (s, 1H), 7.21 - 7.16 (m, 2H), 7.15 - 7.07 (m, 3H), 7.01 (s, 1H), 6.84 (t, *J =* 8.4 Hz, 2H), 5.99 (dd, *J =* 12.0, 9.2 Hz, 1H), 3.96 (m, 4H), 3.71 (dd, *J =* 17.2, 12 Hz, 1H), 3.53 (dd, *J* = 17.2, 9.2 Hz, 1H), 3.15 - 3.03 (m, 1H), 2.72 (t, *J =* 12.0 Hz, 2H), 2.20 (s, 3H), 1.86 (s, 2H), 1.38 (s, 2H), 1.23 (t, *J =* 7.1 Hz, 3H). ¹³C NMR (100 MHz, CDCl₃) δ 174.88, 170.07, 162.55 (d, *J =* 7.6 Hz), 160.05 (d, *J =* 7.5 Hz), 153.67, 152.17, 144.94, 136.42, 133.92, 130.79 - 130.27 (m), 130.06, 127.32, 125.60, 117.54, 114.22, 111.88 (d, *J =* 5.8 Hz), 111.68 (d, *J =* 5.6 Hz), 72.76 (d, *J =* 3.8 Hz), 69.83, 41.47, 40.44, 32.01, 20.14, 15.32. HRMS(MALDI) calculated value C₂₉H₂₉F₂N₃NaO₃S [M+Na]⁺ :560.1790, found 560.1790.

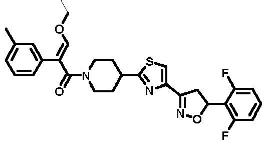

**Formula (I-60)** . white solid; m.p. = 123-124°C. ¹H NMR (400 MHz, CDCl₃) δ 7.56 (d, *J =* 3.5 Hz, 1H), 7.25 (dd, *J =* 8.4, 6.4 Hz, 1H), 7.19 (s, 2H), 7.18 - 7.11 (m, 1H), 7.02 (s, 1H), 6.98 (t, *J =* 6.2 Hz, 1H), 6.85 (t, *J =* 8.0 Hz, 2H), 6.10 - 5.91 (m, 1H), 4.63 (d, *J =* 12.4 Hz, 1H), 3.94 - 3.82 (m, 1H), 3.73 (dd, *J =* 16.4, 4.0 Hz, 1H), 3.66 (s, 2H), 3.54 (ddd, *J =* 17.2, 9.2, 2.4 Hz, 1H), 3.18 (tdd, *J =* 14.8, 7.2, 3.6 Hz, 1H), 3.12 - 3.02 (m, 1H), 2.81 - 2.68 (m, 1H), 2.26 (s, 3H), 2.08 (d, *J* = 14.4 Hz, 1H), 1.96 (d, *J =* 10.4 Hz, 1H), 1.75 - 1.52 (m, 3H), 1.50 - 1.38 (m, 1H), 1.19 (t, *J =* 7.2 Hz, 1H)_{.} ¹³C NMR (100 MHz, CDCl₃) δ 174.40, 174.16, 165.17, 164.81, 163.91 (d, *J* = 6.2 Hz), 160.00, 159.42, 145.68, 141.94, 134.78, 131.20, 130.08, 129.68, 128.59, 126.78, 125.40, 124.92 (d, *J* = 8.3 Hz), 124.07, 104.20, 61.51, 51.40, 44.95, 42.19 - 41.80 (m), 40.45, 38.61, 32.52, 31.68, 21.75, 14.37. HRMS(MALDI) calculated value C₂₉H₂₉F₂N₃NaO₃S [M+Na]⁺ :560.1790, found 560.1792. 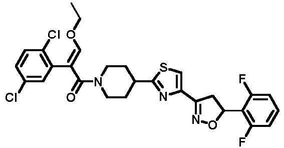 **Formula (I-63).** white solid; m.p. = 120-122°C. ¹H NMR (400 MHz, CDCl₃) δ 7.66 (s, 1H), 7.46 (d, *J* = 2.8 Hz, 1H), 7.38 - 7.27 (m, 2H), 7.26 - 7.16 (m, 1H), 7.07 (s, 1H), 6.94 (t, *J* = 8.4 Hz, 2H), 6.14 - 6.03 (m, 1H), 4.24 (d, *J* = 12.4 Hz, 1H), 4.15-4.06 (m, 2H), 3.81 (dd, *J* = 21.2, 8.8 Hz, 2H), 3.63 (dd, *J =* 17.6, 9.6 Hz, 1H), 3.23 (d, *J =* 11.6 Hz, 1H), 2.94 (t, *J =* 12.8 Hz, 2H), 1.63 (q, *J =* 12.4 Hz, 2H), 1.34 (t, *J* = 7.2 Hz, 3H), 1.26 (d, *J* = 16.8 Hz, 2H). ¹³C NMR (100 MHz, CDCl₃) δ 174.86, 168.68, 162.68 (d, *J* = 7.5 Hz), 160.18 (d, *J* = 7.4 Hz), 154.94, 152.28, 145.12, 135.01, 132.40, 131.87, 130.86, 130.58 (t, *J =* 10.6 Hz), 128.60, 117.70, 112.02, 111.77, 111.25, 72.92, 70.44, 41.60, 40.56, 32.17, 15.42. HRMS(MALDI) calculated value C₂₈H₂₆Cl₂F₂N₃O₃S [M+H]⁺ : 592.1035, found 592.1041. 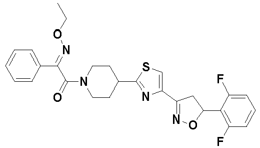 **Formula (I-64)** . white solid; m.p. =74-75°C. ¹H NMR (400 MHz, CDCl₃) δ 7.68 - 7.60 (m, 3H), 7.40 - 7.36 (m, 3H), 7.30 (ddt, *J* = 8.4, 6.4, 2.0 Hz, 1H), 6.91 (td, *J* = 8.4, 2.8 Hz, 2H), 6.07 (ddd, *J =* 12.0, 9.2, 5.2 Hz, 1H), 4.78 (dd, *J =* 50.8, 13.6 Hz, 1H), 4.33 - 4.22 (m, 2H), 3.86 - 3.66 (m, 2H), 3.61 (dt, *J* = 17.2, 8.4 Hz, 1H), 3.39 - 3.27 (m, 1H), 3.10 (tdd, *J* = 14.4, 11.6, 2.8 Hz, 1H), 3.30 - 2.90 (m, 1H), 2.28 (dd, *J* = 23.6, 13.2 Hz, 1H), 2.12 (d, *J* = 10.4 Hz, 1H), 2.01 - 1.78 (m, 2H), 1.37 - 1.30 (m, 3H). ¹³C NMR (100 MHz, CDCl₃) δ 174.36 (d, *J* = 41.1 Hz), 163.51, 163.08, 160.14 (d, *J* = 7.5 Hz), 152.18, 151.96, 145.22 (d, *J* = 2.0 Hz), 130.99 (d, *J* = 7.6 Hz), 130.58 (t, *J* = 10.6 Hz), 130.20 (d, *J =* 1.6 Hz), 128.83 (d, *J =* 2.9 Hz), 126.15 (d, *J =* 2.7 Hz), 117.70 (d, *J =* 6.8 Hz), 115.77 (t, *J =* 16.2 Hz), 111.97 (d, *J* = 5.8 Hz), 111.78 (d, *J* = 5.6 Hz), 73.06 - 72.63 (m), 70.67, 46.11, 45.33, 41.54, 40.52 (dd, *J* = 38.6, 9.6 Hz), 32.97, 32.50 - 31.83 (m), 14.76 (d, *J* = 20.8 Hz). HRMS(MALDI) calculated value C₂₇H₂₆F₂N₄NaO₃S [M+Na]⁺ :547.1586, found 547.1583. 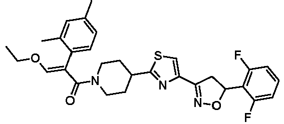 **Formula (I-78)** . white solid; m.p.= 78-80 °C. ¹H NMR (400 MHz, CDCl₃) δ 7.71 - 7.55 (m, 1H), 7.34 - 7.20 (m, 3H), 7.12 (t, *J* = 6.8 Hz, 2H), 6.91 (t, *J* = 8.4 Hz, 2H), 6.06 (q, *J* = 10.4 Hz, 1H), 4.97 (dd, *J* = 8.0, 1.2 Hz, 1H), 4.65 (dd, *J* = 36.0, 13.6 Hz, 1H), 4.12 - 3.93 (m, 2H), 3.88 - 3.70 (m, 2H), 3.62 (dd, *J =* 19.6, 9.2 Hz, 1H), 3.21 (d, *J =* 11.2 Hz, 1H), 3.15 (s, 3H), 2.76 (t, *J* = 12.4 Hz, 1H), 2.43 - 2.27 (m, 3H), 2.09 (t, *J =* 20.0 Hz, 2H), 1.95 - 1.70 (m, 2H), 0.91 (t, *J =* 6.8 Hz, 3H). ¹³C NMR (100 MHz, CDCl₃) δ 174.84, 169.95 - 169.10 (m), 162.63 (d, *J =* 7.4 Hz), 160.13 (d, *J =* 7.7 Hz), 152.22, 145.02 (d, *J =* 14.6 Hz), 137.01, 132.23, 130.54 (t, *J =* 10.6 Hz), 129.79 (d, *J =* 6.1 Hz), 129.49 - 129.21 (m), 128.76 (d, *J* = 6.9 Hz), 128.62 (d, *J* = 7.2 Hz), 117.64 (d, *J* = 8.3 Hz), 115.86 (d, *J =* 16.2 Hz), 111.96 (d, *J* = 5.8 Hz), 111.77 (d, *J* = 5.7 Hz), 72.86, 64.66 (d, *J* = 8.4 Hz), 56.16 (d, *J* = 10.1 Hz), 45.43 (d, *J =* 8.2 Hz), 41.62 (d, *J =* 14.2 Hz), 40.47, 32.19 (t, *J =* 39.6 Hz), 21.12, 15.07. HRMS(MALDI) calculated value C₃₀H₃₁F₂N₃NaO₃S [M+Na]⁺ :574.1946, found 574.1947. 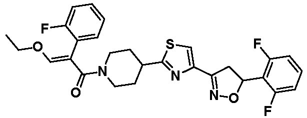 **Formula** (**I-81)** . white solid; m.p.= 82-84°C. ¹H NMR (400 MHz, CDCl₃) δ 7.63 (s, 1H), 7.48 (td, *J* = 7.2, 1.6 Hz, 1H), 7.34 - 7.27 (m, 1H), 7.25 - 7.21 (m, 1H), 7.17 - 7.10 (m, 1H), 7.05 (ddd, *J =* 10.4, 8.0, 1.2 Hz, 1H), 6.95 - 6.87 (m, 3H), 6.06 (dd, *J* = 12.0, 9.2 Hz, 1H), 4.21 (s, 1H), 4.12 (qd, *J =* 7.2, 1.6 Hz, 1H), 4.04 (q, *J* = 7.2 Hz, 2H), 3.78 (dd, *J* = 17.2, 12.0 Hz, 1H), 3.60 (dd, *J* = 17.2, 9.2 Hz, 1H), 3.26 - 3.12 (m, 1H), 2.89 (t, *J* = 12.4 Hz, 2H), 2.00 (s, 2H), 1.57 (s, 2H), 1.31 (td, *J* = 6.8, 2.8 Hz, 3H). ¹³C NMR (100 MHz, CDCl₃) δ 174.98, 169.39, 161.38 (dd, *J* = 251.5, 7.6 Hz), 160.49, 158.02, 153.00, 152.26, 145.04, 131.10 (d, *J* = 3.6 Hz), 130.56 (t, *J* = 10.6 Hz), 128.95 (d, *J* = 8.3 Hz), 124.01 (d, *J =* 3.3 Hz), 122.28 (d, *J =* 14.4 Hz), 117.70, 115.98 - 115.36 (m), 111.99, 111.74, 108.77, 72.86, 70.10, 41.54, 40.57, 32.11, 15.36. HRMS(MALDI) calculated value C₂₈H₂₆F₃N₃NaO₃S [M+Na]⁺ :564.1539, found 564.1534. 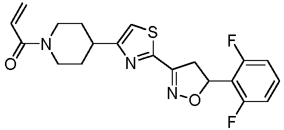 **Formula (I-134)** . light yellow oil. ¹H NMR (400 MHz, CDCl₃) δ 7.36 - 7.28 (m, 1H), 7.00 (s, 1H), 6.96 - 6.89 (m, 2H), 6.61 (dd, *J =* 16.8, 10.6 Hz, 1H), 6.28 (dd, *J =* 16.8, 1.6 Hz, 1H), 6.13 (dd, *J =* 12.0, 9.2 Hz, 1H), 5.69 (dd, *J =* 10.6, 2.0 Hz, 1H), 4.81 - 4.69 (m, 1H), 4.15 - 4.04 (m, 1H), 3.87 (dd, *J =* 17.6, 12.0 Hz, 1H), 3.68 (dd, *J =* 17.4, 9.2 Hz, 1H), 3.28 - 3.13 (m, 1H), 3.07 (tt, *J =* 11.6, 3.6 Hz, 1H), 2.86 - 2.73 (m, 1H), 2.12 (d, *J =* 13.2 Hz, 2H), 1.75 - 1.64 (m, 2H). HRMS(MALDI) calculated value C₂₀H₂₀F₂N₃O₂S [M+H]⁺: 404.1239, found 404.1242. 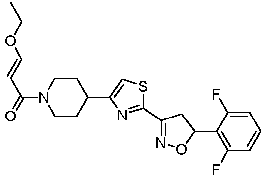 **Formula (I-135)** . white solid; m.p.= 81-83°C. ¹H NMR (400 MHz, CDCl₃) δ 7.58 (d, *J* = 11.6 Hz, 1H), 7.35 - 7.27 (m, 1H), 7.01 - 6.98 (m, 1H), 6.96 - 6.89 (m, 2H), 6.12 (dd, *J* = 12.0, 9.2 Hz, 1H), 5.70 (d, *J =* 11.6 Hz, 1H), 4.72 (d, *J* = 13.6 Hz, 1H), 3.97 - 3.82 (m, 3H), 3.72 - 3.64 (m, 2H), 3.26 - 3.12 (m, 1H), 3.07 - 3.01 (m, 1H), 2.83 - 2.69 (m, 1H), 2.12 - 2.07 (m, 2H), 1.72 - 1.65 (m, 2H), 1.33 (t, *J* = 7.2 Hz, 3H). ¹³C NMR (100 MHz, CDCl₃) δ 166.21, 162.54 (d, *J* = 7.6 Hz), 161.80, 161.20, 160.04 (d, *J* = 7.5 Hz), 156.72, 153.31, 130.75 (t, *J* = 10.6 Hz), 115.34 (t, *J* = 16.0 Hz), 114.32, 111.96 (d, *J =* 5.9 Hz), 111.77 (d, *J* = 5.2 Hz), 95.55, 73.94, 67.45, 46.44 - 44.90 (m), 40.56, 38.69, 32.15, 31.33, 27.06, 14.72. HRMS(MALDI) calculated value C₂₂H₂₄F₂N₃O₃S[M+H]⁺ : 448.1501, found 448.1501. 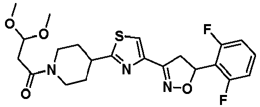 **Formula(II-1).** white solid; m.p.= 96-98°C. ¹H NMR (400 MHz, CDCl₃) δ 7.66 (s, 1H), 7.35 - 7.27 (m, 1H), 6.92 (t, *J =* 8.0 Hz, 2H), 6.07 (dd, *J* = 12.0, 9.2 Hz, 1H), 4.84 (t, *J* = 5.6 Hz, 1H), 4.69 (d, *J* = 13.6 Hz, 1H), 4.02 (d, *J* = 13.6 Hz, 1H), 3.80 (dd, *J* = 17.2, 12.0 Hz, 1H), 3.63 (dd, *J* = 17.2, 9.2 Hz, 1H), 3.42 (d, *J =* 2.4 Hz, 6H), 3.35 - 3.27 (m, 1H), 3.20 (t, *J =* 12.8 Hz, 1H), 2.79 (t, *J =* 12.8 Hz, 1H), 2.71 (t, *J =* 5.2 Hz, 2H), 2.18 (t, *J =* 13.6 Hz, 2H), 1.87 - 1.68 (m, 2H). ¹³C NMR (100 MHz, CDCl₃) δ 174.62, 167.84, 162.62 (d, *J =* 7.6 Hz), 160.12 (d, *J =* 7.5 Hz), 152.26, 145.18, 130.55 (t, *J* = 10.7 Hz), 117.66, 115.77 (t, *J =* 16.1 Hz), 111.98 - 111.73 (m), 103.33, 72.85 (t, *J =* 3.0 Hz), 54.82, 54.58, 45.76, 41.56 (d, *J* = 2.4 Hz), 41.44, 40.50, 37.75, 32.80, 32.09. HRMS (MALDI) calculated value C₂₂H₂₅F₂N₃NaO₄S [M+Na]⁺ 488.1426, found 488.1426. 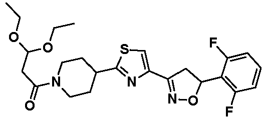 **Formula(II-2).** yellow oil. ¹H NMR (400 MHz, CDCl₃) δ 7.69 (s, 1H), 7.38 - 7.30 (m, 1H), 6.95 (t, *J* = 8.0 Hz, 2H), 6.10 (dd, *J* = 12.0, 9.2 Hz, 1H), 4.98 (t, *J* = 5.6 Hz, 1H), 4.73 (d, *J* = 13.6 Hz, 1H), 4.11 (d, *J =* 12.0 Hz, 1H), 3.89 - 3.72 (m, 3H), 3.70 - 3.57 (m, 3H), 3.39 - 3.29 (m, 1H), 3.23 (t, *J =* 12.8 Hz, 1H), 2.86 - 2.69 (m, 3H), 2.20 (s, 2H), 1.93 - 1.71 (m, 2H), 1.25 (t, *J =* 7.2 Hz, 6H). ¹³C NMR (100 MHz, CDCl₃) δ 174.67, 168.07, 162.62 (d, *J* = 7.6 Hz), 160.12 (d, *J* = 7.7 Hz), 152.29, 145.16, 130.55 (t, *J =* 10.6 Hz), 117.60, 115.78 (t, *J* = 16.1 Hz), 111.98 - 111.73 (m), 101.72, 72.85 (t, *J* = 3.0 Hz), 63.35, 63.15, 45.96, 41.57, 41.45, 40.57, 38.76, 32.81, 32.20, 15.40, 15.38. HRMS (MALDI) calculated value C₂₄H₂₉F₂N₃NaO₄S [M+Na]⁺ 516.1739, found 516.1738. 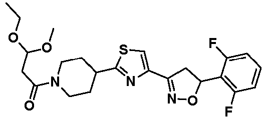 **Formula(II-3).** yellow oil. ¹H NMR (400 MHz, CDCl₃) δ 7.66 (s, 1H), 7.35 - 7.26 (m, 1H), 6.92 (t, *J* = 8.0 Hz, 2H), 6.07 (dd, *J* = 12.0, 9.2 Hz, 1H), 4.93 - 4.87 (m, 1H), 4.73 - 4.63 (m, 1H), 4.09 - 4.01 (m, 1H), 3.80 (dd, *J =* 17.2, 12.0 Hz, 1H), 3.76 - 3.68 (m, 1H), 3.67 - 3.54 (m, 2H), 3.42 (d, *J* = 3.2 Hz, 3H), 3.35 - 3.26 (m, 1H), 3.25 - 3.16 (m, 1H), 2.85 - 2.76 (m, 1H), 2.76 - 2.66 (m, 2H), 2.23 - 2.13 (m, 2H), 1.86 - 1.67 (m, 2H), 1.22 (t, *J* = 7.2 Hz, 3H). ¹³C NMR (100 MHz, CDCl₃) δ 174.66, 168.06, 162.63 (d, *J* = 7.6 Hz), 160.13 (d, *J* = 7.6 Hz), 152.27, 145.16, 130.54 (t, *J* = 10.6 Hz), 117.63, 115.77 (t, *J* = 16.2 Hz), 111.98 - 111.73 (m), 102.54, 72.86 (t, *J* = 3.0 Hz), 63.45, 54.52, 45.90, 41.56, 41.49, 40.52, 38.16, 32.80, 32.10, 15.35 (d, *J* = 2.9 Hz). HRMS (MALDI) calculated value C₂₃H₂₈F₂N₃O₄S [M+H]⁺: 480.1763, found 480.1761. 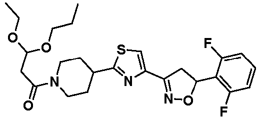 **Formula(II-4).** yellow oil. ¹H NMR (400 MHz, CDCl₃) δ 7.70 (s, 1H), 7.39 - 7.31 (m, 1H), 6.95 (t, *J=* 8.0 Hz, 2H), 6.11 (dd, *J =* 12.0, 9.2 Hz, 1H), 4.98 (q, *J =* 4.8 Hz, 1H), 4.77 - 4.69 (m, 1H), 4.15 - 4.08 (m, 1H), 3.90 - 3.73 (m, 2H), 3.70 - 3.58 (m, 3H), 3.54 - 3.46 (m, 1H), 3.39 - 3.29 (m, 1H), 3.23 (t, *J =* 12.8 Hz, 1H), 2.86 - 2.70 (m, 3H), 2.20 (s, 2H), 1.84 (s, 2H), 1.64 (q, *J =* 7.2 Hz, 2H), 1.25 (t, *J =* 7.2 Hz, 3H), 0.99 - 0.92 (m, 3H). ¹³C NMR (100 MHz, CDCl₃) δ 174.66, 168.08, 162.60 (d, *J* = 7.7 Hz), 160.10 (d, *J =* 7.5 Hz), 152.27, 145.14, 130.54 (t, *J =* 10.6 Hz), 117.62, 115.76 (t, *J =* 16.2 Hz), 111.96 - 111.71 (m), 101.80 (d, *J =* 7.1 Hz), 72.82 (t, *J =* 2.9 Hz), 69.43 (d, *J =* 16.3 Hz), 63.15 (d, *J* = 20.2 Hz), 45.95 (d, *J* = 6.2 Hz), 41.55, 41.42 (d, *J* = 4.0 Hz), 40.55 (d, *J* = 3.1 Hz), 38.65, 32.80, 32.17, 23.09, 15.37 (d, *J =* 2.9 Hz), 10.63 (d, *J =* 2.2 Hz). HRMS (MALDI) calculated value C₂₅H₃₁F₂N₃NaO₄S [M+Na]⁺ 530.1896, found 530.1899. 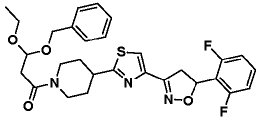 **Formula(II-5).** yellow oil. ¹H NMR (400 MHz, CDCl₃) δ 7.70 (s, 1H), 7.38 (t, *J =* 4.8 Hz, 4H), 7.35 - 7.32 (m, 2H), 6.96 (t, *J =* 8.0 Hz, 2H), 6.11 (dd, *J =* 12.0, 9.2 Hz, 1H), 5.15 (t, *J =* 5.6 Hz, 1H), 4.81 - 4.70 (m, 2H), 4.64 (dd, *J =* 11.6, 4.8 Hz, 1H), 4.08 (d, *J =* 13.6 Hz, 1H), 3.90 - 3.75 (m, 2H), 3.71 - 3.60 (m, 2H), 3.31 (d, *J* = 4.4 Hz, 1H), 3.19 (q, *J =* 12.0 Hz, 1H), 2.92 - 2.73 (m, 3H), 2.19 (d, *J= 13.6* Hz, 2H), 1.89 - 1.68 (m, 2H), 1.27 (t, *J =* 7.2 Hz, 3H). ¹³C NMR (100 MHz, CDCl₃) δ174.64, 167.91, 162.64 (d, *J =* 7.6 Hz), 160.14 (d, *J =* 7.6 Hz), 152.30, 145.18, 137.99, 130.55 (t, *J =* 10.6 Hz), 128.40, 127.83, 127.75, 127.69, 117.60, 115.80 (t, *J =* 16.2 Hz), 111.99 - 111.74 (m), 101.52, 72.86 (t, *J =* 3.2 Hz), 69.35, 63.09, 45.92, 41.58, 40.52, 38.70, 32.69, 32.00, 15.40, 15.38. HRMS (MALDI) calculated value C₂₉H₃₁F₂N₃NaO₄S [M+Na]⁺ 530.1896, found 530.1897. 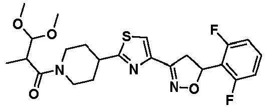 **Formula(II-6).** white solid; m.p.= 58-60°C. ¹H NMR (400 MHz, CDCl₃) δ 7.66 (s, 1H), 7.34 - 7.27 (m, 1H), 6.92 (t, *J =* 8.0 Hz, 2H), 6.07 (dd, *J =* 12.0, 9.2 Hz, 1H), 4.74 (d, *J =* 13.6 Hz, 1H), 4.51 (dd, *J* = 15.2, 8.0 Hz, 1H), 4.10 (dd, *J i=* 25.2, 13.6 Hz, 1H), 3.81 (dd, *J* = 17.2, 12.0 Hz, 1H), 3.63 (dd, *J* = 17.2, 9.2 Hz, 1H), 3.42 (d, *J =* 6.8 Hz, 6H), 3.32 (s, 1H), 3.19 (dd, *J* = 22.8, 11.4 Hz, 1H), 3.05 (q, *J* = 7.2 Hz, 1H), 2.89 - 2.69 (m, 1H), 2.21 (dd, *J =* 27.9, 12.8 Hz, 2H), 1.92 - 1.65 (m, 2H), 1.16 (dd, *J* = 7.2, 3.2 Hz, 3H). ¹³C NMR (100 MHz, CDCl₃) δ 175.00, 172.25, 162.64 (d, *J =* 7.8 Hz), 160.14 (d, *J* = 7.5 Hz), 152.27, 145.15, 130.55 (t, *J* = 10.6 Hz), 117.63, 115.79 (t, *J* = 16.1 Hz), 111.99 - 111.74 (m), 107.72, 72.88, 56.80, 53.75, 45.82, 41.84, 41.59, 40.78, 39.44, 32.95, 32.38, 13.81. HRMS (MALDI) calculated value C₂₃H₂₇F₂N₃NaO₄S [M+Na]⁺ 502.1583, found 502.1583. 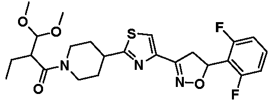 **Formula(II-7).** yellow oil. ¹H NMR (400 MHz, CDCl₃) δ 7.66 (s, 1H), 7.38 - 7.24 (m, 1H), 6.92 (t, *J =* 8.0 Hz, 2H), 6.07 (dd, *J =* 12.0, 9.2 Hz, 1H), 4.74 (dd, *J =* 32.8, 13.6 Hz, 1H), 4.48 (dd, *J =* 8.0, 5.6 Hz, 1H), 4.23 - 4.10 (m, 1H), 3.80 (dd, *J =* 17.2, 12.0 Hz, 1H), 3.63 (dd, *J =* 17.2, 9.2 Hz, 1H), 3.45 - 3.34 (m, 6H), 3.34 - 3.26 (m, 1H), 3.26 - 3.15 (m, 1H), 3.02 - 2.93 (m, 1H), 2.92 - 2.73 (m, 1H), 2.29 - 2.12 (m, 2H), 1.91 - 1.59 (m, 4H), 0.92 - 0.81 (m, 3H). ¹³C NMR (100 MHz, CDCl₃) δ 175.09, 171.50, 162.63 (d, *J =* 7.5 Hz), 160.13 (d, *J =* 7.6 Hz), 152.29, 145.17, 130.55 (t, *J =* 10.6 Hz), 117.61, 115.77 (t, *J* = 16.1 Hz), 111.98 - 111.73 (m), 107.37, 72.86 (t, *J =* 3.0 Hz)., 56.82, 53.99, 46.89, 41.95, 41.56, 40.83, 40.51, 32.99, 32.47, 22.10, 11.63. HRMS (MALDI) calculated value C₂₃H₂₇F₂N₃NaO₄S [M+Na]⁺ 516.1739, found 516.1742. 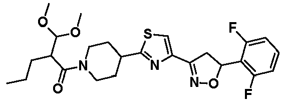 **Formula(II-8).** yellow oil. ¹H NMR (400 MHz, CDCl₃) δ 7.66 (d, *J =* 3.6 Hz, 1H), 7.34 - 7.26 (m, 1H), 6.92 (t, *J =* 8.0 Hz, 2H), 6.07 (dd, *J =* 12.0, 9.2 Hz, 1H), 4.82 - 4.63 (m, 1H), 4.46 (dd, *J =* 8.4, 2.0 Hz, 1H), 4.16 (dd, *J =* 18.8, 14.4 Hz, 1H), 3.80 (dd, *J =* 16.8, 12.0 Hz, 1H), 3.63 (dd, *J =* 17.2, 9.2 Hz, 1H), 3.41 (d, *J =* 2.8 Hz, 3H), 3.37 (s, 1H), 3.34 - 3.25 (m, 1H), 3.25 - 3.15 (m, 1H), 3.09 - 3.00 (m, 1H), 2.93 - 2.70 (m, 1H), 2.28 - 2.13 (m, 2H), 1.94 (s, 2H), 1.80 - 1.69 (m, 2H), 1.58 - 1.47 (m, 1H), 1.43 - 1.33 (m, 1H), 1.33 - 1.17 (m, 2H), 0.98 - 0.88 (m, 3H). ¹³C NMR (100 MHz, CDCl₃) δ175.10, 171.63, 162.63 (d, *J =* 7.5 Hz), 160.13 (d, *J =* 7.7 Hz), 152.29, 145.17, 130.54 (t, *J* = 10.6 Hz), 117.68, 115.78 (t, *J =* 16.2 Hz), 111.98 - 111.73 (m), 107.51, 77.39, 77.07, 76.75, 72.86, 56.86, 53.97, 46.03, 45.19, 41.96, 41.55, 40.84, 33.18, 31.07, 22.61, 20.42, 14.16. HRMS (MALDI) calculated value C₂₅H₃₁F₂N₃NaO₄S [M+Na]⁺ 530.1896, found 530.1899. 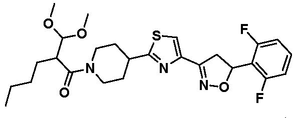 **Formula(II-9).** yellow oil. ¹H NMR (400 MHz, CDCl₃) δ 7.66 (d, *J =* 4.0 Hz, 1H), 7.34 - 7.27 (m, 1H), 6.92 (t, *J =* 8.0 Hz, 2H), 6.07 (dd, *J =* 12.0, 9.2 Hz, 1H), 4.73 (dd, *J =* 42.0, 13.2 Hz, 1H), 4.46 (dd, *J =* 8.0, 3.6 Hz, 1H), 4.16 (t, *J =* 16.7 Hz, 1H), 3.80 (dd, *J =* 17.2, 12.0 Hz, 1H), 3.63 (dd, *J =* 17.2, 9.2 Hz, 1H), 3.47 (s, 1H), 3.44 - 3.39 (m, 3H), 3.37 (s, 1H), 3.35 - 3.27 (m, 1H), 3.26 - 3.15 (m, 1H), 3.06 - 2.99 (m, 1H), 2.93 - 2.72 (m, 1H), 2.18 (t, *J =* 15.6 Hz, 2H), 1.98 - 1.86 (m, 1H), 1.83 - 1.68 (m, 2H), 1.62 - 1.52 (m, 1H), 1.40 - 1.11 (m, 5H), 0.88 (t, *J =* 7.2 Hz, 3H). ¹³C NMR (100 MHz, CDCl₃) δ 174.72, 171.65, 162.63 (d, *J* = 7.5 Hz), 160.14 (d, *J =* 7.7 Hz), 152.28, 145.16, 130.54 (t, *J =* 10.6 Hz), 117.67, 115.78 (t, *J =* 16.3 Hz), 111.98 - 111.73 (m), 107.52, 72.87, 56.87, 53.95, 46.03, 45.36, 41.98, 41.56, 40.85, 33.01, 32.50, 29.41, 28.71, 22.83, 13.99. HRMS (MALDI) calculated value C₂₆H₃₃F₂N₃NaO₄S [M+Na]⁺ 544.2052, found 544.2055. 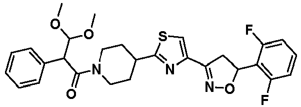 **Formula(II-10).** white solid; m.p.= 69-71°C. ¹H NMR (400 MHz, CDCl₃) δ 7.68 - 7.56 (m, 1H), 7.38 (d, *J* = 5.6 Hz, 2H), 7.36 - 7.26 (m, 4H), 6.91 (t, *J =* 8.4 Hz, 2H), 6.05 (q, *J =* 11.2 Hz, 1H), 4.98 (d, *J* = 8.0 Hz, 1H), 4.65 (dd, *J =* 40.0, 13.6 Hz, 1H), 4.11 - 3.96 (m, 2H), 3.85 - 3.68 (m, 1H), 3.67 - 3.55 (m, 1H), 3.54 - 3.48 (m, 3H), 3.25 - 3.16 (m, 2H), 3.14 (d, *J* = 2.4 Hz, 3H), 2.77 (s, 1H), 2.16 - 2.04 (m, 1H), 1.87 (d, *J* = 12.8 Hz, 1H), 1.82 - 0.77 (m, 2H). ¹³C NMR (100 MHz, CDCl₃) δ 174.72, 169.09, 162.63 (d, *J* = 7.4 Hz), 160.13 (d, *J =* 7.4 Hz), 152.19, 144.95, 135.33, 130.54 (t, *J =* 10.7 Hz), 128.84, 128.76, 127.50, 117.67, 117.59, 115.78 (t, *J =* 16.0 Hz), 111.99 - 111.74 (m), 107.42, 107.30, 72.86, 56.56, 55.50, 53.15, 45.43, 41.76, 41.55, 40.44, 32.22, 31.77. HRMS (MALDI) calculated value C₂₈H₂₉F₂N₃NaO₄S [M+Na]⁺ 564.1739, found 564.1738. 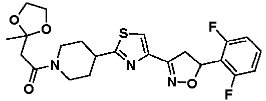 **Formula(II-11).** white solid; m.p.= 126-128°C. ¹H NMR (400 MHz, CDCl₃) δ 7.69 (s, 1H), 7.38 - 7.31 (m, 1H), 6.95 (t, *J =* 8.4 Hz, 2H), 6.11 (dd, *J* = 12.0, 9.2 Hz, 1H), 4.77 (d, *J =* 13.2 Hz, 1H), 4.14 (dd, *J =* 14.4, 7.4 Hz, 1H), 4.05 - 3.94 (m, 4H), 3.84 (dd, *J =* 17.2, 12.0 Hz, 1H), 3.66 (dd, *J =* 17.2, 9.2 Hz, 1H), 3.39 - 3.29 (m, 1H), 3.24 (t, *J =* 12.8 Hz, 1H), 2.89 - 2.75 (m, 3H), 2.22 (s, 2H), 1.81 (dd, *J =* 28.8, 13.6 Hz, 2H), 1.52 (s, 3H). ¹³C NMR (100 MHz, CDCl₃) δ 174.79, 167.48, 162.61 (d, *J* = 7.5 Hz), 160.11 (d, *J* = 7.5 Hz), 152.26, 145.15, 130.56 (t, *J =* 10.7 Hz), 117.69, 115.76 (t, *J =* 16.2 Hz), 111.98 - 111.73 (m), 108.71, 72.85 (t, *J =* 3.0 Hz), 64.78, 64.74, 46.49, 43.10, 41.55, 41.41, 40.51, 32.82, 32.15, 24.66. HRMS (MALDI) calculated value C₂₃H₂₅F₂N₃NaO₄S [M+Na]⁺ 500.1426, found 500.1425. 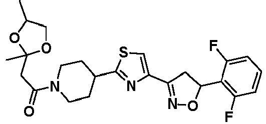 **Formula(II-12).** yellow oil. ¹H NMR (400 MHz, CDCl₃) δ 7.69 (s, 1H), 7.37 - 7.31 (m, 1H), 6.95 (t, *J* = 8.0 Hz, 2H), 6.10 (dd, *J =* 12.0, 9.2 Hz, 1H), 4.76 (d, *J =* 12.8 Hz, 1H), 4.32 - 4.18 (m, 1H), 4.14 - 4.06 (m, 2H), 3.84 (dd, *J =* 17.2, 12.0 Hz, 1H), 3.66 (dd, *J =* 17.2, 9.2 Hz, 1H), 3.54 - 3.40 (m, 1H), 3.38 - 3.29 (m, 1H), 3.24 (s, 1H), 2.86 (s, 1H), 2.85 - 2.76 (m, 2H), 2.21 (s, 2H), 1.81 (dd, *J =* 32.4, 12.7 Hz, 2H), 1.52 (d, *J =* 21.2 Hz, 3H), 1.32 (d, *J =* 6.0 Hz, 3H). ¹³C NMR (100 MHz, CDCl₃) δ 174.79, 167.64, 162.61 (d, *J =* 7.6 Hz), 160.11 (d, *J =* 7.5 Hz), 152.27, 145.14, 130.56 (t, *J =* 10.6 Hz), 117.68, 115.76 (t, *J =* 16.1 Hz), 111.97 - 111.72 (m), 108.95, 72.84, 70.98, 46.49, 44.05, 43.30, 41.54, 41.39, 40.49, 32.77, 32.16, 24.83, 18.26. HRMS (MALDI) calculated value C₂₄H₂₇F₂N₃NaO₄S [M+Na]⁺ 514.1583, found 514.1580. 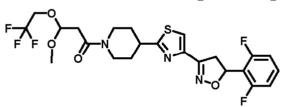 **Formula (II-18)** . light yellow oil. ¹H NMR (400 MHz, CDCl₃) δ 7.65 (s, 1H), 7.31 (tt, *J =* 8.4, 6.6 Hz, 1H), 6.92 (t, *J =* 8.3 Hz, 2H), 6.07 (dd, *J =* 12.0, 9.2 Hz, 1H), 5.07 (q, *J =* 5.6 Hz, 1H), 4.72 - 4.61 (m, 1H), 4.04 - 3.90 (m, 3H), 3.80 (dd, *J* = 17.1, 12.0 Hz, 1H), 3.63 (dd, *J =* 17.1, 9.2 Hz, 1H), 3.44 (d, *J* = 2.8 Hz, 3H), 3.31 (tt, *J =* 11.4, 3.9 Hz, 1H), 3.21 (t, *J* = 11.9 Hz, 1H), 2.86 - 2.68 (m, 3H), 2.24 - 2.12 (m, 2H), 1.87 - 1.66 (m, 2H). ¹³C NMR (100 MHz, CDCl₃) δ 174.53, 167.10 (d, *J =* 2.5 Hz), 162.67 (d, *J =* 7.6 Hz), 160.17 (d, *J =* 7.5 Hz), 152.31, 145.23 (d, *J =* 3.6 Hz), 130.61 (t, *J =* 10.6 Hz), 122.47 (q, *J =* 278.2 Hz), 117.77 (d, *J =* 3.5 Hz), 115.80 (t, *J =* 16.3 Hz), 112.00 (d, *J =* 5.8 Hz), 111.81 (d, *J =* 5.9 Hz), 102.94 (d, *J =* 7.3 Hz), 72.90, 64.75 - 63.33 (m), 54.71 (d, *J =* 20.6 Hz), 45.71 (d, *J* = 3.0 Hz), 41.57, 41.49 (d, *J =* 3.8 Hz), 40.47 (d, *J =* 8.5 Hz), 37.69 (d, *J* = 20.2 Hz), 32.73, 32.05. HRMS(MALDI) calculated value C₂₃H₂₄F₅N₃NaO₄S [M+Na]⁺ : 556.1300, found 556.1301. 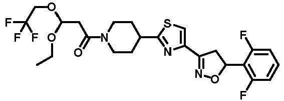 **Formula (II-19)** . light yellow oil. ¹H NMR (400 MHz, CDCl₃) δ 7.66 (s, 1H), 7.42 - 7.19 (m, 1H), 6.92 (t, *J =* 8.2 Hz, 2H), 6.07 (dd, *J =* 11.9, 9.1 Hz, 1H), 5.12 (q, *J =* 5.2 Hz, 1H), 4.68 (d, *J =* 13.3 Hz, 1H), 4.05 - 3.90 (m, 3H), 3.86 - 3.69 (m, 2H), 3.69 - 3.55 (m, 2H), 3.31 (tt, *J =* 11.4, 3.9 Hz, 1H), 3.21 (t, *J =* 12.6 Hz, 1H), 2.86 - 2.67 (m, 3H), 2.19 (t, *J =* 13.7 Hz, 2H), 1.88 - 1.64 (m, 2H), 1.27 - 1.20 (m, 3H). ¹³C NMR (100 MHz, CDCl₃) δ 174.53, 171.20, 167.21, 162.66 (d, *J =* 7.6 Hz), 160.16 (d, *J =* 7.7 Hz), 152.31, 145.22 (d, *J* = 4.2 Hz), 130.59 (t, *J =* 10.6 Hz), 123.87 (q, *J =* 278.1 Hz), 117.73 (d, *J =* 6.2 Hz), 115.81 (t, *J =* 16.2 Hz), 111.99 (d, *J =* 5.7 Hz), 111.80 (d, *J =* 5.6 Hz), 102.01 (d, *J =* 9.7 Hz), 72.90, 64.58 - 63.56 (m), 63.42 (d, *J =* 17.7 Hz), 45.80, 41.55 (d, *J =* 7.6 Hz), 40.51 (d, *J =* 6.4 Hz), 38.19 (d, *J =* 6.6 Hz), 32.73 (d, *J =* 6.6 Hz), 32.10, 15.16. HRMS(MALDI) calculated value C₂₄H₂₆F₅N₃NaO₄S [M+Na]⁺ :570.1456, found 570.1455. 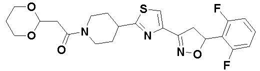 **Formula (II-21)** . light yellow oil. ¹H NMR (400 MHz, CDCl₃) δ 7.68 (s, 1H), 7.36 - 7.30 (m, 1H), 6.99 - 6.88 (m, 2H), 6.09 (dd, *J =* 12.0, 9.2 Hz, 1H), 5.07 (t, *J =* 5.2 Hz, 1H), 4.71 (d, *J =* 13.4 Hz, 1H), 4.15 - 4.09 (m, 2H), 4.05 (d, *J =* 13.4 Hz, 1H), 3.89 - 3.78 (m, 3H), 3.65 (dd, *J =* 17.2, 9.2 Hz, 1H), 3.32 (tt, *J =* 11.6, 3.6 Hz, 1H), 3.25 - 3.16 (m, 1H), 2.85 - 2.76 (m, 1H), 2.73 (dd, *J =* 5.2, 1.6 Hz, 2H), 2.25 - 2.09 (m, 3H), 1.88 - 1.70 (m, 2H), 1.43 - 1.33 (m, 1H). ¹³C NMR (100 MHz, CDCl₃) δ 174.65, 167.33, 162.55 (d, *J =* 7.6 Hz), 160.05 (d, *J =* 7.6 Hz), 152.21, 145.07, 130.47 (t, *J =* 10.6 Hz), 117.60, 115.70 (t, *J =* 16.2 Hz), 111.88 (d, *J* = 5.8 Hz), 111.69 (d, *J =* 5.8 Hz), 99.82, 72.79 (t, *J* = 2.9 Hz), 66.93 (d, *J* = 1.9 Hz), 45.74, 41.48 (t, *J =* 2.1 Hz), 41.39, 40.45, 39.20, 32.71, 31.96, 25.50. HRMS(MALDI) calculated value C₂₃H₂₅F₂N₃NaO₄S [M+Na]⁺ :500.1426, found 500.1424. 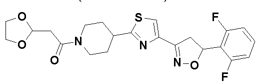 **Formula (II-22)** . light yellow oil. ¹H NMR (400 MHz, CDCl₃) δ 7.64 (s, 1H), 7.36 - 7.25 (m, 1H), 6.91 (t, *J =* 8.4 Hz, 2H), 6.07 (dd, *J =* 12.0, 9.2 Hz, 1H), 5.31 (t, *J =* 4.8 Hz, 1H), 4.71 (d, *J =* 13.6 Hz, 1H), 4.03 - 3.88 (m, 5H), 3.79 (dd, *J =* 17.1, 12.0 Hz, 1H), 3.62 (dd, *J =* 16.8, 9.2 Hz, 1H), 3.34 - 3.24 (m, 1H), 3.24 - 3.16 (m, 1H), 2.85 - 2.74 (m, 3H), 2.18 (t, *J =* 16.2 Hz, 2H), 1.87 - 1.70 (m, 2H). ¹³C NMR (100 MHz, CDCl₃) δ 174.57, 167.24, 162.60 - 162.51 (m), 160.17 - 160.00 (m), 152.21, 145.11, 130.50, 117.65, 115.71, 111.91, 111.71, 101.82, 72.82, 64.94, 45.63, 41.50, 41.30, 40.45, 38.29, 32.71, 31.93. HRMS(MALDI) calculated value C₂₂H₂₃F₂N₃NaO₄S [M+Na]⁺ :486.1270, found 486.1268. 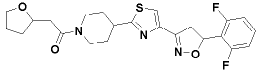 **Formula (II-23)** . light yellow oil. ¹H NMR (400 MHz, CDCl₃) δ 7.65 (s, 1H), 7.35 - 7.26 (m, 1H), 6.91 (t, *J =* 8.0 Hz, 2H), 6.06 (t, *J =* 10.4 Hz, 1H), 4.68 (s, 1H), 4.26 (t, *J =* 6.4 Hz, 1H), 4.07 - 3.96 (m, 1H), 3.91 - 3.68 (m, 3H), 3.62 (dd, *J =* 17.2, 9.2 Hz, 1H), 3.30 (t, *J =* 11.6 Hz, 1H), 3.19 (s, 1H), 2.83-2.72 (m, 1H), 2.55-2.44 (m, 1H), 2.24 - 2.02 (m, 4H), 1.90 (t, *J =* 7.2 Hz, 2H), 1.84 - 1.69 (m, 2H), 1.64 - 1.51 (m, 1H). HRMS(MALDI) calculated value C₂₃H₂₅F₂N₃NaO₃S [M+Na]⁺ :484.1477, found 484.1476. 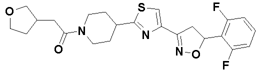 **Formula (II-24)** . light yellow oil. ¹H NMR (400 MHz, CDCl₃) δ 7.64 (s, 1H), 7.33 - 7.27 (m, 1H), 6.95 - 6.87 (m, 2H), 6.06 (dd, *J =* 12.0, 9.2 Hz, 1H), 4.66 (d, *J =* 13.2 Hz, 1H), 4.02 - 3.90 (m, 2H), 3.91 - 3.80 (m, 1H), 3.80 - 3.71 (m, 2H), 3.62 (dd, *J =* 17.2, 9.2 Hz, 1H), 3.43 (t, *J =* 6.8 Hz, 1H), 3.29 (tt, *J =* 11.2, 4.0 Hz, 1H), 3.18 (t, *J =* 12.8 Hz, 1H), 2.79 (m, 1H), 2.71 (m, 1H), 2.52 - 2.39 (m, 2H), 2.25 - 2.11 (m, 3H), 1.82 - 1.69 (m, 2H), 1.56 (m, 1H). ¹³C NMR (100 MHz, CDCl₃) δ 174.45, 170.00, 162.56 (d, *J* = 7.7 Hz), 160.06 (d, *J* = 7.5 Hz), 152.16, 145.12, 130.50 (t, *J* = 10.6 Hz), 117.64, 115.69 (t, *J =* 16.2 Hz), 111.89 (d, *J =* 5.9 Hz), 111.70 (d, *J =* 5.7 Hz), 73.27, 72.80 (t, *J =* 3.1 Hz), 67.58, 45.14, 41.49, 41.32, 40.45, 37.02, 35.49, 32.72, 32.18, 32.05. HRMS(MALDI) calculated value C₂₃H₂₅F₂N₃NaO₃S [M+Na]⁺ :484.1477, found 484.1475. 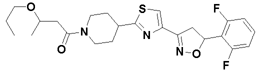 **Formula (II-25)** . light yellow oil. ¹H NMR (400 MHz, CDCl₃) δ 7.64 (s, 1H), 7.33 - 7.26 (m, 1H), 6.94 - 6.87 (m, 2H), 6.06 (dd, *J =* 11.6, 9.2 Hz, 1H), 4.69 (t, *J =* 14.8 Hz, 1H), 4.08 - 3.99 (m, 1H), 3.96 - 3.91 (m, 1H), 3.84 - 3.74 (m, 2H), 3.61 (dd, *J =* 17.2, 9.2 Hz, 1H), 3.51 - 3.40 (m, 1H), 3.34 - 3.21 (m, 1H), 3.23 - 3.11 (m, 1H), 2.84 - 2.71 (m, 1H), 2.73 - 2.61 (m, 1H), 2.39 - 2.28 (m, 1H), 2.21 - 2.10 (m, 2H), 1.85 - 1.67 (m, 4H), 1.59 - 1.45 (m, 3H), 1.37 - 1.26 (m, 1H). ¹³C NMR (100 MHz, CDCl₃) δ 174.88, 174.67, 169.46, 169.30, 162.58 (d, *J* = 7.5 Hz), 160.08 (d, *J* = 7.6 Hz), 152.21, 145.07 (d, *J =* 5.0 Hz), 130.48 (t, *J =* 10.7 Hz), 117.60, 115.72 (t, *J =* 16.1 Hz), 111.90 (d, *J* = 6.0 Hz), 111.71 (d, *J =* 5.6 Hz), 75.12 (d, *J =* 12.7 Hz), 72.81 (t, *J =* 2.9 Hz), 68.54, 45.74 (d, *J =* 7.4 Hz), 41.50, 40.53 (d, *J* = 8.0 Hz), 40.19 (d, *J* = 13.7 Hz), 32.82, 32.03 (d, *J* = 9.9 Hz), 25.78, 23.35. HRMS(MALDI) calculated value C₂₄H₂₇F₂N₃NaO₃S [M+Na]⁺ : 498.1633, found 498.1634. 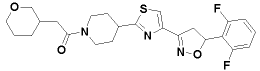 **Formula (II-26)** . light yellow oil. ¹H NMR (400 MHz, CDCl₃) δ 7.68 (s, 1H), 7.39 - 7.30 (m, 1H), 6.94 (t, *J =* 8.5 Hz, 2H), 6.14 - 6.05 (m, 1H), 4.71 (d, *J =* 13.0 Hz, 1H), 3.99 (d, *J =* 11.2 Hz, 1H), 3.94 - 3.78 (m, 3H), 3.65 (dd, *J =* 12.8, 9.6 Hz, 1H), 3.53 - 3.41 (m, 1H), 3.40 - 3.27 (m, 1H), 3.23 (t, *J =* 10.0 Hz, 2H), 2.81 (t, *J =* 12.4 Hz, 1H), 2.33 (dd, *J =* 14.8, 6.8 Hz, 1H), 2.29 - 2.13 (m, 3H), 2.01 (s, 1H), 1.98 - 1.90 (m, 1H), 1.85 - 1.70 (m, 2H), 1.69 - 1.61 (m, 2H), 1.39 - 1.28 (m, 1H). HRMS(MALDI) calculated value C₂₄H₂₇F₂N₃NaO₃S [M+Na]⁺ :498.1633, found 498.1631. 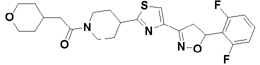 **Formula (II-27)** . light yellow oil. ¹H NMR (400 MHz, CDCl₃) δ 7.63 (s, 1H), 7.33 - 7.26 (m, 1H), 6.96 - 6.80 (m, 2H), 6.05 (dd, *J =* 12.0, 9.2 Hz, 1H), 4.67 (d, *J =* 13.4 Hz, 1H), 4.02 - 3.90 (m, 3H), 3.78 (dd, *J =* 16.8, 12.0 Hz, 1H), 3.61 (dd, *J =* 17.2, 9.2 Hz, 1H), 3.46 - 3.37 (m, 2H), 3.29 (tt, *J =* 11.6, 4.0 Hz, 1H), 3.18 (t, *J =* 12.8 Hz, 1H), 2.77 (t, *J =* 12.4 Hz, 1H), 2.27 (d, *J =* 7.2 Hz, 2H), 2.24 - 2.12 (m, 2H), 2.11 - 2.03 (m, 1H), 1.81 - 1.70 (m, 2H), 1.70 - 1.64 (m, 2H), 1.38-1.26 (m, 2H). ¹³C NMR (100 MHz, CDCl₃) δ 174.49, 169.93, 162.54 (d, *J* = 7.5 Hz), 160.04 (d, *J* = 7.6 Hz), 152.12, 145.10, 130.49 (t, *J* = 10.6 Hz), 117.65, 115.66 (t, *J* = 16.2 Hz), 111.88 (d, *J* = 5.9 Hz), 111.69 (d, *J=* 5.7 Hz), 72.81 (t, *J* = 3.1 Hz), 67.80, 45.38, 41.46, 41.31, 40.45, 39.91, 33.06, 32.82, 32.60, 32.08. HRMS(MALDI) calculated value C₂₄H₂₇F₂N₃NaO₃S [M+Na]⁺ :498.1633, found 498.1627. 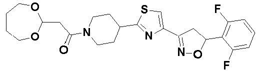 **Formula (11-28)** . light yellow oil. ¹H NMR (400 MHz, CDCl₃) δ 7.66 - 7.63 (m, 1H), 7.33 - 7.26 (m, 1H), 6.94 - 6.86 (m, 2H), 6.09 - 6.02 (m, 1H), 5.18 - 5.13 (m, 1H), 4.68 (d, *J =* 13.4 Hz, 1H), 4.01 (d, *J* = 13.6 Hz, 1H), 3.94 - 3.86 (m, 2H), 3.84 - 3.74 (m, 1H), 3.72 - 3.63 (m, 2H), 3.63 - 3.57 (m, 1H), 3.34 - 3.24 (m, 1H), 3.18 (t, *J =* 12.8 Hz, 1H), 2.77 (t, *J =* 12.8 Hz, 1H), 2.72 - 2.66 (m, 2H), 2.14 (t, *J* = 14.8 Hz, 2H), 1.80 - 1.70 (m, 6H). HRMS(MALDI) calculated value C₂₄H₂₇F₂N₃NaO₄S [M+Na]⁺ :514.1583, found 514.1581. 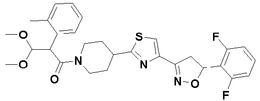 **Formula (II-31)** . white solid; m.p. =75-77 °C. ¹H NMR (400 MHz, CDCl₃) δ 7.62 (s, 1H), 7.50 - 7.41 (m, 1H), 7.34 - 7.26 (m, 1H), 7.20 - 7.12 (m, 3H), 6.91 (t, *J =* 8.4 Hz, 2H), 6.11 - 6.00 (m, 1H), 4.91 (d, *J =* 7.6 Hz, 1H), 4.76 - 4.53 (m, 1H), 4.28 - 4.17 (m, 1H), 3.91 - 3.68 (m, 2H), 3.67 - 3.56 (m, 1H), 3.54 (s, 3H), 3.27 - 3.13 (m, 1H), 3.09 (s, 3H), 2.95 - 2.83 (m, 1H), 2.82 - 2.69 (m, 1H), 2.43 (s, 3H), 2.15 - 1.95 (m, 2H), 1.57 - 1.45 (m, 1H), 0.73 - 0.58 (m, 1H). HRMS(MALDI) calculated value C₂₉H₃₁F₂N₃NaO₄S [M+Na]⁺ :578.1896, found 578.1894. 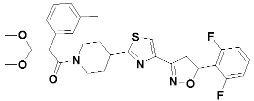 Formula (II-32) . white solid; m.p. = 89-91 °C. ¹H NMR (400 MHz, CDCl₃) δ 7.62 (s, 1H), 7.34 - 7.27 (m, 1H), 7.23 - 7.14 (m, 3H), 7.07 (t, *J =* 6.8 Hz, 1H), 6.91 (td, *J =* 8.0, 3.2 Hz, 2H), 6.05 (dd, *J* = 11.2 Hz, *J =* 20. 8 Hz, 1H), 4.98 (d, *J =* 8.0 Hz, 1H), 4.72 - 4.57 (m, 1H), 4.03 - 3.96 (m, 1H), 3.80 - 3.72 (m, 1H), 3.66 - 3.56 (m, 1H), 3.51 (s, 3H), 3.26 - 3.18 (m, 1H), 3.15 (s, 3H), 2.99 (t, *J* = 11.6 Hz, 1H), 2.81 - 2.72 (m, 1H), 2.33 (s, 3H), 2.16 - 2.06 (m, 1H), 1.93 - 1.80 (m, 1H), 1.81 - 1.73 (m, 1H), 1.58 - 1.48 (m, 1H), 0.93 - 0.81 (m, 1H). HRMS(MALDI) calculated value C₂₉H₃₁F₂N₃NaO₄S [M+Na]⁺ :578.1896, found 578.1895. 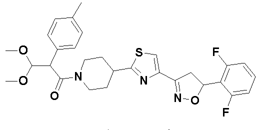 **Formula (II-33)** . light yellow solid; m.p. =90-92 °C. ¹H NMR (400 MHz, CDCl₃) δ 7.67 - 7.56 (m, 1H), 7.33 - 7.26 (m, 3H), 7.13 (d, *J =* 7.6 Hz, 2H), 6.91 (t, *J =* 8.0 Hz, 2H), 6.05 (dd, *J =* 20.4 Hz, *J* = 10.0 Hz, 1H), 4.96 (dd, *J =* 8.0, 2.0 Hz, 1H), 4.73 - 4.55 (m, 1H), 4.05 - 3.96 (m, 1H), 3.85 - 3.69 (m, 1H), 3.66 - 3.54 (m, 1H), 3.51 (d, *J =* 2.0 Hz, 3H), 3.27 - 3.17 (m, 1H), 3.15 (s, 3H), 2.97 (t, *J =* 12.4 Hz, 1H), 2.76 (t, *J =* 12.4 Hz, 1H), 2.35 - 2.27 (m, 3H), 2.16 - 2.02 (m, 2H), 1.91 - 1.78 (m, 1H), 1.59 - 1.46 (m, 1H), 0.96 - 0.81 (m, 1H). HRMS(MALDI) calculated value C₂₉H₃₁F₂N₃NaO₄S [M+Na]⁺ :578.1896, found 578.1897. 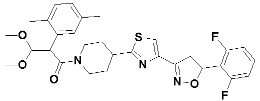 **Formula (II-34)** . white solid; m.p. =93-95 °C. ¹H NMR (400 MHz, CDCl₃) δ 7.66 - 7.56 (m, 1H), 7.34 - 7.26 (m, 2H), 7.06 (d, *J* = 7.6 Hz, 1H), 6.99 - 6.86 (m, 3H), 6.10 - 6.01 (m, 1H), 4.90 (d, *J=* 8.0 Hz, 1H), 4.76 - 4.56 (m, 1H), 4.22 - 4.09 (m, 1H), 3.90 - 3.60 (m, 3H), 3.54 (d, *J =* 6.4 Hz, 3H), 3.26 - 3.14 (m, 1H), 3.10 (d, *J =* 4.8 Hz, 3H), 2.93 - 2.68 (m, 1H), 2.38 (d, *J =* 3.2 Hz, 3H), 2.28 (d, *J = 4.0* Hz, 3H), 2.16 - 2.05 (m, 1H), 1.92 - 1.72 (m, 2H), 1.56 - 1.44 (m, 1H), 0.68 - 0.56 (m, 1H). HRMS(MALDI) calculated value C₃₀H₃₃F₂N₃NaO₄S [M+Na]⁺ :592.2052, found 592.2044. 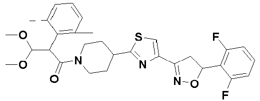 **Formula (II-35)** . white solid; m.p. =90-92 °C. ¹H NMR (400 MHz, CDCl₃) δ 7.66 - 7.56 (m, 1H), 7.37 - 7.28 (m, 2H), 7.00 - 6.95 (m, 2H), 6.94 - 6.87 (m, 2H), 6.10 - 6.00 (m, 1H), 4.92 - 4.86 (m, 1H), 4.74 - 4.54 (m, 1H), 4.23 - 4.13 (m, 1H), 3.91 - 3.68 (m, 2H), 3.67 - 3.56 (m, 1H), 3.53 (d, *J =* 2.8 Hz, 3H), 3.23 - 3.12 (m, 1H), 3.10 (s, 3H), 2.94 - 2.68 (m, 1H), 2.39 (d, *J =* 2.8 Hz, 3H), 2.30 - 2.24 (m, 3H), 2.19 - 1.96 (m, 2H), 1.91 - 1.72 (m, 1H), 1.57 - 1.44 (m, 1H), 0.75 - 0.63 (m, 1H). HRMS(MALDI) calculated value C₃₀H₃₃F₂N₃NaO₄S [M+Na]⁺ : 592.2052, found 592.2044. 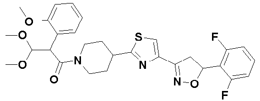 **Formula (II-37)** . white solid; m.p. =98-100 °C. ¹H NMR (400 MHz, CDCl₃) δ 7.67 - 7.56 (m, 1H), 7.53 - 7.46 (m, 1H), 7.34 - 7.27 (m, 1H), 7.25 - 7.20 (m, 1H), 6.98 - 6.87 (m, 4H), 6.10 - 6.00 (m, 1H), 5.13 - 5.04 (m, 1H), 4.72 - 4.52 (m, 2H), 4.21 (t, *J =* 15.6 Hz, 1H), 3.86 (s, 3H), 3.82 - 3.70 (m, 1H), 3.67 - 3.53 (m, 1H), 3.50 (s, 3H), 3.28 - 3.19 (m, 1H), 3.15 (s, 3H), 3.02 - 2.93 (m, 1H), 2.80 - 2.68 (m, 1H), 2.15 - 2.07 (m, 1H), 1.91 - 1.72 (m, 1H), 1.52 - 1.40 (m, 1H), 0.88 - 0.76 (m, 1H). HRMS(MALDI) calculated value C₂₉H₃₁F₂N₃NaO₅S [M+Na]⁺ : 594.1845, found 594.1843. 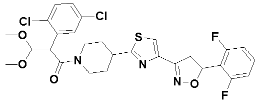 **Formula(II-39).** white solid; m.p. = 113-115 °C. ¹H NMR (400 MHz, CDCl₃) δ 7.71 - 7.62 (m, 2H), 7.33 - 7.28 (m, 2H), 7.21 - 7.16 (m, 1H), 6.95 - 6.87 (m, 2H), 6.12 - 6.02 (m, 1H), 5.00 (dd, *J =* 8.0, 2.8 Hz, 1H), 4.72 - 4.58 (m, 2H), 4.27 - 4.10 (m, 1H), 3.88 - 3.55 (m, 2H), 3.51 (d, *J =* 4.0 Hz, 3H), 3.39 - 3.24 (m, 1H), 3.21 (s, 3H), 3.16 - 3.08 (m, 1H), 2.88 - 2.75 (m, 1H), 2.20 - 2.09 (m, 2H), 1.97 - 1.72 (m, 1H), 1.64 - 1.05 (m, 1H). HRMS(MALDI) calculated value C₂₈H₂₇Cl₂F₂N₃NaO₄S[M+Na]⁺: 632.0960, found 632.0963. 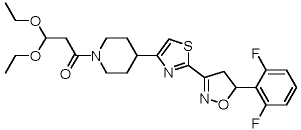 **Formula (II-70)** .light yellow oil. ¹H NMR (400 MHz, CDCl₃) δ 7.36 - 7.28 (m, 1H), 6.98 (s, 1H), 6.92 (t, *J =* 8.4 Hz, 2H), 6.12 (t, *J =* 10.8 Hz, 1H), 4.97 - 4.92 (m, 1H), 4.72 (d, *J =* 13.6 Hz, 1H), 4.06 (d, *J =* 13.6 Hz, 1H), 3.90 - 3.80 (m, 1H), 3.77 - 3.65 (m, 3H), 3.61 - 3.53 (m, 2H), 3.15 (t, *J =* 12.8 Hz, 1H), 3.02 (t, *J =* 12.4 Hz, 1H), 2.79 - 2.65 (m, 3H), 2.12 - 2.02 (m, 2H), 1.77 - 1.58 (m, 2H), 1.26 - 1.16 (m, 6H). ¹³C NMR (100 MHz, CDCl₃) δ 167.97, 162.51 (d, *J =* 7.6 Hz), 161.10, 160.00 (d, *J* = 7.6 Hz), 156.72, 153.29, 130.74 (t, *J* = 10.6 Hz), 115.30 (t, *J* = 16.2 Hz), 114.27, 111.94 (d, *J =* 5.9 Hz), 111.74 (d, *J =* 5.5 Hz), 101.67, 73.89 (t, *J =* 3.0 Hz), 63.17 (d, *J =* 17.2 Hz), 46.24, 41.76, 40.50 (d, *J =* 2.3 Hz), 38.71, 38.50, 32.14 (d, *J =* 4.1 Hz), 31.30 (d, *J =* 4.7 Hz), 15.31 (d, *J* = 2.6 Hz). HRMS(MALDI) calculated value C₂₄H₃₀F₂N₃O₄S[M+H]⁺ : 494.1920, found 494.1923. 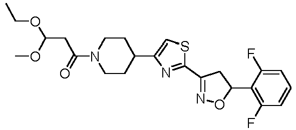 **Formula (II-71)** .light yellow oil. ¹H NMR (400 MHz, CDCl₃) δ 7.36 - 7.28 (m, 1H), 6.98 (s, 1H), 6.92 (t, *J =* 8.4 Hz, 2H), 6.12 (t, *J =* 10.8 Hz, 1H), 4.90 (s, 1H), 4.72 (d, *J =* 13.2 Hz, 1H), 4.03 (d, *J =* 13.6 Hz, 1H), 3.90 - 3.81 (m, 1H), 3.76 - 3.55 (m, 3H), 3.41 (s, 3H), 3.16 (t, *J* = 12.8 Hz, 1H), 3.01 (t, *J* = 12.0 Hz, 1H), 2.71 (s, 3H), 2.14 - 2.02 (m, 2H), 1.76 - 1.58 (m, 2H), 1.26 - 1.18 (m, 3H). ¹³C NMR (100 MHz, CDCl₃) δ 167.85, 162.52 (d, *J =* 7.5 Hz), 161.08, 160.02 (d, *J =* 7.4 Hz), 156.74, 153.30, 130.75 (t, *J =* 10.6 Hz), 115.32 (t, *J =* 16.2 Hz), 114.30, 111.95 (d, *J =* 5.8 Hz), 111.76 (d, *J* = 5.7 Hz), 102.49, 73.90 (d, *J =* 2.9 Hz), 63.28 (d, *J =* 15.2 Hz), 54.59 (d, *J =* 24.3 Hz), 46.15 (d, *J =* 2.2 Hz), 41.77 (d, *J =* 1.9 Hz), 40.53, 38.49 (d, *J =* 2.9 Hz), 3 8.22 (d, *J =* 17.6 Hz), 32.18 (d, *J =* 3.8 Hz), 31.34 - 31.19 (m), 15.30 (d, *J* = 3.1 Hz). HRMS(MALDI) calculated value C₂₃H₂₈F₂N₃O₄S [M+H]⁺ : 480.1763, found 480.1760. 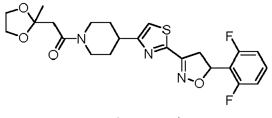 **Formula** (II-79) .light yellow oil. ¹H NMR (400 MHz, CDCl₃) δ 7.36 - 7.28 (m, 1H), 7.00 (s, 1H), 6.96 - 6.89 (m, 2H), 6.13 (dd, *J =* 12.0, 9.2 Hz, 1H), 4.77 (d, *J =* 11.6 Hz, 1H), 4.11 - 4.04 (m, 1H), 3.99 - 3.84 (m, 5H), 3.69 (dd, *J =* 17.6, 9.2 Hz, 1H), 3.23 - 3.12 (m, 1H), 3.05 (tt, *J =* 12.0, 3.6 Hz, 1H), 2.81 (s, 2H), 2.76 - 2.67 (m, 1H), 2.14 - 2.06 (m, 2H), 1.74 - 1.61 (m, 2H), 1.48 (s, 3H). ¹³C NMR (100 MHz, CDCl₃) δ 167.42, 162.51 (d, *J =* 7.6 Hz), 161.15, 160.01 (d*, J =* 7.4 Hz), 156.71, 153.29, 130.75 (t, *J =* 10.7 Hz), 115.30 (t, *J =* 16.2 Hz), 114.47 - 114.15 (m), 111.95 (d, *J =* 5.8 Hz), 111.75 (d, *J =* 5.9 Hz), 108.70, 73.91 (t, *J =* 3.1 Hz), 64.69 (d, *J =* 4.7 Hz), 46.87 - 46.49 (m), 43.00, 41.97 - 41.70 (m), 40.52 (d, *J* = 2.5 Hz), 38.52 - 38.26 (m), 32.30 - 31.91 (m), 31.40 - 31.02 (m), 30.17, 24.64. HRMS(MALDI) calculated value C₂₃H₂₆F₂N₃O₄S [M+H]⁺ : 478.1607, found 478.1611. 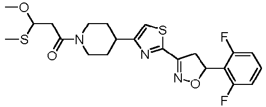 **Formula (II-81)** .light yellow oil. ¹H NMR (400 MHz, CDCl₃) δ 7.36 - 7.28 (m, 1H), 6.99 (s, 1H), 6.96 - 6.89 (m, 2H), 6.13 (dd, *J =* 12.0, 9.2 Hz, 1H), 4.87 (d, *J =* 7.2 Hz, 1H), 4.73 (d, *J =* 13.6 Hz, 1H), 3.98 (d, *J =* 13.2 Hz, 1H), 3.87 (dd, *J =* 17.2, 12.0 Hz, 1H), 3.68 (dd, *J =* 17.2, 9.2 Hz, 1H), 3.42 (s, 3H), 3.18 (t, *J =* 12.8 Hz, 1H), 3.09 - 2.97 (m, 2H), 2.80 - 2.70 (m, 2H), 2.17 - 2.05 (m, 5H), 1.74 - 1.61 (m, 2H). HRMS(MALDI) calculated value C₂₂H₂₆F₂N₃O₃S₂ [M+H]⁺ : 482.1378, found 482.1380. 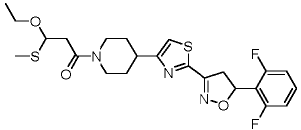 **Formula (II-82)** .light yellow oil. ¹H NMR (400 MHz, CDCl₃) δ 7.36 - 7.28 (m, 1H), 7.00 (s, 1H), 6.96 - 6.89 (m, 2H), 6.13 (dd, *J =* 12.0, 9.2 Hz, 1H), 4.95 - 4.90 (m, 1H), 4.73 (d, *J =* 13.2 Hz, 1H), 3.98 (d, *J =* 13.2 Hz, 1H), 3.89 (dd, *J =* 17.2, 12.0 Hz, 1H), 3.69 (dd, *J =* 17.2, 9.2 Hz, 1H), 3.42 (s, 3H), 3.18 (t, *J =* 12.8 Hz, 1H), 3.10 - 2.99 (m, 2H), 2.82 - 2.72 (m, 2H), 2.68 - 2.61 (m, 2H), 2.16 - 2.05 (m, 2H), 1.74 - 1.61 (m, 2H), 1.28 (t, *J* = 7.6 Hz, 3H). HRMS(MALDI) calculated value C₂₃H₂₈F₂N₃O₃S₂ [M+H]⁺ : 496.1535, found 496.1530. 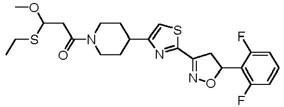 **Formula (II-85)** .light yellow oil. ¹H NMR (400 MHz, CDCl₃) δ 7.36 - 7.27 (m, 1H), 6.99 (s, 1H), 6.96 - 6.89 (m, 2H), 6.13 (dd, *J =* 12.0, 9.2 Hz, 1H), 4.97 - 4.92 (m, 1H), 4.78 - 4.68 (m, 1H), 4.05 - 3.96 (m, 1H), 3.91 - 3.78 (m, 2H), 3.68 (dd, *J =* 17.2, 9.2 Hz, 1H), 3.51 - 3.45 (m, 1H), 3.18 (t, *J =* 12.8 Hz, 1H), 3.09 - 2.99 (m, 2H), 2.81 - 2.69 (m, 2H), 2.13 - 2.06 (m, 5H), 1.75 - 1.62 (m, 2H), 1.20 (t, *J* = 7.2 Hz, 3H). HRMS(MALDI) calculated value C₂₃H₂₈F₂N₃O₃S₂ [M+H]⁺ : 496.1535, found 496.1536. 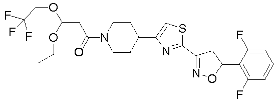 **Formula (II-87)** .light yellow oil. ¹H NMR (400 MHz, CDCl₃) δ 7.36 - 7.27 (m, 1H), 6.99 (s, 1H), 6.96 - 6.89 (m, 2H), 6.12 (dd, *J =* 12.0, 9.2 Hz, 1H), 5.14 - 5.09 (m, 1H), 4.71 (d, *J =* 13.4 Hz, 1H), 4.03 - 3.82 (m, 4H), 3.78 - 3.57 (m, 3H), 3.18 (t, *J =* 12.8 Hz, 1H), 3.09 - 3.00 (m, 1H), 2.78 - 2.68 (m, 3H), 2.14 - 2.05 (m, 2H), 1.74 - 1.62 (m, 2H), 1.24 - 1.19 (m, 3H). HRMS(MALDI) calculated value C₂₄H₂₇F₅N₃O₄S [M+H]⁺ : 548.1637, found 548.1642. 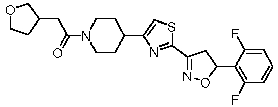 **Formula (11-92)** .light yellow oil. ¹H NMR (400 MHz, CDCl₃) δ 7.36 - 7.28 (m, 1H), 7.00 (s, 1H), 6.96 - 6.89 (m, 2H), 6.13 (dd, *J =* 12.4, 9.2 Hz, 1H), 4.72 (d, *J =* 13.4 Hz, 1H), 4.01 - 3.92 (m, 2H), 3.91 - 3.83 (m, 2H), 3.79 - 3.74 (m, 1H), 3.73 - 3.64 (m, 1H), 3.48 - 3.42 (m, 1H), 3.22 - 3.12 (m, 1H), 3.08 - 2.98 (m, 1H), 2.76 - 2.67 (m, 2H), 2.53 - 2.40 (m, 2H), 2.21 - 2.06 (m, 3H), 1.71 - 1.61 (m, 2H), 1.60 - 1.52 (m, 1H).¹³C NMR (100 MHz, CDCl₃) δ 169.98, 162.53 (d, *J =* 7.3 Hz), 160.97, 160.03 (d, *J* = 7.6 Hz), 156.80, 153.28, 130.76 (t, *J* = 10.7 Hz), 115.24 (d, *J* = 16.4 Hz), 114.38, 111.96 (d, *J* = 5.7 Hz), 111.77 (d, *J =* 5.5 Hz), 73.93, 73.31, 67.61, 45.56, 41.72, 40.56, 38.52, 37.05, 35.56, 32.28, 32.21, 31.25. HRMS(MALDI) calculated value C₂₉H₃₁F₂N₃NaO₅S [M+H]⁺ : 594.1845, found 594.1843. 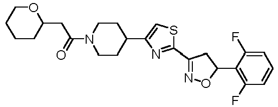 **Formula (II-93)** .light yellow oil. ¹H NMR (400 MHz, CDCl₃) δ 7.37 - 7.29 (m, 1H), 6.99 (s, 1H), 6.97 - 6.89 (m, 2H), 6.17 - 6.08 (m, 1H), 4.78 - 4.69 (m, 1H), 4.04 (d, *J =* 13.6 Hz, 1H), 3.98 - 3.89 (m, 1H), 3.88 - 3.76 (m, 2H), 3.68 (dd, *J =* 17.4, 9.2 Hz, 1H), 3.50 - 3.42 (m, 1H), 3.21 - 3.09 (m, 1H), 3.08 - 2.98 (m, 1H), 2.79 - 2.63 (m, 2H), 2.39 - 2.31 (m, 1H), 2.14 - 2.03 (m, 2H), 1.86 - 1.60 (m, 4H), 1.58 - 1.46 (m, 3H), 1.37 - 1.27 (m, 1H).¹³C NMR (100 MHz, CDCl₃) δ 169.22 (d, *J =* 8.4 Hz), 162.51 (d, *J =* 7.5 Hz), 161.16 (d, *J =* 8.8 Hz), 160.01 (d, *J =* 7.5 Hz), 156.69 (d, *J =* 3.0 Hz), 153.29, 130.73 (t, *J =* 10.6 Hz), 115.31 (t, *J =* 16.2 Hz), 114.29 (d, *J =* 5.0 Hz), 111.94 (d, *J =* 5.8 Hz), 111.74 (d, *J =* 5.9 Hz), 75.06 (d, *J* = 5.9 Hz), 73.90 (t, *J =* 3.0 Hz), 68.52 (d, *J =* 1.8 Hz), 46.04 (d, *J* = 2.3 Hz), 41.78 (d, *J* = 2.0 Hz), 40.53, 40.16 (d, *J* = 13.0 Hz), 38.52, 32.37 - 32.20 (m), 32.00 (d, *J* = 8.4 Hz), 31.24 (t, *J* = 5.2 Hz), 25.79, 23.33 (d, *J* = 2.2 Hz). HRMS(MALDI) calculated value C₂₄H₂₇F₂N₃NaO₃S[M+Na]⁺ : 498.1633, found 498.1637. 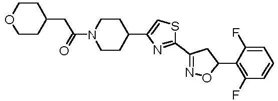 **Formula (II-95)** .light yellow oil. ¹H NMR (400 MHz, CDCl₃) δ 7.36 - 7.27 (m, 1H), 7.00 (s, 1H), 6.96 - 6.89 (m, 2H), 6.12 (dd, *J =* 12.0, 9.2 Hz, 1H), 4.73 (d, *J =* 12.8 Hz, 1H), 4.01 - 3.89 (m, 3H), 3.88 - 3.82 (m, 1H), 3.68 (dd, *J =* 17.4, 9.2 Hz, 1H), 3.42 (td, *J =* 11.8, 2.0 Hz, 2H), 3.22 - 3.11 (m, 1H), 3.04 (tt, *J =* 11.6, 3.6 Hz, 1H), 2.71 (t, *J =* 10.6 Hz, 1H), 2.28 (d, *J =* 6.8 Hz, 2H), 2.17 - 2.05 (m, 3H), 1.72 - 1.61 (m, 4H), 1.39 - 1.27 (m, 2H). HRMS(MALDI) calculated value C₂₄H₂₈F₂N₃O₃S [M+H]⁺ : 476.1814, found 476.1818. 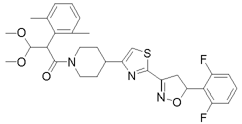 **Formula(II-103).**white solid; m.p. = 95-97 °C. ¹H NMR (400 MHz, CDCl₃) δ 7.38 - 7.27 (m, 2H), 7.00 - 6.89 (m, 5H), 6.16 - 6.06 (m, 1H), 4.89 (dd, *J =* 7.6, 3.2 Hz, 1H), 4.77 - 4.63 (m, 1H), 4.19 (dd, *J =* 17.2, 8.0 Hz, 1H), 3.89 - 3.57 (m, 3H), 3.53 (d, *J =* 3.2 Hz, 3H), 3.09 (d, *J =* 2.0 Hz, 3H), 3.00 - 2.80 (m, 1H), 2.68 (t, *J =* 13.2 Hz, 1H), 2.39 (d, *J =* 2.6 Hz, 3H), 2.29 - 2.20 (m, 3H), 1.98 (d, *J= 13.6* Hz, 1H), 1.81 - 1.65 (m, 2H), 1.46 - 1.35 (m, 1H), 0.70 - 0.59 (m, 1H). HRMS(MALDI) calculated value C₃₀H₃₄F₂N₃O₄S[M+H]⁺ : 570.2233, found 570.2238. 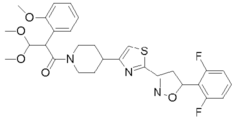 **Formula(II-105).** white solid; m.p. = 97-99 °C. ¹H NMR (400 MHz, CDCl₃) δ 7.54 - 7.47 (m, 1H), 7.36 - 7.28 (m, 1H), 7.24 - 7.17 (m, 1H), 6.99 - 6.86 (m, 4H), 6.74 (s, 1H), 6.16 - 6.07 (m, 1H), 5.12 - 5.06 (m, 1H), 4.74 - 4.59 (m, 2H), 4.22 (t, *J =* 15.6 Hz, 1H), 3.86 (s, 3H), 3.82 - 3.76 (m, 1H), 3.70 - 3.59 (m, 1H), 3.49 (d, *J =* 1.8 Hz, 3H), 3.15 (s, 3H), 3.13 - 3.07 (m, 1H), 3.02 - 2.86 (m, 1H), 2.71 - 2.63 (m, 1H), 2.02 - 1.93 (m, 1H), 1.84 - 1.75 (m, 1H), 1.42 - 1.30 (m, 1H), 0.79 - 0.65 (m, 1H). HRMS(MALDI) calculated value C₂₉H₃₂F₂N₃O₅S [M+H]⁺ : 572.2025, found 572.2029.

### Test example 1: activity in vivo for inhibiting cucumber downy mildew

The test and investigation method refers to a SOP-SC-1098 cucumber downy mildew potting method in the fungicide section of *Standard operation Specification for testing biological Activity of pesticides* written by Kang Zhuo and Gu Baogen. The test results are shown in tables 1, 2 and 3, and the "/" item in the tables indicates no test (the same applies hereinafter). Wherein, the structural formula of the control agent oxathiapiprolin(OXA) is as follows:

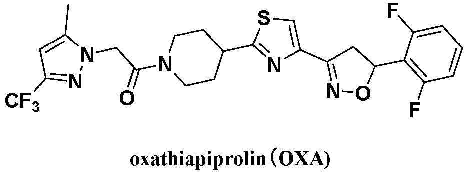

Description of the respective control effect grades in the activity test results: 90%≤A≤100%; 75%≤B<90%; 60%≤C<75%; 45%≤D<60%; 0%≤E<45%.

Screening results of activity of the target compounds against cucumber downy mildew *in vivo* (in the greenhouse).

**Table 1: activity of target compounds against cucumber downy mildew in vivo**

| Number | Application concentration (mg/L)/Control effect | | |
|---|---|---|---|
| | 20 | 5 | 1.25 |
| I -1 | A | A | A |
| I -2 | A | B | B |
| I -3 | A | A | A |
| I -4 | A | A | B |
| I -5 | B | B | C |
| I -6 | B | / | / |
| I -7 | A | B | / |
| I -8 | A | A | A |
| I -9 | A | A | A |
| I -10 | A | A | A |
| I -11 | C | / | / |
| I -12 | B | D | / |
| I -13 | A | B | / |
| I -14 | A | A | A |
| I -15 | C | C | / |
| I -16 | B | C | D |
| I -17 | A | A | A |
| I -18 | D | / | / |
| I -19 | A | A | A |
| I -20 | D | / | / |
| I -21 | D | / | / |
| I -22 | A | A | A |
| I -23 | B | B | D |
| I -24 | A | B | C |
| I -25 | A | B | C |
| I -26 | C | D | D |
| I -28 | A | B | / |
| I -29 | A | A | A |
| I -30 | A | B | B |
| II -1 | A | A | A |
| II -2 | A | A | A |
| II -3 | A | A | A |
| II -4 | A | A | A |
| II -5 | A | D | / |
| II -6 | C | D | / |
| II -7 | B | D | / |
| II -8 | A | C | D |
| II -9 | A | B | D |
| II -10 | D | / | / |
| II -11 | A | C | D |
| II -12 | A | D | / |
| II -18 | A | A | A |
| II -19 | A | A | A |
| II -21 | A | B | D |
| II -22 | A | D | / |
| II -28 | C | C | / |

In order to further explore the relationship between the structure and the activity of the compounds, this study the invention conducted a fungicidal activity re-screening experiment at lower concentrations on some of the compounds that showed a control efficacy of over 75% at the lowest concentration (1.25 mg/L) in the preliminary fungicidal activity test of cucumber downy mildew *in vivo.* The results of the experiment are shown in table 2.

**Table 2: the re-screening of fungicidal activity of target compounds against cucumber downy mildew in vivo**

| Number | Application concentration (mg/L)/Control effect | | | | |
|---|---|---|---|---|---|
| | 1.25 | 0.312 | 0.078 | 0.02 | 0.005 |
| I-19 | A | A | B | / | / |
| I-22 | A | B | B | / | / |
| II-2 | A | A | B | / | / |
| II-3 | A | A | A | A | A |
| II-4 | A | A | A | / | / |
| II-18 | A | A | A | A | A |
| II-19 | A | A | A | A | B |
| OXA | A | A | A | A | A |
| dimethomorph | A | A | B | C | / |
| cyazofamid | A | A | B | E | / |

As can be seen from the above activity results, many of the compounds of the present invention have excellent control effects on cucumber downy mildew, at three application concentrations ranging from 20 mg/L to 1.25 mg/L, the control effect of a plurality of compounds is 75% to 100 %, which is equivalent to that of a control agent oxathiapiprolin, in the active re-screening stage, 4 compounds (II-3, II-4, II-18 and II-19) of the present invention exhibit excellent bactericidal activity, with a control effect of 90% or more at an application concentration of 0.078 mg/L. Their efficacy is equivalent to that of oxathiapiprolin at the same concentration and superior to that of dimethomorph and cyazofamid. Most importantly, the efficacy of the compounds II-3 and II-18 of the present invention at a concentration of 0.005 mg/L is comparable to that of the control agent OXA, and these two compounds have further development value.

Comparison with the closest existing compounds: the activity data sources of the comparative compounds (Comparative 1 to Comparative 7) in Table 3 are from Li, J. L. et al. Pestic. Biochem. Phys. 2020, 169, 104673. DOI: doi.org/10.1016/j.pestbp.2020.104673. As can be seen from the comparison in Table 3, most of the compounds of the present invention showed higher control efficacy against cucumber downy mildew than the comparative compounds at a concentration of 0.625 mg/L.

**Table 3: comparison of in vivo fungicidal activity results of compounds against cucumber downy mildew**

| Number | Structural formula | Application concentration (mg/L)/Control effect | | |
|---|---|---|---|---|
| | | 10 | 2.5 | 0.625 |
| comparative 1 | 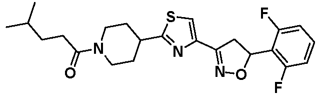 | B | B | E |
| comparative 2 | 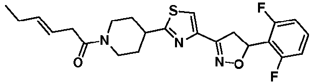 | A | B | D |
| comparative 3 | 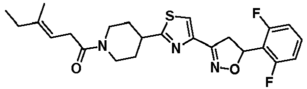 | A | c | E |
| comparative 4 | 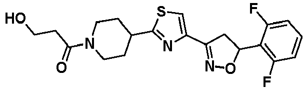 | D | E | E |
| comparative 5 | 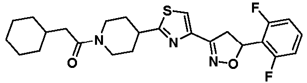 | A | D | E |
| comparative 6 | 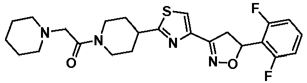 | E | E | E |
| comparative 7 | 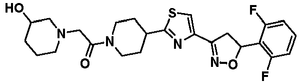 | E | E | E |
| OXA | | A | A | A |
| II-3 | | A | A | A |
| II-18 | | A | A | A |
| II-19 | | A | A | A |

| | | | | |
|---|---|---|---|---|
| Note: the control effect grade is as follows: 90%≤A≤100%; 75%≤B<90%; 60%≤C<75%; 45%≤D<60%; 0%≤E<45% | | | | |

### Test example 2: in vitro activity for inhibiting hypha growth of oomycete pathogens

The micropore plate method was adopted to measure the biological activity in a medicament chamber. Culture of *Phytophthora capsici, Phytophthora sojae, Phytophthora infestans* and *Phytophthora nicotianae*: inoculate the target pathogen in PDB or V8 juice liquid medium, and shake-culture for 72h to 96h. After filtration, collect the fresh mycelium, Then weigh 0.1g mycelium and add it to 50 ml of PDB liquid medium. Mash the mycelium into small mycelium segments using a tissue homogenizer to prepare a mycelial segment suspension, and store it at 4°C for later use.

Culture of *Phytophthora infestans*: inoculate *Phytophthora infestans* in V8 culture medium and culture for 12 days. After a large number of sporangia are produced, add sterile water and transfer the plate to a refrigerator at 4°C for 1h, then transfer it to room temperature to induce zoospore release. After the zoospores are fully released, a spore suspension with a concentration of 10⁵ spores /mL was prepared and placed at 4°C for later use.

Preparing the test reagent into a 2000 mg/L mother liquor using DMSO, then diluting it with sterile water to form a series of gradient concentration solutions, and storing them at 4 °C for use.

The spore suspension (mycelium segment suspension) and the series of gradient solutions prepared in advance were sequentially added into each well at a volume of 100µL+100µL. then the 96-well microplate was placed in an incubator at 25 °C (18 °C for *Phytophthora infestans*) for static culture, after 3-4 days (7 days for *Phytophthora infestans* )*,* a microplate reader was used to measure the OD₅₉₅ value of each treatment. Then the *in vitro* toxicity of the agents against the target pathogens were calculated. The results are shown in Table 4. Inhibition rate%= (blank treatment OD595- dose treatment OD595) / (blank treatment OD595-reference OD595) *100

**Table 4: inhibitory activity of compounds II-3 and II-18 on hypha growth of oomycete pathogens**

| Species of pathogens | Compound number | Application concentration (mg/L)/ inhibitory activity | | | |
|---|---|---|---|---|---|
| | | 5 | 0.5 | 0.05 | 0.005 |
| *Phytophthora capsici* | OXA | A | A | A | A |
| | II -3 | A | A | A | A |
| | II-18 | A | A | A | A |
| *Phytophthora sojae* | OXA | A | A | A | A |
| | II -3 | A | A | A | B |
| | II-18 | A | A | A | A |
| *Phytophthora infestans* | OXA | A | A | A | A |
| | II -3 | A | A | A | B |
| | II -18 | A | A | A | A |
| *Phytophthora nicotianae* | OXA | A | A | A | A |
| | II -3 | A | A | A | A |
| | II -18 | A | A | A | B |

| | | | | | |
|---|---|---|---|---|---|
| Note: grade of inhibitory activity: 90%≤A≤100%; 75%≤B<90%; 60%≤C<75%; | | | | | |

As can be seen from the results shown in Table 4, the compounds II-3 and II-18 showed excellent inhibitory activities against the growth of hyphae of oomycete pathogens, and the inhibitory activities against Phytophthora capsici, Phytophthora sojae, Phytophthora infestans and Pythium nicotianae were all comparable to that of the control agent OXA at the application concentrations of 5, 0.5 and 0.05 mg/L.

### Test example 3: field efficacy test for controlling cucumber downy mildew

The test was carried out according to the *Guidelines for the Field Efficacy Trials of Pesticides (Part 1): Fungicides Against Downy Mildew of Cucumber* (GB/T17980.26-2000). The test site was a mature vegetable base of Hushu in Nanjing, Jiangsu Province. Cucumber downy mildew (*Pseudoperonospora cubensis*) was used as the target, oxathiapiprolin and Yin Fali ( 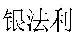 ) were used as control agents. The test crops was cucumber and the variety was 'Green Core'. The plots were arranged in a randomized block design, with each plot approximately 8 m² and each treatment replicated three times. The whole stem and leaf spray was employed, the test was carried out at the early stage of cucumber downy mildew occurrence, and the agents were applied twice consecutively, with the second application 5 days after the first one. The investigation method was to take one investigation point for every row of cucumbers in each treatment district, assess the severity of downy mildew of all leaves of all cucumber plants, and calculate the disease index and the prevention effect. The results are shown in table 5.

**Table 5: field control effect of compound II-3 against cucumber downy mildew**

| Agent treatment | Dosage (mg/L) | Control effect after the first pesticide application | | Control effect after the second pesticide application | |
|---|---|---|---|---|---|
| | | Average disease index | control effect (%) | Average disease index | control effect (%) |
| OXA | 50 | 5.36 | 36.02 | 8.59 | 74.87 |
| II -3 | 50 | 2.80 | 66.63 | 6.93 | 79.72 |
| Yin Fali | 50 | 5.28 | 36.95 | 19.64 | 42.53 |
| | 100 | 5.14 | 38.64 | 11.13 | 67.43 |
| Blank control | - | 8.38 | | 34.18 | |

Test results show that the compound II-3 has good effect in controlling cucumber downy mildew, it can effectively protect new leaves and can effectively inhibit the spread of disease spots on the infected leaves. The dosage of the composition for controlling the cucumber downy mildew in the field is 50 mg/L. Applying it 2 - 3 times continuously at intervals of 5-7 days can effectively control the occurrence of the downy mildew. At the test dose of 50 mg/L, its control effect is better than that of the control agents OXA and Yinfali.

### Test example 4: field efficacy test for controlling potato late blight

The test was carried out according to the "Guidelines for the Field Efficacy Trials of Pesticides (Part 1): Fungicides Against Potato Late Blight" (GB/T17980.34-2000). The test site was Qishan, Shanxi Province, the potato late blight was taken as the target, and the commercially available DuPont^{™} Zorvec Enicade^{®} (10% oxathiapiprolin dispersible oil suspension agent) was used as a control agent. The test crop was potato.The plots were arranged in a randomized block design, with each treatment replicated three times. The whole stem and leaf spray was adopted, the test was carried out at the early stage of potato late blight occurrence. The pesticide was applied twice continuously, with the second application carried out 7 days after the first one. The investigation method was to take five samples along the diagonal of each plot. Two plants were selected at each point, and about 20 upper leaves were investigated in each plant. The total number of leaves, the number of diseased leaves, and the disease grades were recorded, and the disease index and control efficacy were calculated. The results are shown in Table 6.

**Table 6: field control effect of compound II-3 against potato late blight**

| Agent treatment | Dosage of the active ingredients (g.a.i./ha) | Dosage of preparation per mu (ml/mu) | Initial inoculum | Control effect 7 days after the first pesticide application | | Control effect 7 days after the second pesticide application | |
|---|---|---|---|---|---|---|---|
| | | | | Average disease index | Control effect (%) | Average disease index | Control effect (%) |
| 5wt% of the II-3 emulsifiable concentrate | 22.5 | 30 | 0.02 | 0.07 | 76.81 | 0.68 | 75.87 |
| | 30.0 | 40 | 0.03 | 0.05 | 87.72 | 0.62 | 84.34 |
| DuPont^{™} Zorvec Enicade^{®} | 22.5 | 15 | 0.03 | 0.10 | 75.34 | 1.12 | 71.29 |
| | 30.0 | 20 | 0.03 | 0.07 | 84.36 | 0.82 | 80.47 |
| Blank control | | | 0.04 | 0.43 | | 4.13 | |

The result shows that the 5wt% of the II-3 emulsifiable concentrate has a good effect in controlling potato late blight, it can effectively protect new leaves and can effectively inhibit the spread of the disease spots on the infected leaves. When the dosage is 30-40 mL/mu and applied 2 - 3 times continuously at intervals of 5 - 7 days, it can effectively control the occurrence of potato late blight. The control effect is better than that of the commercially available DuPont^{™} Zorvec Enicade^{®} at a dosage of 15-20 mL/mu. That is, under the same dosage of the active ingredients, the control effect of the 5wt% of the II-3 emulsifiable concentrate is better than that of the DuPont^{™} Zorvec Enicade^{®}.

The preferred embodiments of the present invention have been described in detail above, but the present invention is not limited thereto. Within the scope of the technical idea of the invention, many simple modifications can be made to its technical solution, including combining various technical features in any other suitable way, and these simple modifications and combinations should also be regarded as the disclosure of the invention, and all fall within the protection scope of the invention.

## Claims

1. A compound containing a chain-shaped carboxylic acid amide structure, which has a structure represented by formula (I), or an agrochemically acceptable salt, hydrate and solvate thereof, 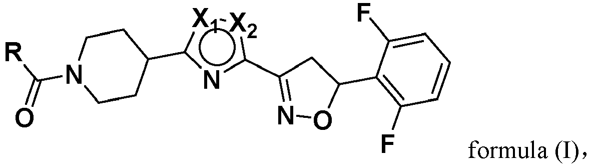 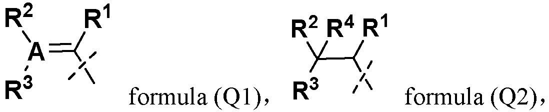 wherein, in formula (I), one of X₁ and X₂ is S, and the other is CH; R is a structure represented by formula (Q1) or a structure represented by formula (Q2);
in formula (Q1), R¹, R², R³ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; or, R² and R³ are ring-combined together to form a 3-7 membered saturated heterocyclic group containing at least one O atom, which is unsubstituted or substituted with at least one group from the combination A; A is N or CH; and when A is N, one of R² and R³ is absent;
in formula (Q2), R¹, R², R³, R⁴ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; and at least one of R¹, R², R³, R⁴ is alkoxy of C₁-C₁₂ or alkylthio of C₁-C₁₂; or, R² and R³ are ring-combined together to form a 3-7 membered saturated heterocyclic group containing at least one O atom as a ring-forming atom, which is unsubstituted or substituted with at least one group from the combination A;
the combination A consists of alkyl of C₁-C₁₂, halogen, alkyl of C₁-C₁₂ substituted with at least one halogen, alkoxy of C₁-C₁₂.

2. The compound according to claim 1, wherein, in formula (I),
one of X₁ and X₂ is S, and the other is CH;
R is a structure represented by formula (Q1); in formula (Q1), R¹, R², R³ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; or, R² and R³ are ring-combined together to form a 3-7 membered saturated heterocyclic group containing at least one O atom as a ring-forming atom, which is unsubstituted or substituted with at least one group from the combination A;
A is N or CH; and when A is N, one of R² and R³ is absent;
the combination A consists of alkyl of C₁-C₁₂, halogen, alkyl of C₁-C₁₂ substituted with at least one halogen, alkoxy of C₁-C₁₂;
preferably, in formula (I),
one of X₁ and X₂ is S, and the other is CH;
R is a structure represented by formula (Q1); in formula (Q1), R¹, R², R³ are each independently selected from the group consisting of H, alkyl of C₁-C₁₀, alkoxy of C₁-C₁₀, cycloalkyl of C₃-C₁₀, alkylthio of C₁-C₁₀, alkoxy of C₁-C₁₀ substituted with at least one halogen, alkoxy of C₁-C₁₀ substituted with phenyl, halogen, phenyl, phenyl substituted with at least one group of combination A, cyano, nitro, pyridyl, pyrazolyl, pyrazolyl substituted with at least one group of combination A; or, R² and R³ are ring-combined together to form a 3-7 membered saturated heterocyclic group containing at least one O atom as a ring-forming atom, which is unsubstituted or substituted with at least one group from the combination A;
A is N or CH; and when A is N, one of R² and R³ is absent;
the combination A consists of alkyl of C₁-C₁₀, halogen, alkyl of C₁-C₁₀ substituted with at least one halogen, alkoxy of C₁-C₁₀.

3. The compound according to claim 2, wherein the compound of the structure shown in formula (I) is selected from any one of the following:

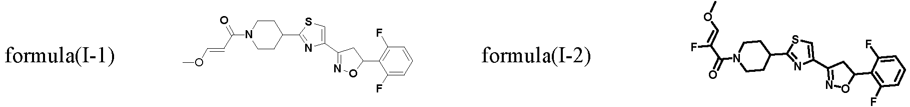

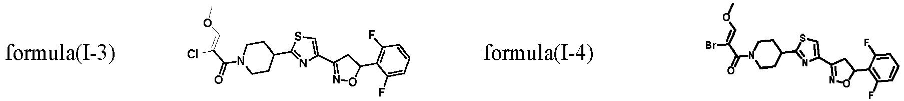

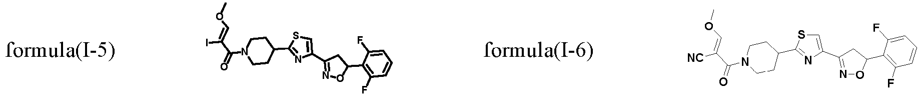

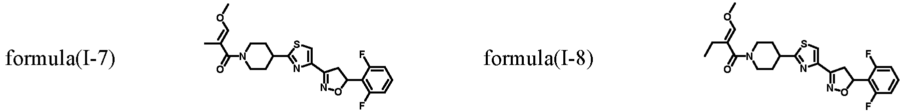

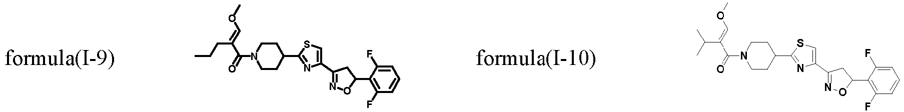

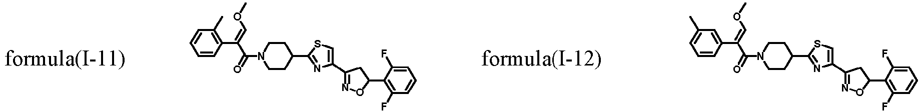

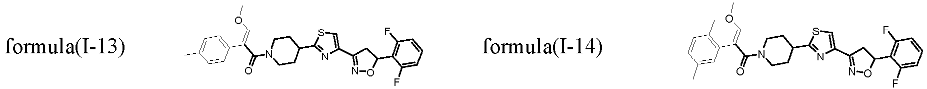

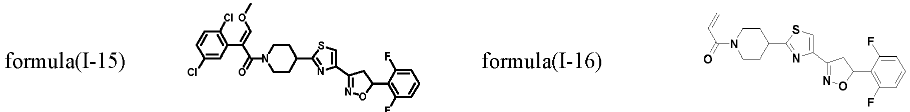

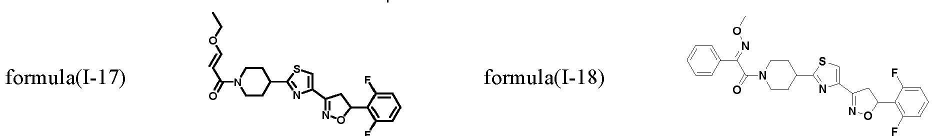

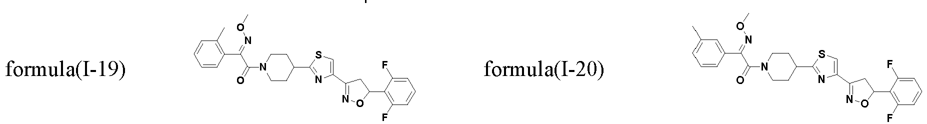

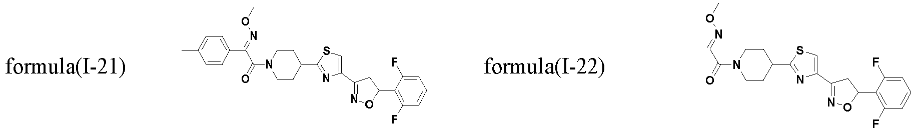

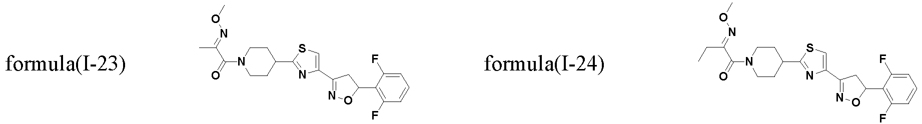

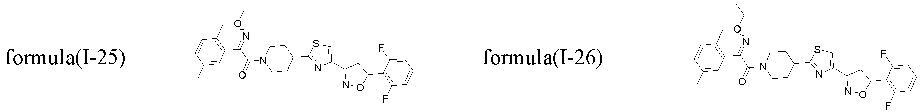

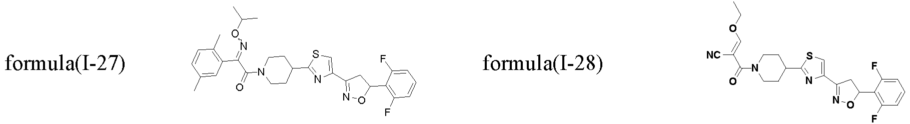

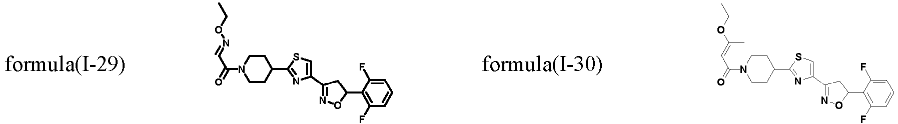

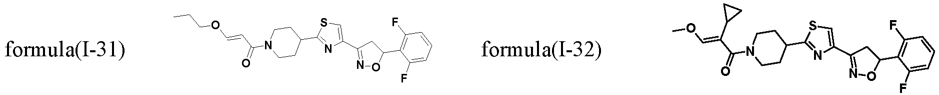

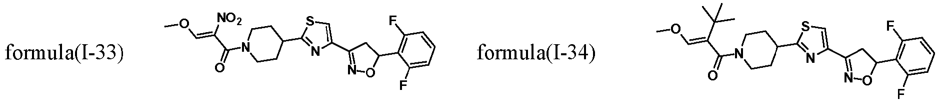

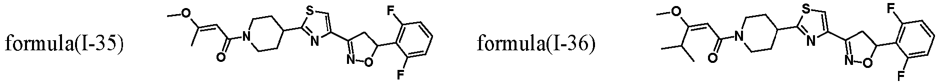

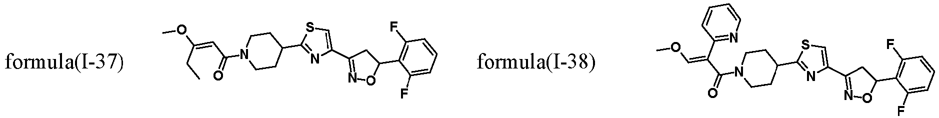

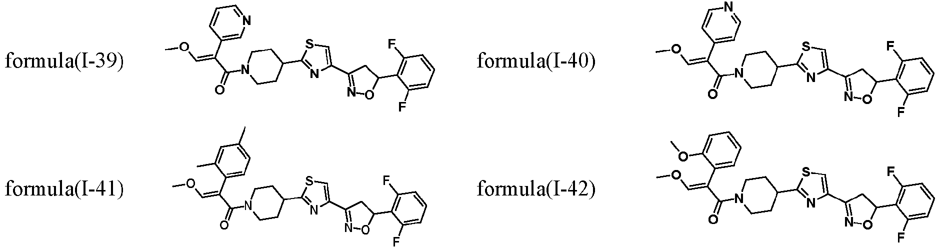

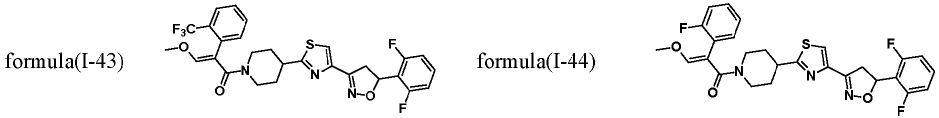

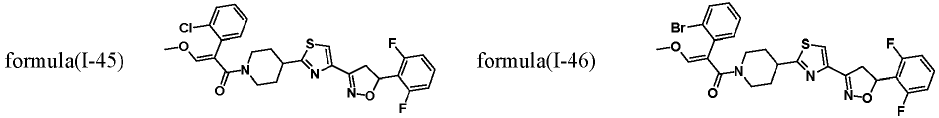

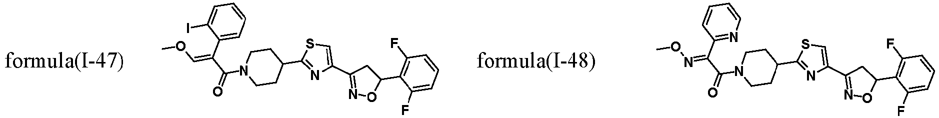

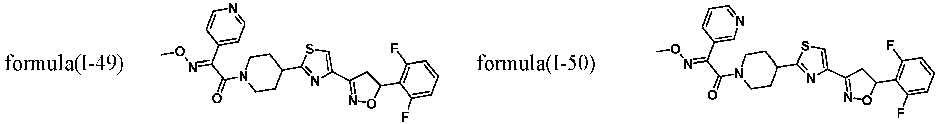

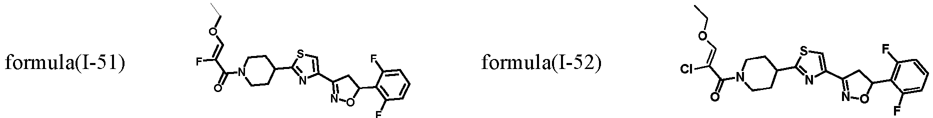

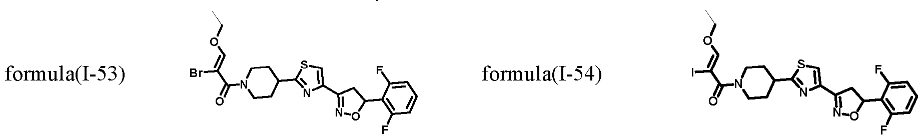

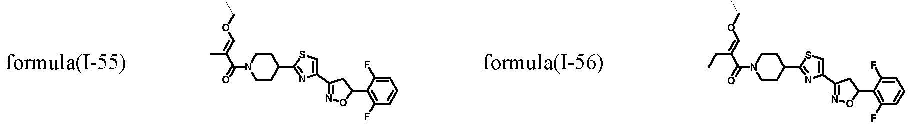

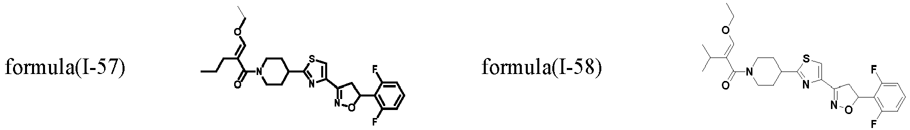

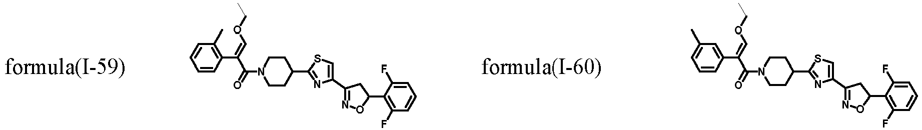

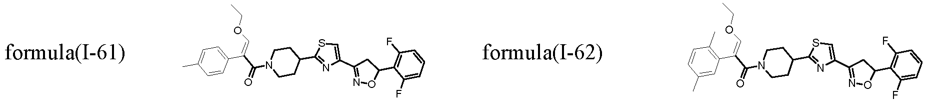

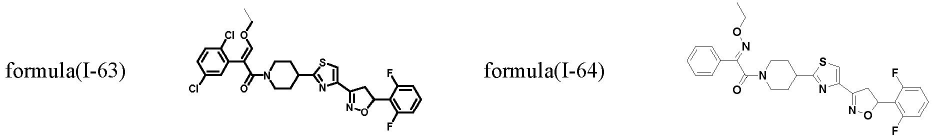

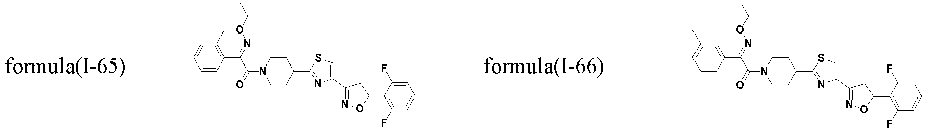

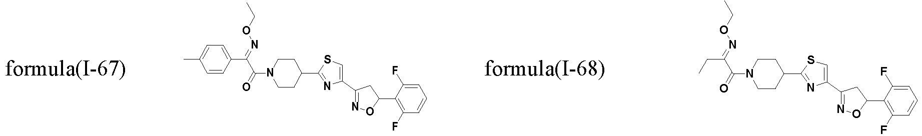

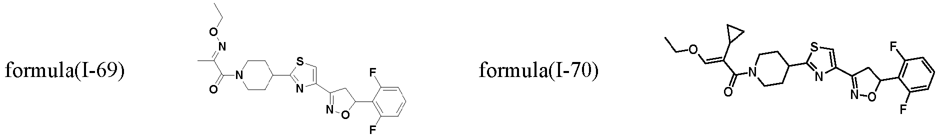

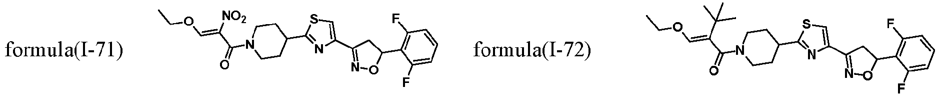

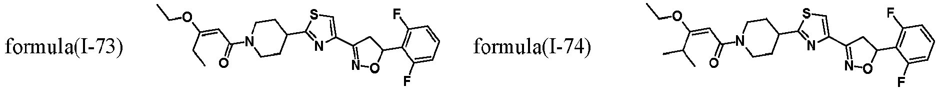

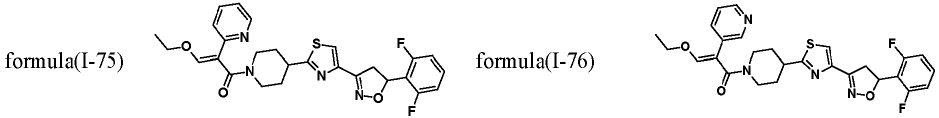

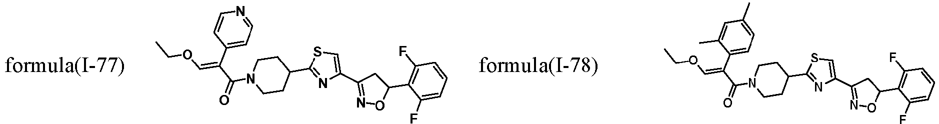

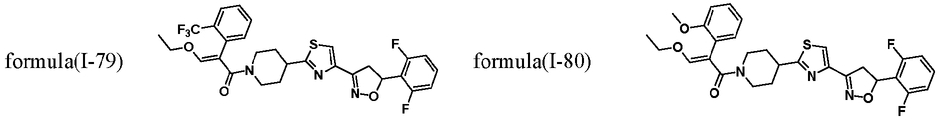

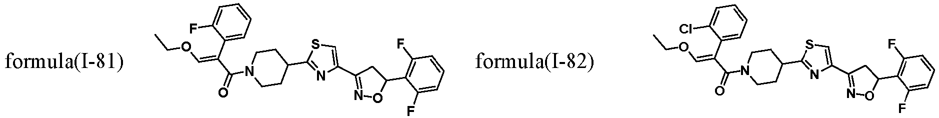

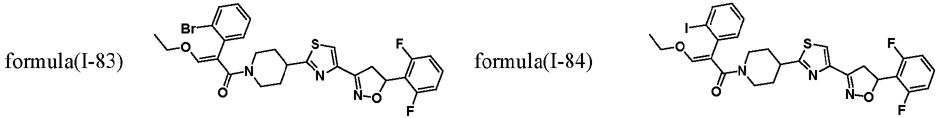

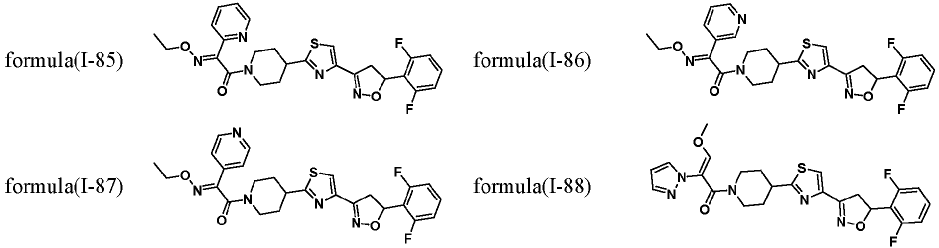

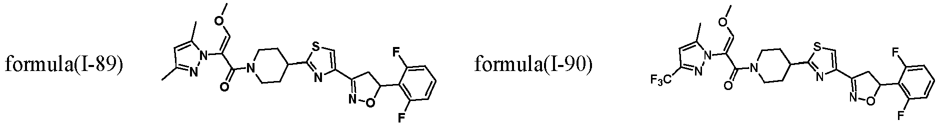

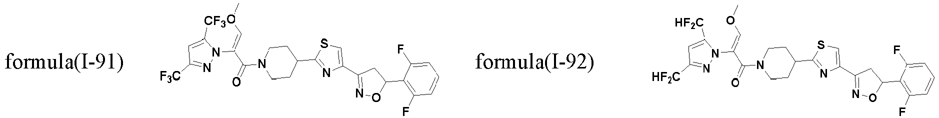

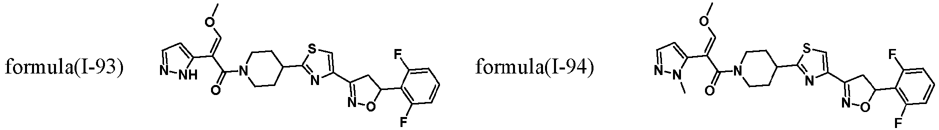

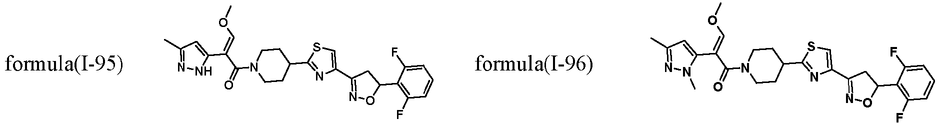

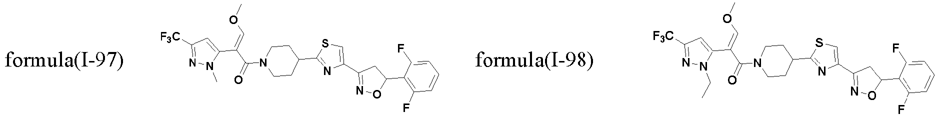

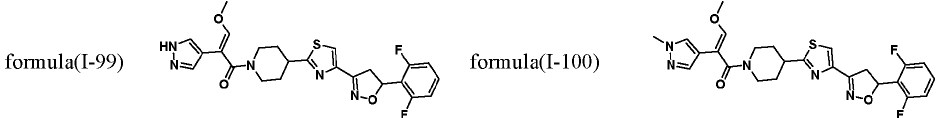

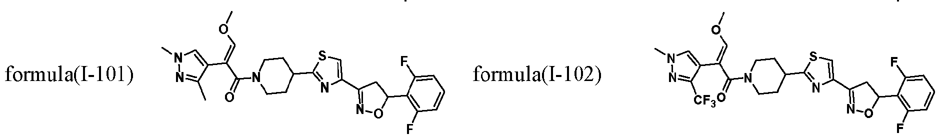

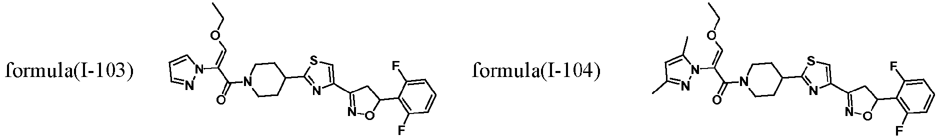

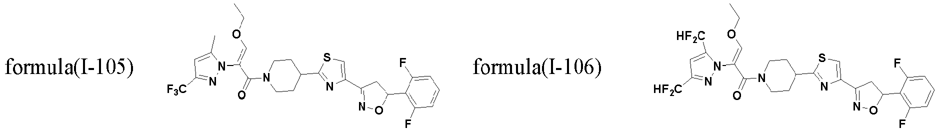

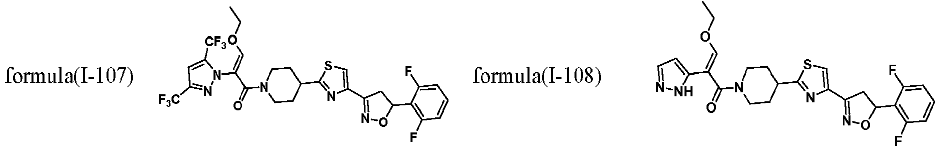

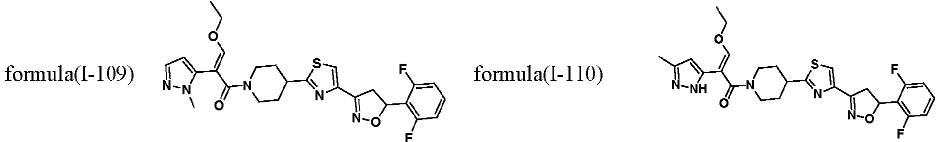

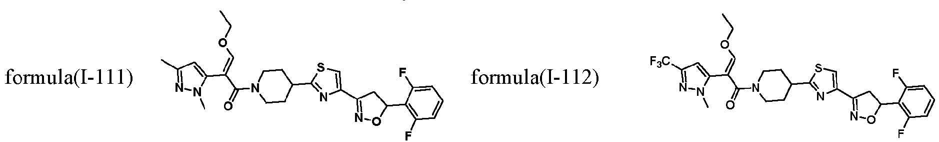

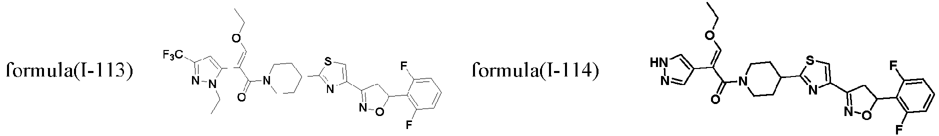

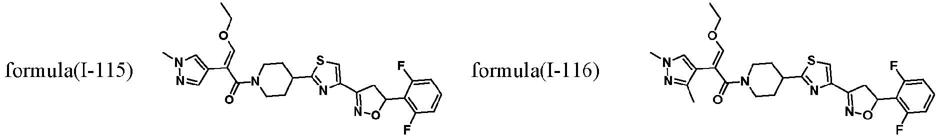

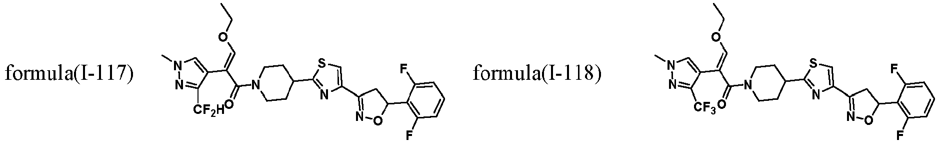

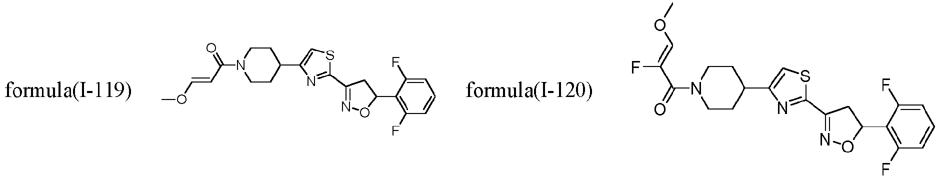

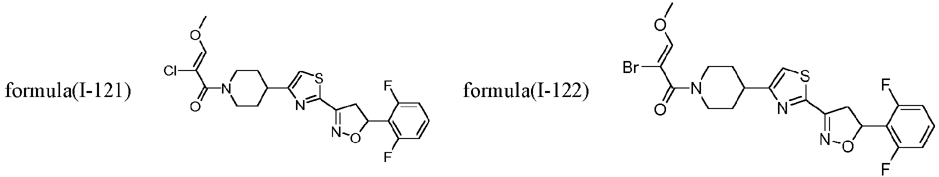

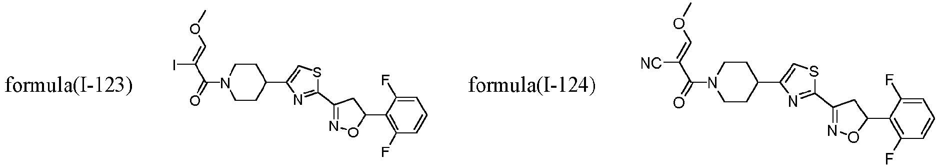

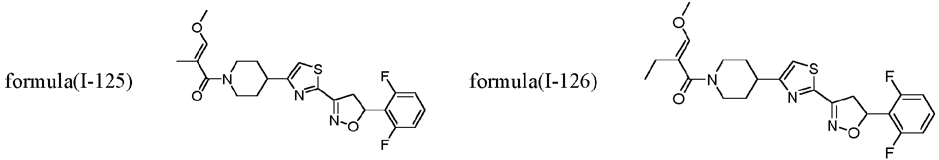

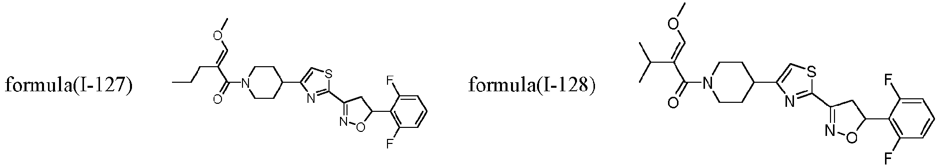

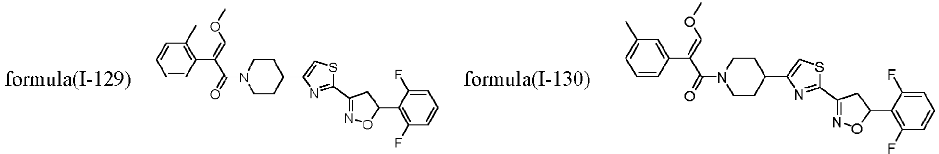

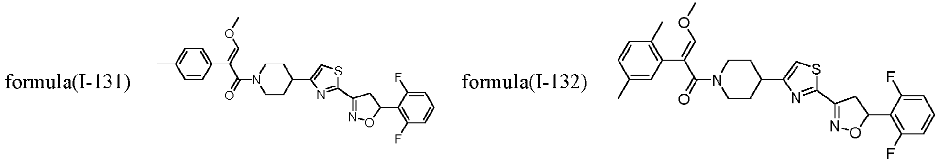

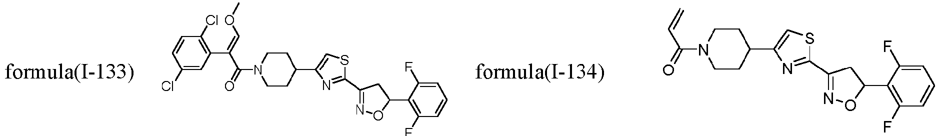

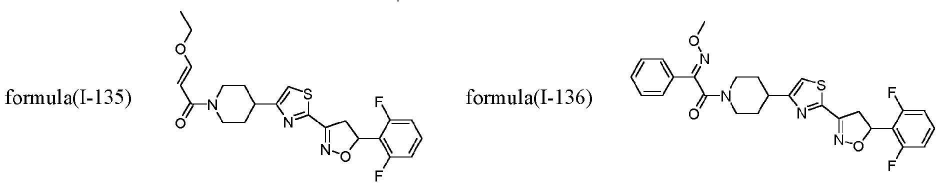

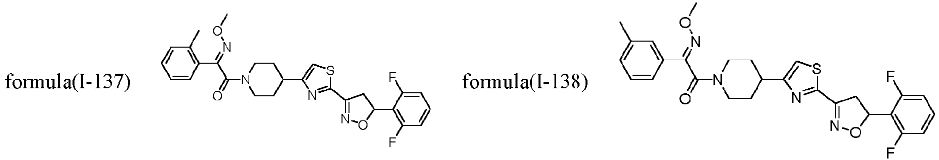

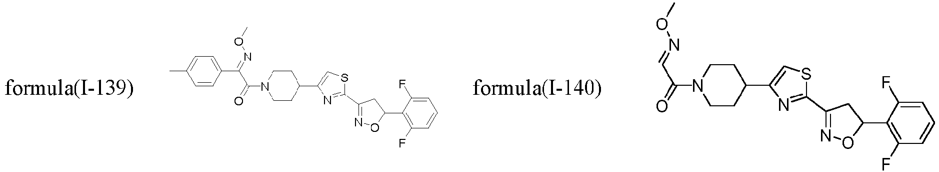

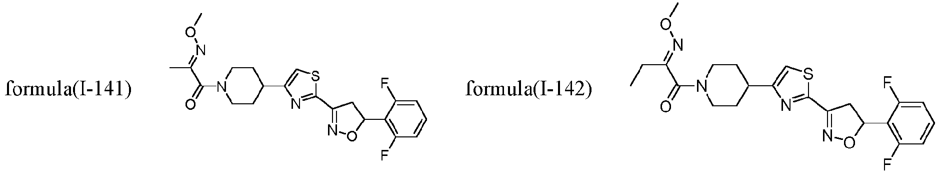

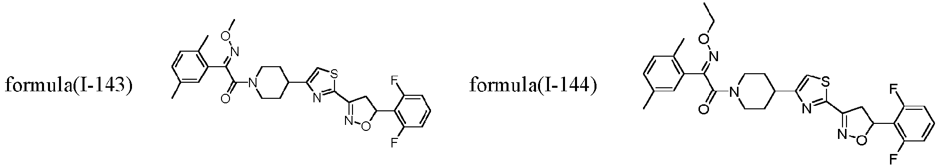

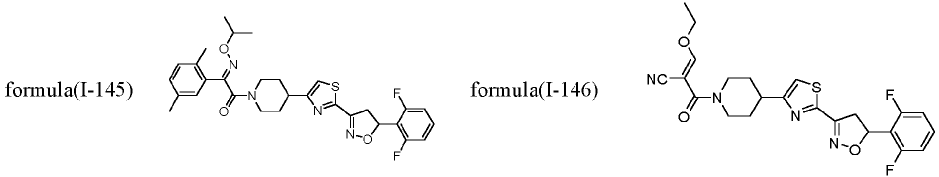

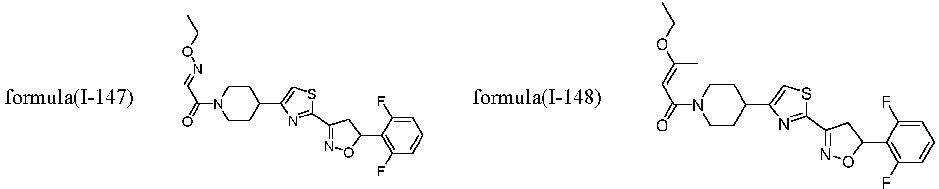

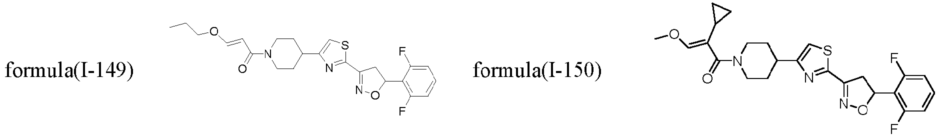

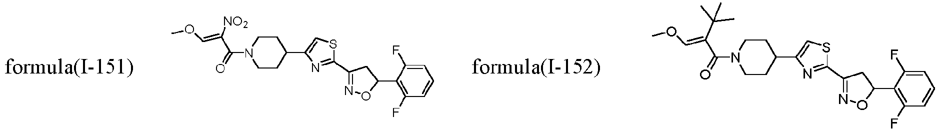

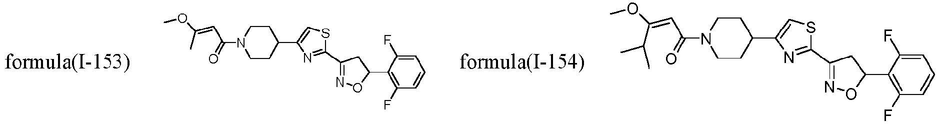

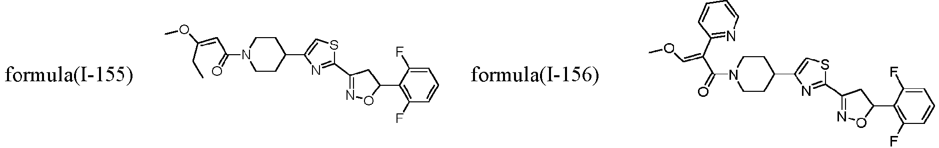

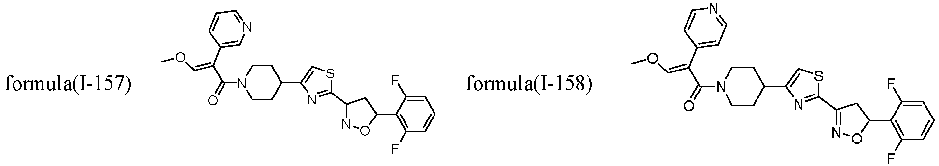

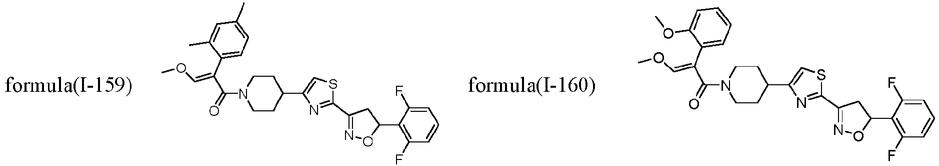

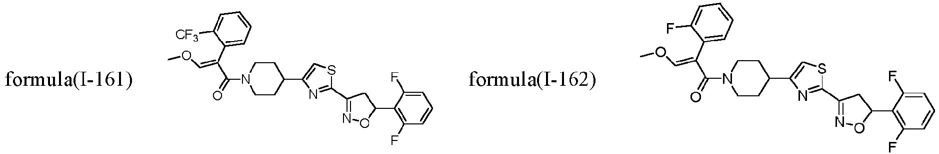

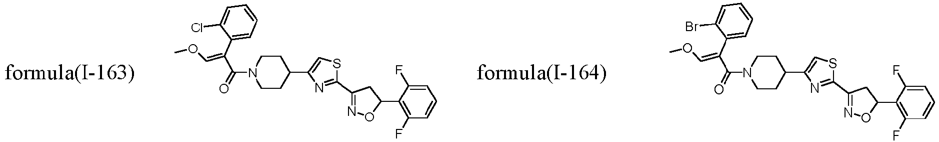

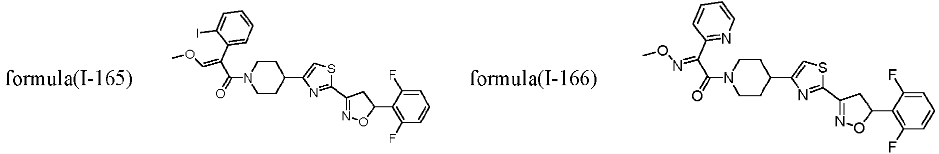

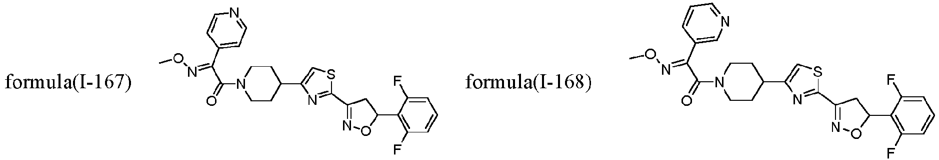

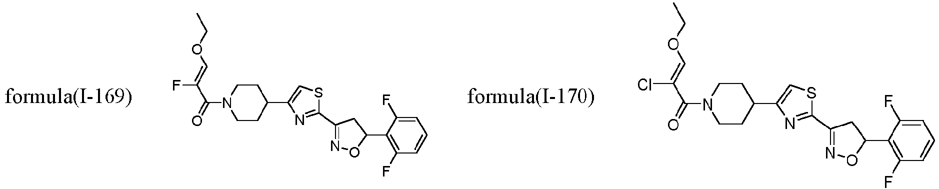

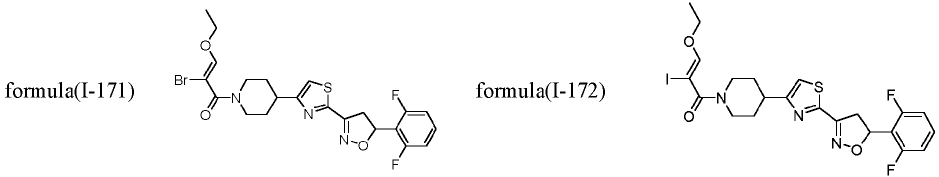

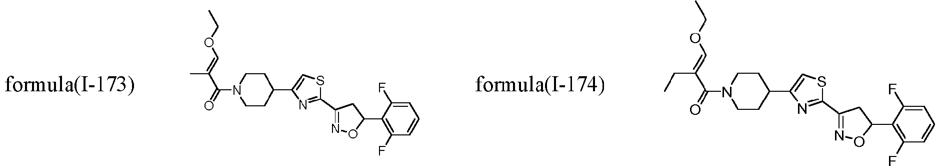

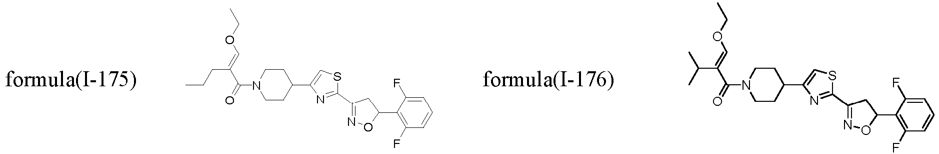

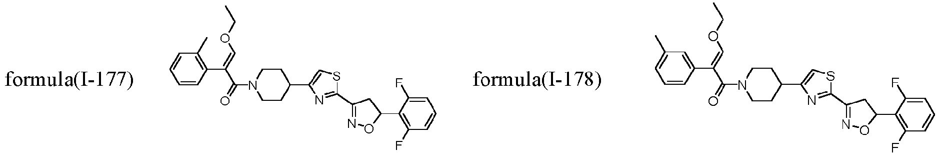

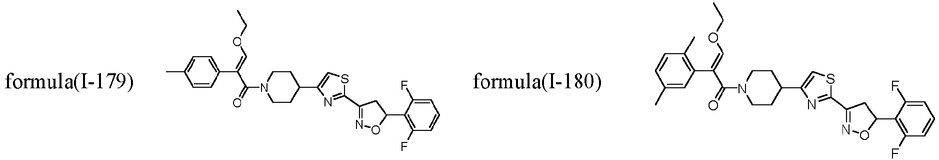

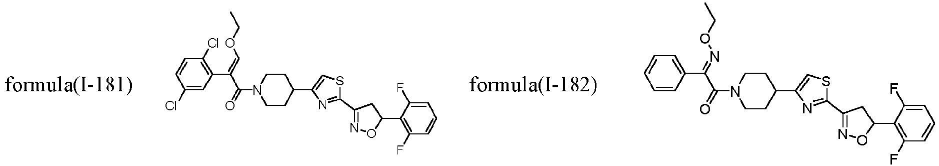

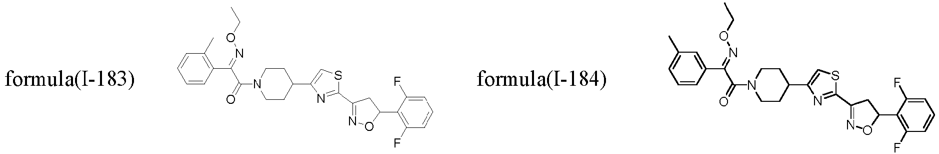

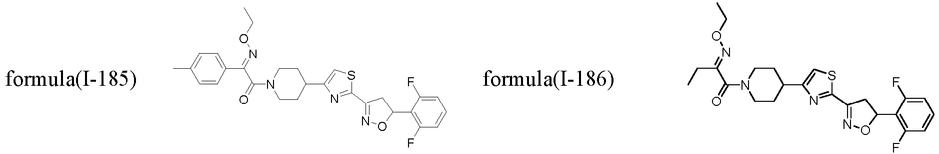

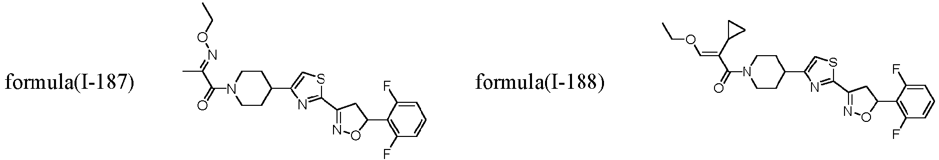

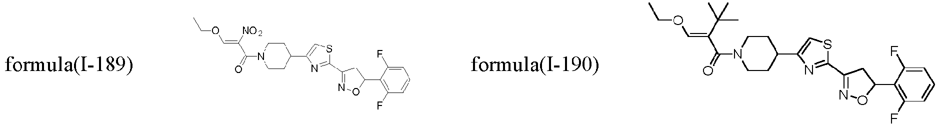

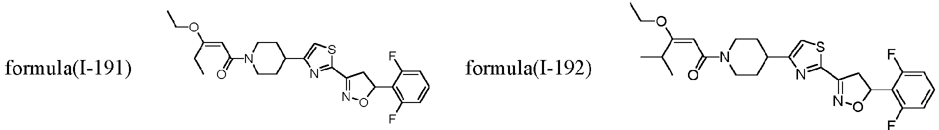

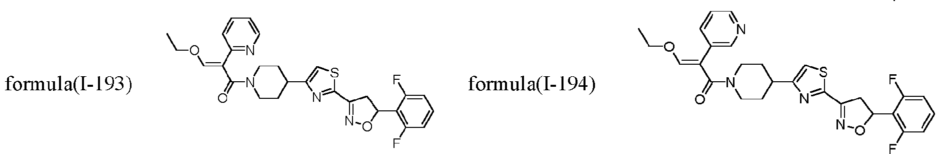

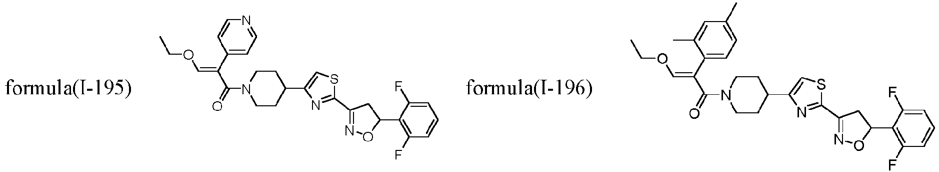

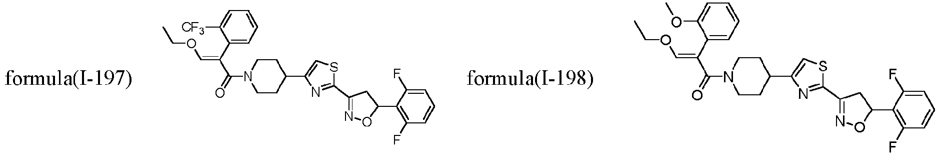

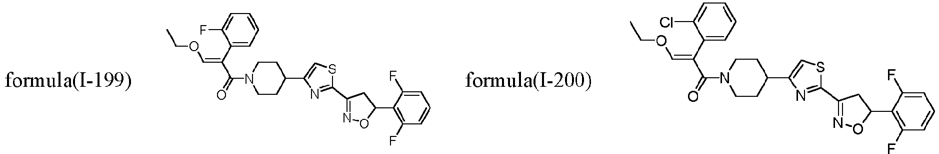

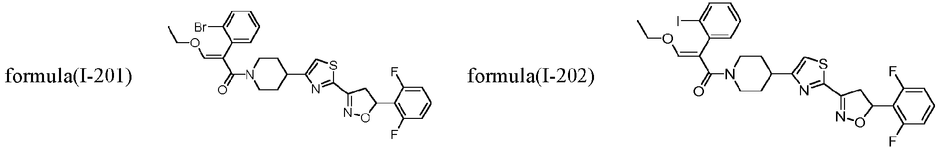

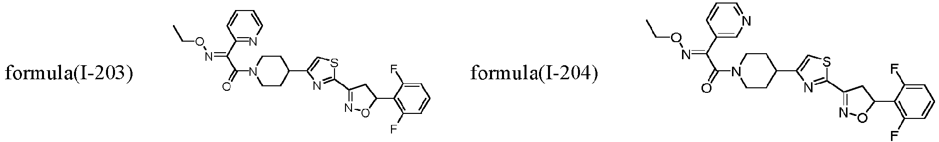

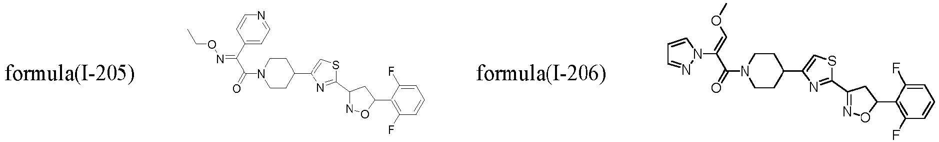

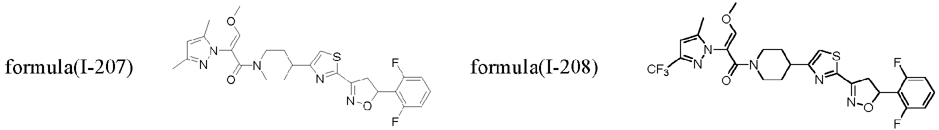

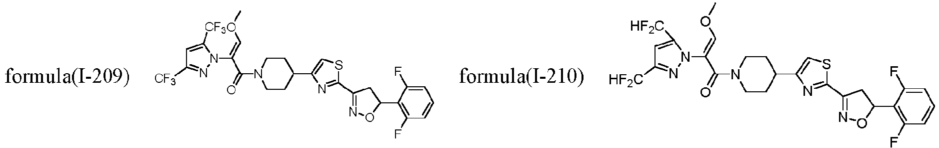

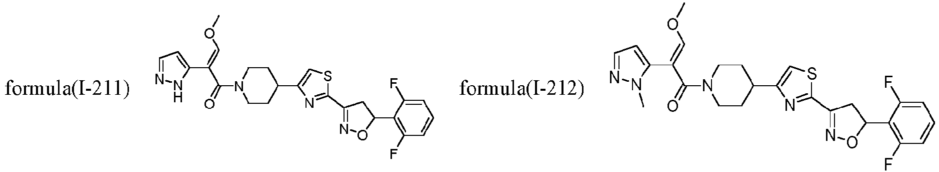

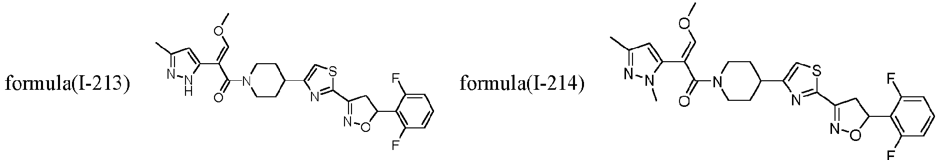

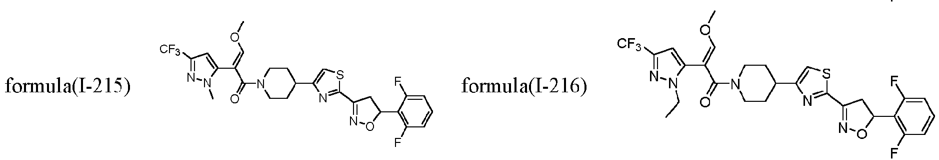

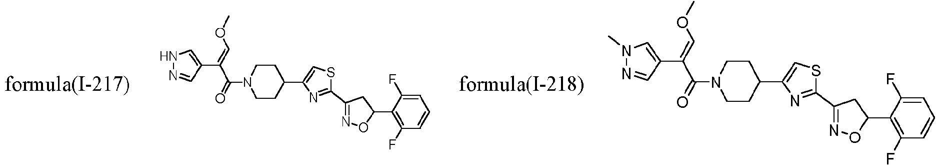

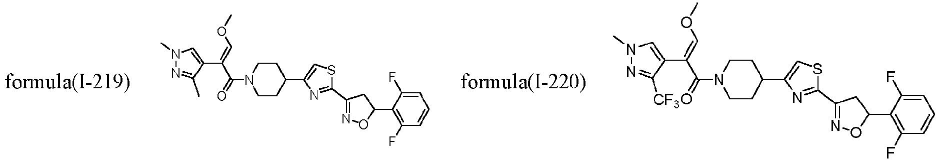

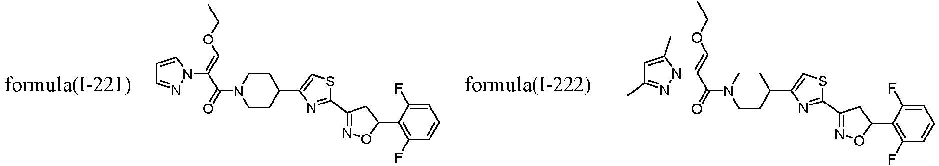

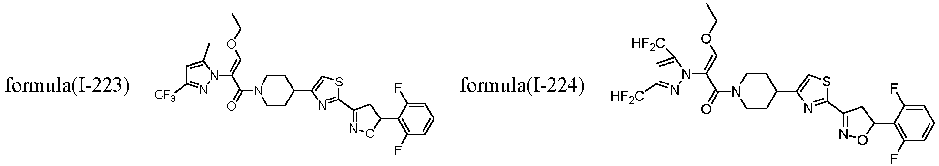

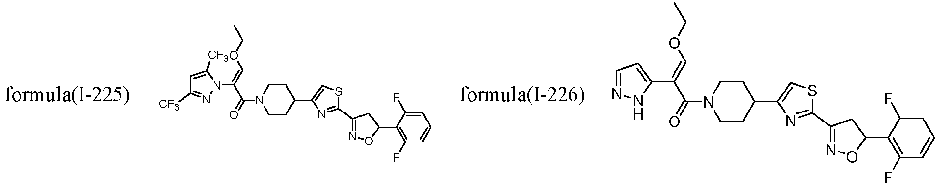

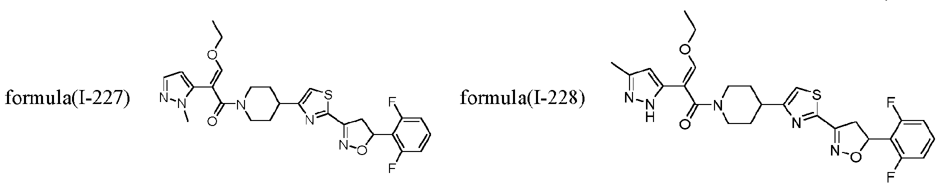

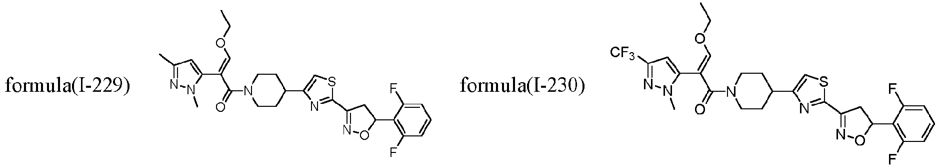

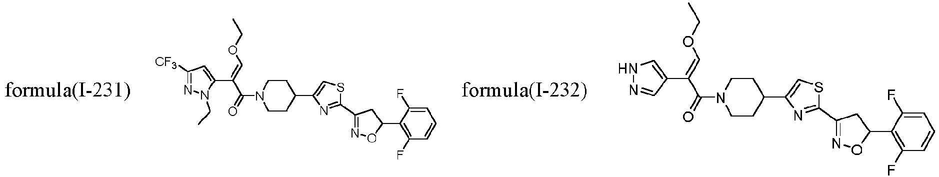

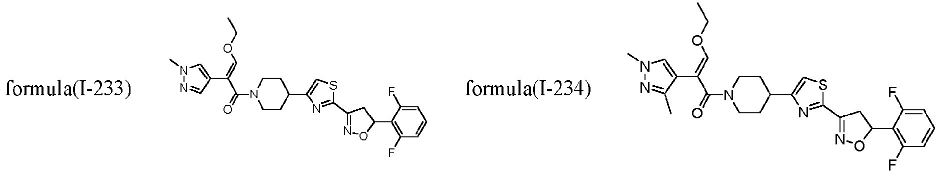

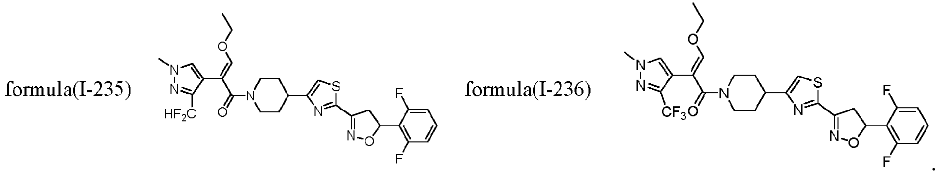

4. The compound according to claim 1, wherein, in formula (I),
one of X₁ and X₂ is S, and the other is CH;
R is a structure represented by formula (Q2); R¹, R², R³, R⁴ are each independently selected from the group consisting of H, alkyl of C₁-C₁₂, alkoxy of C₁-C₁₂, cycloalkyl of C₃-C₁₂, alkylthio of C₁-C₁₂, alkoxy of C₁-C₁₂ substituted with at least one halogen, alkoxy of C₁-C₁₂ substituted with phenyl, halogen, phenyl, phenyl substituted with at least one group of combination A, cyano, nitro, pyridyl, pyrazolyl, pyrazolyl substituted with at least one group of combination A; and at least one of R¹, R², R³, R⁴ is alkoxy of C₁-C₁₂ or alkylthio of C₁-C₁₂; or, R² and R³ are ring-combined together to form a 3-7 membered saturated heterocyclic group containing at least one O atom as a ring-forming atom, which is unsubstituted or substituted with at least one group from the combination A;
the combination A consists of alkyl of C₁-C₁₂, halogen, alkyl of C₁-C₁₂ substituted with at least one halogen, alkoxy of C₁-C₁₂;
preferably, in formula (I),
one of X₁ and X₂ is S, and the other is CH;
R is a structure represented by formula (Q2); R¹, R², R³, R⁴ are each independently selected from the group consisting of H, alkyl of C₁-C₁₀, alkoxy of C₁-C₁₀, cycloalkyl of C₃-C₁₀, alkylthio of C₁-C₁₀, alkoxy of C₁-C₁₀ substituted with at least one halogen, alkoxy of C₁-C₁₀ substituted with phenyl, halogen, phenyl, phenyl substituted with at least one group of combination A, cyano, nitro, pyridyl, pyrazolyl, pyrazolyl substituted with at least one group of combination A; and at least one of R¹, R², R³, R⁴ is alkoxy of C₁-C₁₀ or alkylthio of C₁-C₁₀; or, R² and R³ are ring-combined together to form a 3-7 membered saturated heterocyclic group containing at least one O atom as a ring-forming atom, which is unsubstituted or substituted with at least one group from the combination A;
the combination A consists of alkyl of C₁-C₁₀, halogen, alkyl of C₁-C₁₀ substituted with at least one halogen, alkoxy of C₁-C₁₀.

5. The compound according to claim 4, wherein the compound of the structure shown in formula (I) is selected from any one of the following:

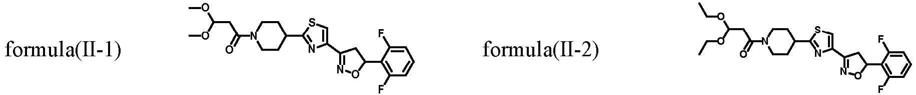

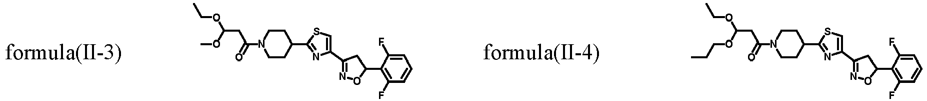

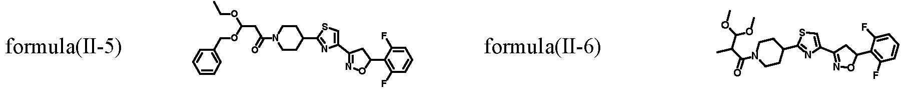

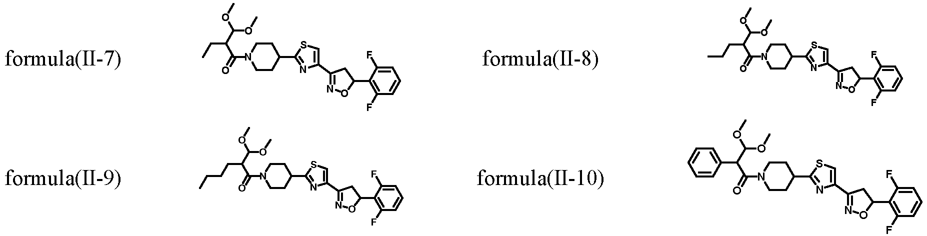

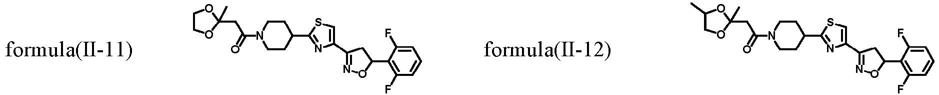

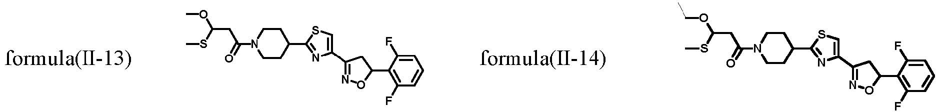

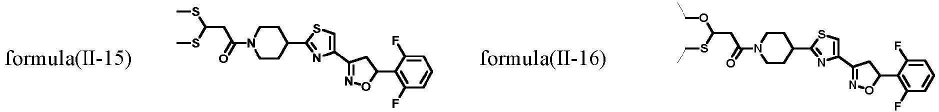

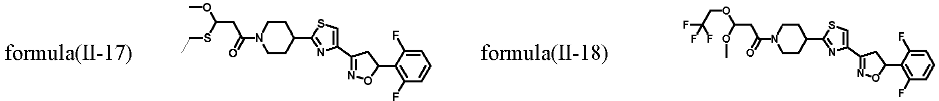

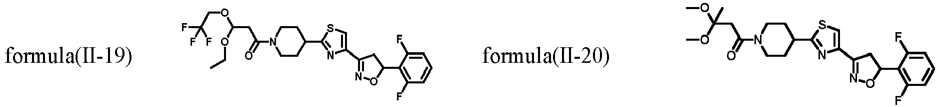

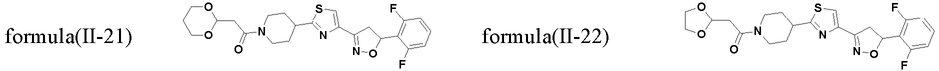

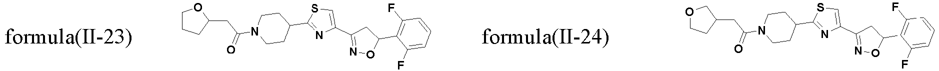

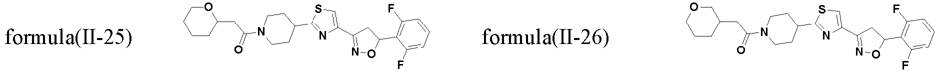

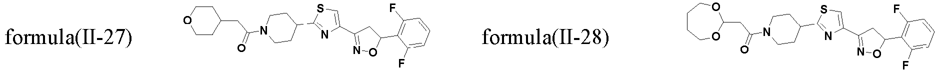

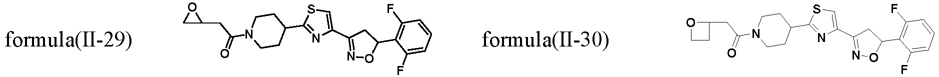

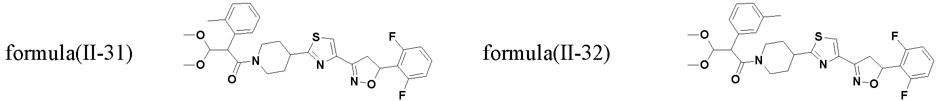

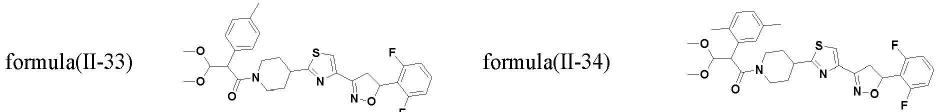

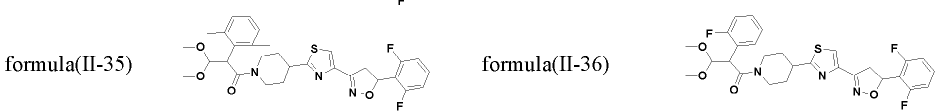

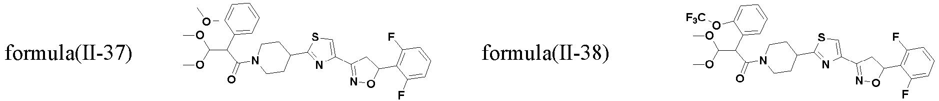

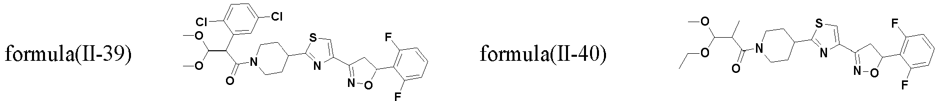

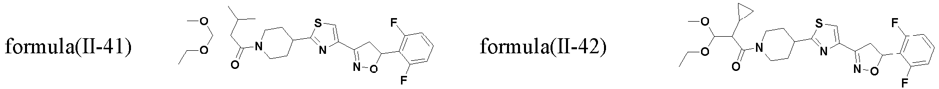

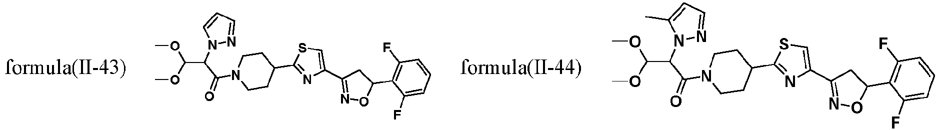

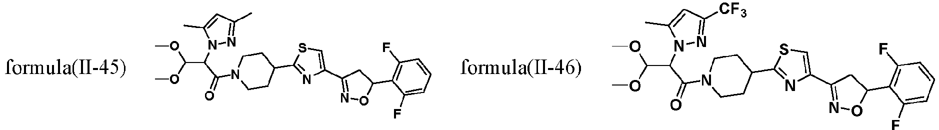

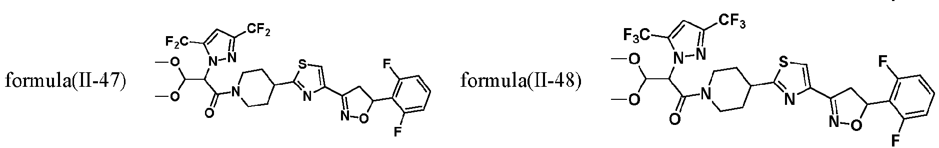

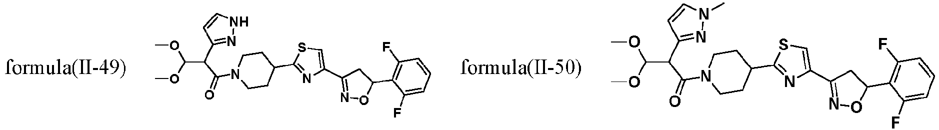

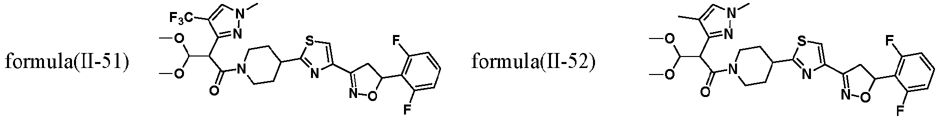

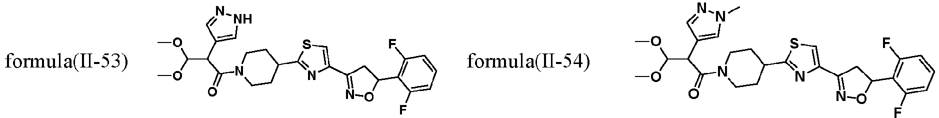

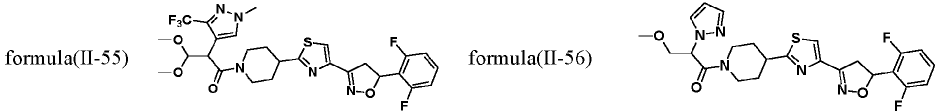

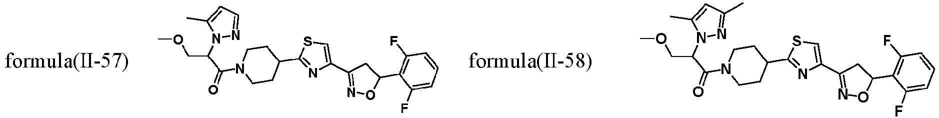

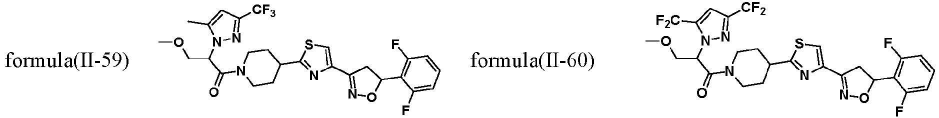

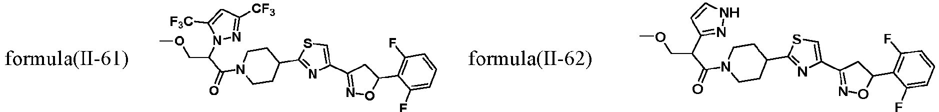

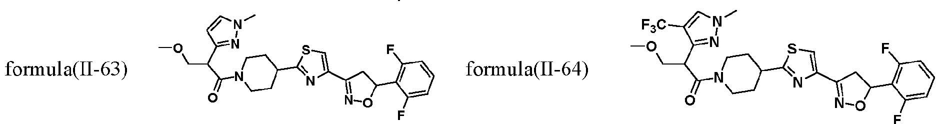

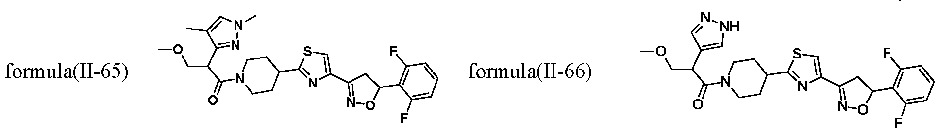

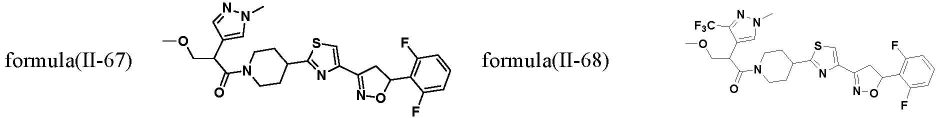

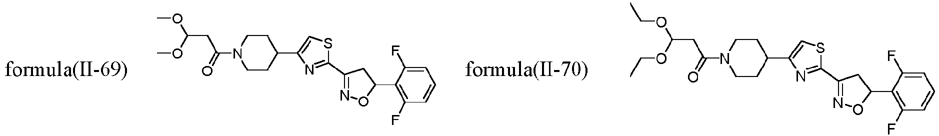

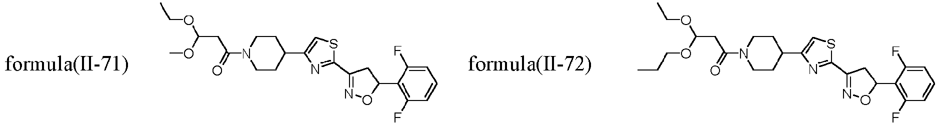

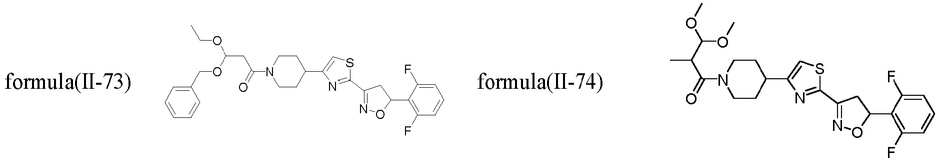

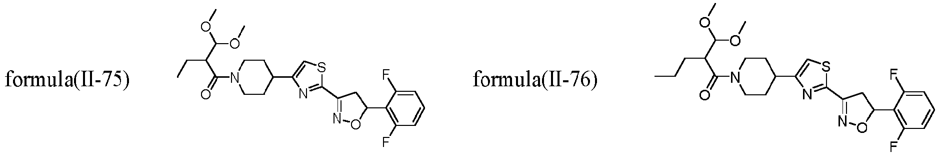

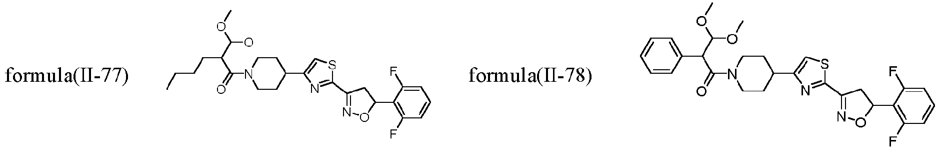

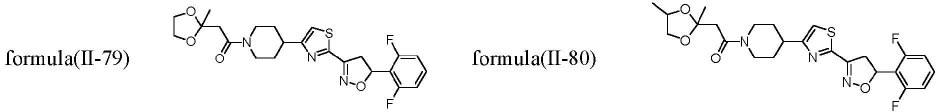

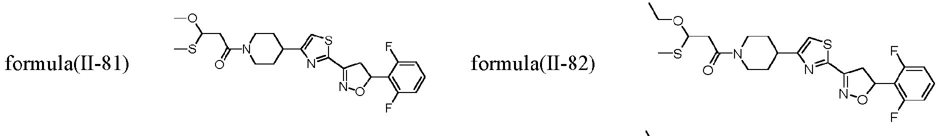

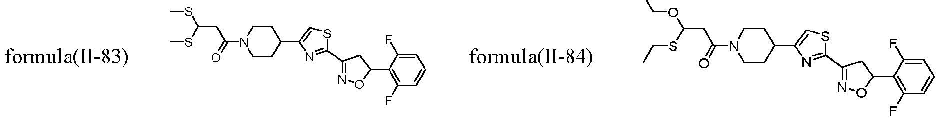

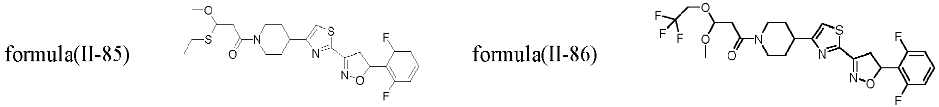

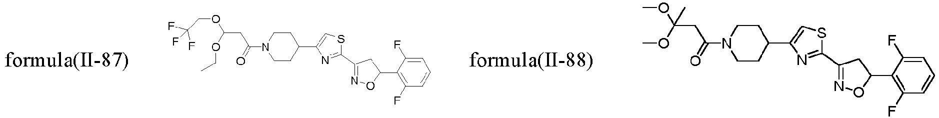

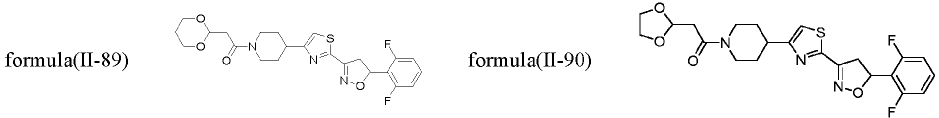

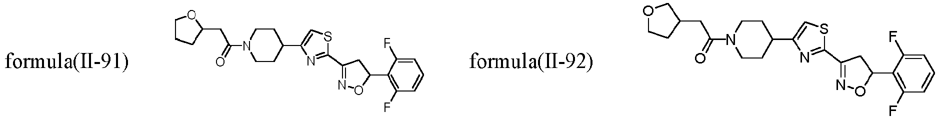

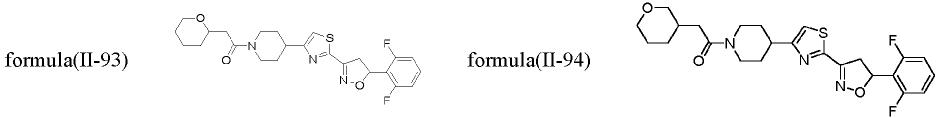

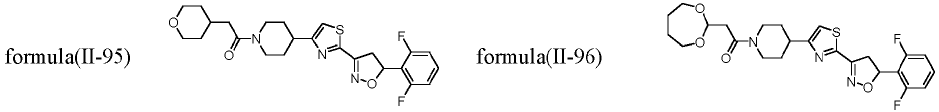

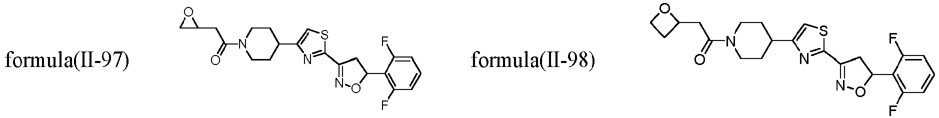

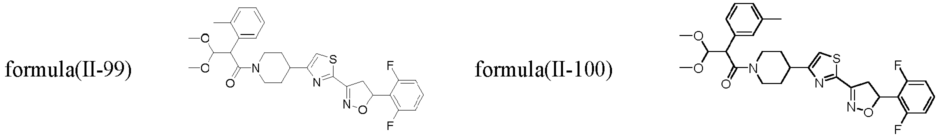

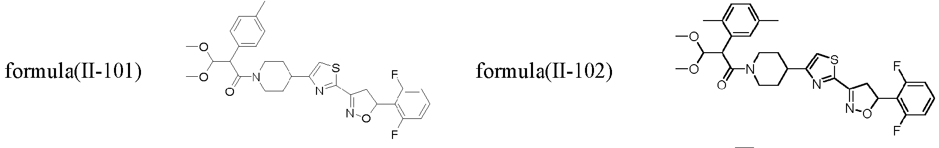

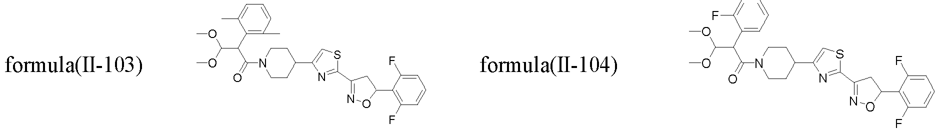

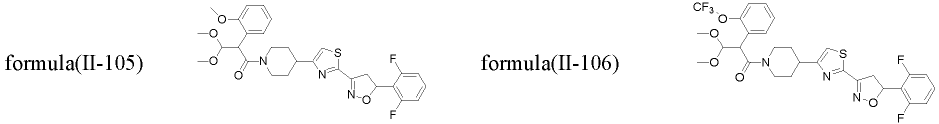

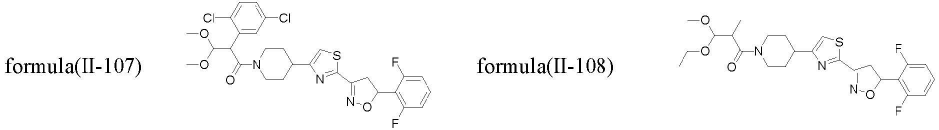

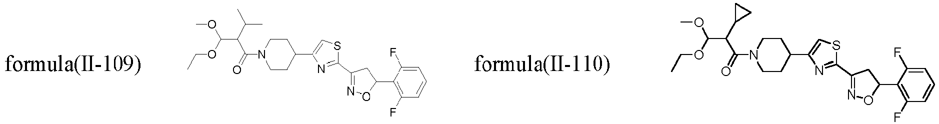

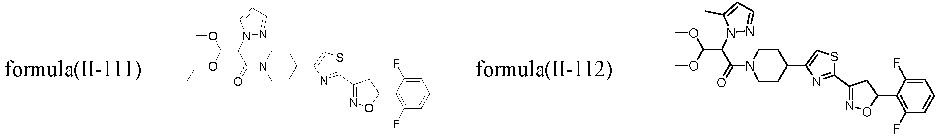

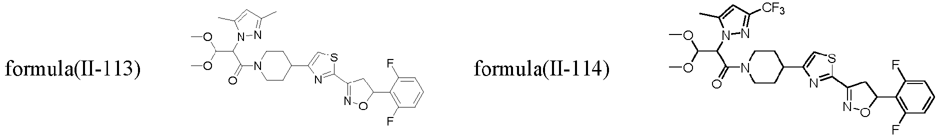

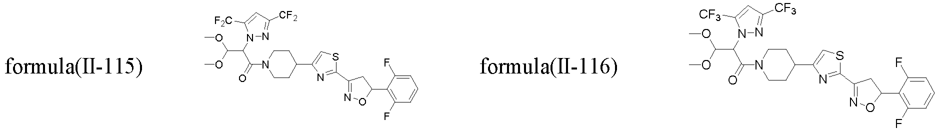

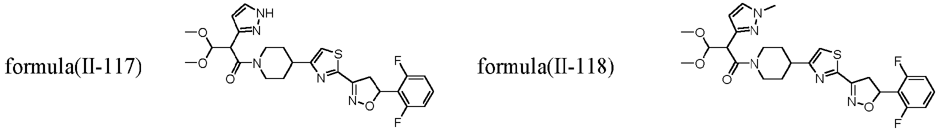

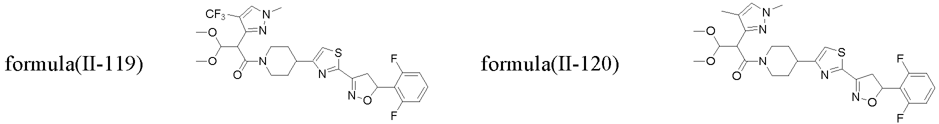

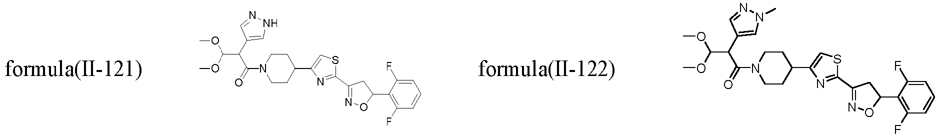

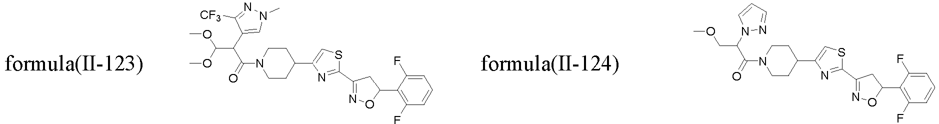

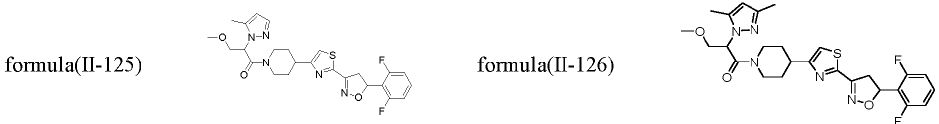

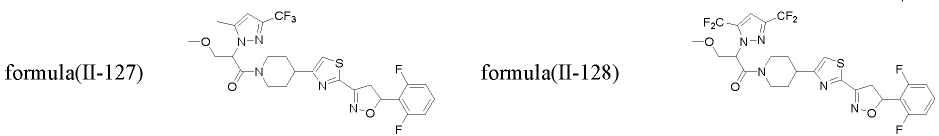

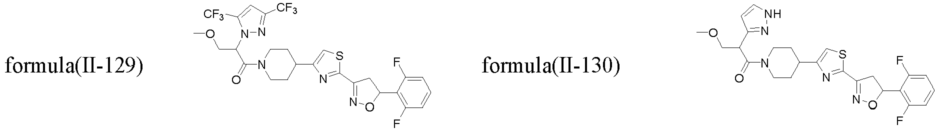

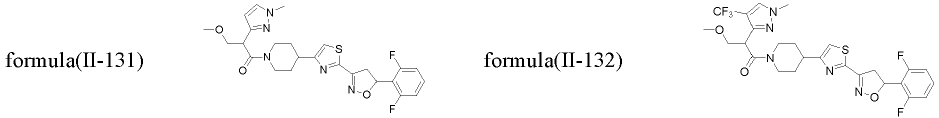

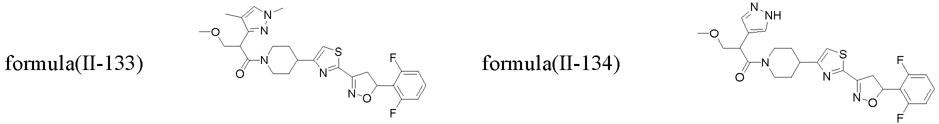

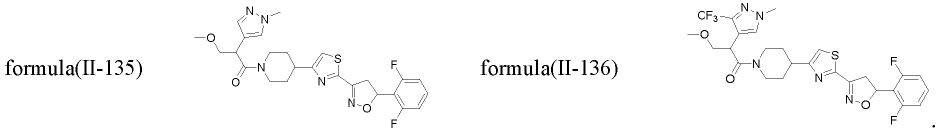

6. A method for producing the compound containing a chain-shaped carboxylic acid amide structure or an agrochemically acceptable salt, hydrate and solvate thereof described in any of claims 1-5; wherein the method comprises: under condensation reaction conditions, a compound shown in formula (II) and a compound shown in formula (III) are subjected to contact reaction, 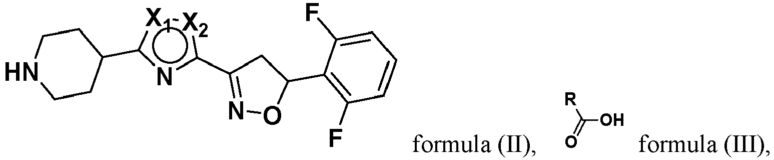 and the definition of substituents in the compound represented by formula (II) and the compound represented by formula (III) is the same as that in any one of claims 1 to 5.

7. Use of the compound containing a chain-shaped carboxylic acid amide structure or an agrochemically acceptable salt, hydrate and solvate thereof according to any one of claims 1 to 5 in controlling plant oomycete diseases;
preferably, the plant oomycete disease is selected from at least one of cucumber downy mildew, potato late blight, and pepper phytophthora blight.

8. Use of the compound containing a chain-shaped carboxylic acid amide structure or an agrochemically acceptable salt, hydrate and solvate thereof according to any one of claims 1 to 5 as an agricultural fungicide.

9. A fungicide, which consists of active ingredients and excipients. The active ingredients include at least one of the compounds containing a chain-shaped carboxylic acid amide structure or an agrochemically acceptable salt, hydrate, and solvate thereof according to any one of claims 1 to 5;
preferably, the content of the active ingredient is 1wt% to 99.9wt%.

10. The fungicide according to claim 9, wherein the dosage form of the fungicide is selected from at least one of emulsifiable concentrate, suspension concentrate, wettable powder, powder, granule, aqueous solution, poison bait, mother liquor and mother powder.
